# EUROPEAN PATENT SPECIFICATION

(11) **EP 0 663 826 B1**
(45) Date of publication and mention of the grant of the patent: **23.12.1998**
(21) Application number: 92922032.5
(22) Date of filing: 26.09.1992
(51) Int. Cl.: A61K 31/415, A61K 38/00

(54) **AMINO ACIDS CONTAINING PARENTERAL FORMULATIONS FOR THE TREATMENT OF HYPOTENSION AND RELATED PATHOLOGIES**
Parenteral anzuwendende Aminosäuren enthaltende Zubereitungen zur Bekämpfung von Hypotension und verwandten Pathologien
FORMULATIONS CONTENANT DES ACIDES AMINES ADMINISTREES PAR VOIE PARENTERALE UTILISEES DANS LE TRAITEMENT DE L'HYPOTENSION ET DE PATHOLOGIES APPARENTEES

(30) Priority: 27.09.1991 US 767265; 23.06.1992 US 902653; 01.07.1992 US 910868
(43) Date of publication of application: 26.07.1995
(73) Proprietor: BOARD OF REGENTS THE UNIVERSITY OF TEXAS SYSTEM, Austin, Texas 78701 (US); CORNELL RESEARCH FOUNDATION, INC., Ithaca, NY 14850 (US)
(72) Inventor: KILBOURN, Robert G., Houston, TX 77096 (US); GRIFFITH, Owen W., New York, NY 10021 (US); GROSS, Steven S., New York, NY 10038 (US)
(74) Representative: Dost, Wolfgang, Dr.rer.nat., Dipl.-Chem.
(86) International application number: US9208227
(87) International publication number: WO9305780

(56) References cited:
- EP-A- 0 295 166
- EP-A- 0 312 612
- EP-A- 0 318 446
- EP-A- 0 405 295
- DE-A- 2 516 027
- US-A- 5 006 559

## Description

### BACKGROUND OF THE INVENTION

### 1. Field of the Invention

The present invention relates to the field of prophylaxis and treatment of hypotension. Most particularly, the present invention proposes a formulation capable of controlling and inhibiting hypotension or systemic shock through its administration Such formulations exhibit a limited nitric oxide- generating potential. In particular, the present invention also relates to specially tailored nutritional formulations for patients at risk of hypotension or systemic shock which include low concentrations of arginine or are arginine-free. Particular embodiments of the formulations also include ornithine or citrulline as urea cycle substrates. Anti-hypotensive TPN formulations for the general nutritional support of patients are also provided. The invention also relates to formulations comprising nitric oxide inhibitors. The present invention also relates to such formulations comprising arginine analogs, anti-tumor necrosis factor antibodies, anti-endotoxin antibodies and interleukin-1 receptor antagonists, which formulations are suitable for the treatment of hypotension, septic shock and related conditions.

### 2. Background of the Art

Hypotension, or low blood pressure, is a complicating and often life-threatening condition attendant to shock, traumatic injury, sepsis, the administration of immunomodulators, as well as other situations. Thus, the risk of hypotension affects a significant number of persons throughout the world. For example, septic shock, a life-threatening complication of bacterial infections, affects 150,000 to 300,000 patients annually in the United States alone.¹

The cardiovascular collapse and multiple metabolic derangements associated with septic shock are due largely to bacterial endotoxin (ET), which has been shown to elicit a septic shock-like condition when administered to animals². ET is known to stimulate the synthesis and release of several cytokines and biological mediators having hypotensive activity; among the factors released, TNF, platelet activating factor (PAF), prostacyclin and complement-derived C5a anaphylatoxin have been proposed as contributors to the cardiovascular collapse of septic shock³⁻⁶.

Although it has been shown that animals pretreated with anti-TNF antibodies⁷, PAF receptor antagonists⁸, and prostacyclin synthesis inhibitors⁹ are significantly protected against septic shock, the relative importance of these mediators in the pathology of septic shock is presently uncertain.

There is also evidence that some of these mediators may act indirectly *via* release of secondary mediators. Thus, the finding that anti-TNF antibodies have little or no protective effect when given after ET exposure⁷ suggests that TNF stimulates the production of another factor that is the actual hypotensive agent. Once initiated, synthesis and release of that factor can apparently continue even in the absence of detectable TNF. In 1980, Furchgott *et al*. (1980)¹⁰ demonstrated that endothelial cells, which line blood vessels, can be stimulated to release a substance which relaxes vascular smooth muscle (i.e., causes vasodilatation). Since the chemical nature of this substance was completely unknown, it was simply named endothelium-derived relaxing factor (EDRF). It is hypothesized that many naturally-occurring substances which act as physiological vasodilators mediate all or part of their action by stimulating release of EDRF; these substances include, acetylcholine, histamine, bradykinin, leukotrienes, ADP, ATP, substance P, serotonin, thrombin and others.

The extremely short lifetime of EDRF (several seconds) hampered early efforts to chemically identify this molecule. In 1987, several laboratories suggested that EDRF may be nitric oxide (NO), which spontaneously decomposes to nitrate and nitrite. However, the fundamental problem in accepting this NO hypothesis was that mammalian systems were not known to contain an enzymatic pathway which could synthesize NO; additionally, a likely precursor for NO biosynthesis was unknown.

After observing that the arginine analog L-N^{G}-methylarginine (L-NMA) could inhibit vascular EDRF/NO synthesis induced by acetylcholine and histamine, and that EDRF/NO synthesis could be restored by adding excess L-arginine, certain of the present inventors proposed that arginine is the physiological precursor of EDRF/NO biosynthesis¹¹. Certain of the present inventors later demonstrated that inhibition of EDRF/NO synthesis in the anesthetized guinea pig raises blood pressure.

The enzyme responsible for NO synthesis (nitric oxide synthase) has been partially characterized by some of the present inventors¹⁴ and acts to oxidize a terminal nitrogen of the guanidino group of arginine, resulting in production of nitric oxide and citrulline. Macrophage-derived nitric oxide is now considered an important tumoricidal and bactericidal agent.

It has been reported that macrophage cells become "activated" by 12-36 hour after treatment with gamma-interferon, bacterial endotoxin and various cytokines *in vitro*. However, this *in vitro* "activation" system had been associated only with the initiation of tumor cell killing.

However, none of the literature or studies available prior to the present inventors work associated hypotension with nitric oxide, or the involvement of macrophages with hypotension.

Macrophages are a guantitatively insignificant component of normal blood vessel walls, and have never been shown to play any role in blood pressure regulation; i.e., there existed no biochemical, physiological or immunological data to suggest that macrophages had any role in pathological hypotension. Thus, the inventors sought to investigate the role of nitric oxide in systems relevant to the manifestation of hypotension, specifically the role of cytokine induced pathological hypotension, particularly on cells which comprise blood vessel walls.

Cytokines are well known to cause morphological and functional alterations in endothelial cells described as "endothelial cell activation". Distinct immune-mediators such as tumor necrosis factor (TNF), interleukin-1 (IL-1), and gamma-interferon (IFN) appear to induce different, but partially overlapping, patterns of endothelial cell activation including increased procoagulant activity, PGI2 production, HLA antigen expression¹⁸ and lymphocyte adhesion molecule activation. Although these cytokines are reported to cause hypotension, vascular hemorrhage, and ischemia, the underlying mechanisms of altered vasoactivity are unclear.

In both clinical and animal studies on the effects of biological response modifiers, a major dose limiting toxicity has been hypotension and vascular leakage. The inventors have observed that endotoxin and tumor necrosis factor can induce overproduction of nitric oxide in animals. Nitric oxide is a vasoactive substance which controls resting blood pressure. This led the present inventors to postulate that hypotension in humans resulting from administration of biological response modifiers or from the development of overwhelming bacterial infections is due to excessive production of nitric oxide in sufficient concentration to relax vasoconstriction. However, macrophages are known to compose quantitatively only an insignificant component of normal blood vessel walls. Moreover, as a practical matter, it was unlikely that the amount of nitric oxide generated by circulating macrophages would be sufficient to elicit a "hypotensive" effect physiologically, as nitric oxide is not produced in vast enough quantities by the limited number of macrophages in blood vessel walls to produce such a pronounced physiological response. This, together with the recognized short half-life nitric oxide *in vivo* (3-5 seconds), opposed the theory that macrophage-derived nitric oxide was involved in hypotension.

The inventors observed that nitric oxide is derived from the amino acid L-arginine. L-arginine is a typical ingredient in commercially available TPN (total parenteral nutrition) formulations.

A TPN regimen of low or essentially arginine-free formulations is proposed by the inventors to reduce, if not eliminate, the risk of hypotension and septic shock in patients with bacterial infections. Clinical regimens which typically require the administration of a TPN formulation include, for example, nutritional support of cancer patients and others who have no or limited ability to tolerate oral feeding.

Interleukin-1-α (IL-1) has been shown to enhance the restoration of peripheral blood leukocytes in mice myelosuppressed by cytotoxic chemotherapeutic agents.¹⁶ In addition, IL-1 inhibits growth of murine tumors *in vitro* and *in vivo* and is cytocidal for several tumor cell lines.^{17,18} Clinical trials in conjunction with the National Cancer Institute are currently underway to assess the efficacy of IL-1 as an immunorestorative agent in cancer patients exhibiting myelosuppression secondary to chemotherapy. In these trials, the does-limiting side effect of IL-1 is hypotension,¹⁹⁻²¹ a complication that may prevent the administration of therapeutically effective doses of IL-1.

The association of IL-1 with compromised cardiovascular function is well documented in both animal and patient studies. thus, Dinarello *et al.*²² have shown that administration of IL-1 causes a shock-like syndrome in rabbits, an effect they attributed to the overproduction of cyclooxygenase products. These authors also have shown a synergistic association between exogenous IL-1 and tumor necrosis factor (TNF) in causing shock. There is now considerable evidence that shock due to exposure to endotoxin (i.e., septic shock) is associated with increased endogenous production of both TNF and IL-1.²³ The critical role of IL-1 in endotoxic shock is substantiated by recent reports showing that administration of a human recombinant IL-1 receptor antagonist improves survival and prevents the development of hypotension in rabbits given endotoxin.

The inventors have previously shown that pathologic overproduction of nitric oxide (NO·) -- initially characterized as endothelium-derived relaxing factor^{10,11} --mediates the hypotension caused by the administration of TNF to dogs.²⁶

NO· is a short-lived but potent vasodilator formed enzymatically by oxidation of one of the two equivalent guanidino (i.e., omega) nitrogens of L-arginine in a reaction catalyzed by nitric oxide synthase. Several N^{ω}-substituted arginine analogues are effective inhibitors of nitric oxide synthase, and some have been shown to inhibit NO· production *in vivo*.

Some of these N^{ω}-substituted arginine analogs include N^{ω}-methyl-L-arginine (NMA), NNA (N^{ω}-nitro-L-arginine) and NAA (N^{ω}-amino-L-arginine). Some enthusiasm has been generated in regard to the use of nitric oxide inhibitors, such as NMA, as antihypotensive agents. However, this prospect has been tempered by the recent demonstration that very large doses of NMA demonstrates an increase in mortality in endotoxic rodents, while other studies have indicated that NMA increases hepatic damage following administration of endotoxin to rats. The inventors own work has also shown that treatment with large doses of arginine analogs, such as NMA results in lower cardiac output *in vivo* while increasing blood pressure, the results of which could compromise oxygen delivery to critical organs. Therefore, methods and regimens which could optimize the potential normotensive action of these agents at lower doses would make these agents significantly more acceptable and useful in the clinical setting.

A method would maximize the beneficial anti-hypotensive effects of low physiological arginine concentrations, for example, by the maintenance of an animal, on an arginine-free dietary support (TPN), coupled with moderate doses of a nitric oxide synthase inhibitor would provide a significant advantage in managing an animal with hypotension or at risk of developing hypotension.

### SUMMARY OF THE INVENTION

The present invention relates to formulations capable of preventing or inhibiting systemic hypotension in an animal induced by a biological response modifier or by bacterial sepsis. By way of example, the biological response modifiers which are linked to causing systematic hypotension include the cytokines IFN, TNF, IL-1 and IL-2. The formulation (arginine-free) is administered, preferably parenterally, to a hypotensial or potentially hypotensive animal to reduce the level of serum arginine systemically, and thereby effect a reduction in the synthesis of nitric oxide.

The inventors have observed that nitric oxide is derived from the amino acid L-arginine. Therefore, the formulations of the present invention are essentially argine-free or have only very low concentrations of arginine. Reduced arginine will serve to reduce nitric oxide synthesis and thus reduce the systemic hypotension manifest in an animal with an elevated level of nitric oxide.

Particular embodiments of the TPN anti-hypotensive formulations of the present invention include those formulations additionally comprising citrulline and/or ornithine. Citrulline and/or ornithine, which do not directly result in nitric oxide production, may be included to satisfy metabolic requirements such as those of the urea cycle, for example. As used in the present application, the term "low" arginine refers to a TPN solution which includes not more than 0.1% arginine in the feeding solution (≼100 mg. arginine/100 ml TPN). A preferred embodiment of the formulation includes a concentration of arginine between 0.1% and 0.001% arginine. In another preferred embodiment the anti-hypotensive TPN of the present invention includes about 0.001% (1 mg/100 ml TPN) arginine. In another preferred embodiment arginine is included in the formulation at trace amounts of less than 0.1 mg/100 ml (about 0.0001%). The formulation in another preferred embodiment is essentially arginine-free.

In a preferred embodiment the formulation is an essentially arginine-free formulation and is also essentially ornithine-free and citrulline-free. Alternatively, the essentially arginine-free formulation may include ornithine or citrulline. So formulated, a specially tailored total parenteral nutrition (TPN) formulation for a patient at risk of systemic sepsis or suffering from other nitric oxide-mediated hypotension is provided.

Although administration of the inventive formulation to an animal is preferably parenteral, it is contemplated that other administration routes, such as by oral administration, for example, may prove useful as the route of administration.

In one embodiment, the arginine-free formulation is administered to an animal which may develop, is possibly developing, or is experiencing NO-mediated systemic hypotension. The arginine-free formulations of the present invention may include any pharmaceutically acceptable addition salts as commensurate with planned treatments.

A particular preferred use of the formulation of the present invention is for the manufacture of a medicament for the prophylaxis or treatment of systemic hypotension manifest in a patient receiving chemotherapeutic agents, such as TNF, IL-2 (interleukin-2), IL-1 (interleukin-1), or a combination thereof. In this respect, administering to the patient, a nutritionally supportive amount of a low arginine or essentially arginine-free amino acid TPN formulation for a period of time until an elevation in the animal's systolic blood pressure to a physiologically acceptable level is demonstrated is necessary. By way of example, a physiologically acceptable systolic blood pressure level in an adult human is 100 mm Hg (100 millimeters of mercury) or greater. An adult human having a systolic blood pressure level of less than 100 mm Hg is defined as manifesting the condition known as "hypotension" for purposes of the invention defined herein. Most preferably, the formulation is prepared so as to be suitable for administration as a parenteral formulation to a patient.

An additional important field for the application of the inventive formulations is the treatment of septic shock, particularly septic shock induced by bacterial endotoxin. Although prophylaxis is not practical here, treatment to improve and eliminate the condition is essential. The treatment of septic shock therefore comprises maintaining the patient on an arginine-free or low arginine formulation comprising a mixture of amino acids until the patient demonstrates a maintained systolic blood pressure within physiologically acceptable levels. In an adult human, a physiologically acceptable systolic blood pressure is at least 100 mm Hg (100 millimeters of mercury). Most preferably, the formulation is prepared so as to be suitable for administration as a parenteral formulation, and thus must be of a physiologically acceptable pH for administration parenterally.

"Page missing at time of publication"

The inventors data also provides a teaching how inhibit or prevent hypotension by administration of the herein described formulations and regimens.

The formulation may optionally include ornithine or citrulline in concentrations sufficient to meet physiological needs, such as urea acid cycle substrate requirements. The formulation should also be adjusted so as to be physiologically compatible for parenteral administration, such as to adjust the pH of the solution to be between 7.0 and 7.4.

If the formulation employed in the described method includes ornithine and/or citrulline, those concentrations of ornithine and citrulline most preferred are in the range of 1-2 g/l (or 0.10-0.20%) ornithine and/or 1-2 g/l (0.10-0.20%) of citrulline. The ornithine concentration most particularly preferred as part of the formulation is between 2-4 g/l of the TPN formulation in a patient-ready feeding formulation.

As used in the present application, a patient "at risk" for developing hypotension is defined as a patient who is receiving a regimen, or who is prescribed a regimen, of immunomodulators, such as, for example, tumor necrosis factor or interleukin-1 or -2, or who is suffering from systemic hypotension. Other patients at risk include patients with overwhelming bacterial infections or whom have been exposed to a bacterial endotoxin.

In order to prevent hypotension in a patient, a patient would first be identified as "at risk" of hypotension. The identified person would then be administered a low-arginine or essentially arginine-free formulation which includes a mixture of amino acids in nutritionally supportive concentrations as specified in the claims. Nutritionally supportive concentrations of amino acids as included within a parenteral formulation are also provided at Table 3. The mixture of amino acids is ornithine-free and citrulline-free in one particularly preferred embodiment.

A mixture of particular essential and non-essential amino acids are included in the claimed anti-hypotensive formulations. A classification of amino acids recognized by those of skill in the art as "essential" or "non-essential" is provided in Lehninger et al.'s *Biochemistry* text,²⁶ and in Wagner ²⁷.

Studies conducted by the inventors also demonstrated that particular analogs of arginine (arginine antagonists) inhibit hypotension *in vivo*. The administration of particular arginine analog antagonists has been found by the inventors to affect a variety of biochemical pathways physiologically, most notably by decreasing the production of nitric oxide, and thus eventually the hypotensive/normotensive state in the animal.

In a more general sense, the present invention may relate to the use of the present anti-hypotensive formulation in the manufacture of a medicament for the prophylaxis or treatment of nitric oxide-induced systemic hypotension in a patient requiring total parenteral nutritional support. The necessity for maintaining a patient on a parenteral nutritional regimen occurs when a patient is unable to swallow, such as is typical of patients receiving chemotherapeutic agents which induce nausea, emesis or anorexia.

As part of such a regimen, the arginine-free formulation is to be administered on an "as needed" basis, as determined by the attending physician until a sustained systolic blood pressure of at least 100 mm Hg is detectable in the patient.

While not intending to be limited to any particular theory or mechanism of action, it is postulated that the administration of an essentially arginine-free parenteral formulation will inhibit hypotension, because such a reduction in serum-arginine levels will elicit a reduction in nitric oxide synthesis. Nitric oxide, as already discussed, has been observed by the inventors to constitute the vasoactive substance which causes hypotension in animals receiving biological response modifiers or endotoxin which causes septic shock. This theory is drawn from the inventors' observations that patients receiving tumor necrosis factor also exhibit elevated blood nitric levels (a stable breakdown product of nitric oxide) when hypotension is manifest. The formulation may also be supplemented with ornithine, citrulline, or both. Employing the herein disclosed formulations, those persons who may have already lapsed into a state of septic shock, or who are already severely ill and being maintained on a respirator with total parenteral nutritional support, may be nutritionally supported without increasing the risk of exacerbating an already existing condition of septic shock or hypotension.

Within this specification, the acronym "NO" will be understood to represent nitric oxide as well as any other additional vasoactive nitrogen oxides. In still another embodiment of the invention, the arginine-free parenteral formulation also comprises a therapeutically effective amount of a nitric oxide synthase inhibiting arginine analog.

These arginine analogs may be administered concurrently with or subsequent to the administration of the parenteral formulation. Most preferably, the parenteral formulation is administered, in an amount sufficient to reduce physiological concentrations of arginine prior to the administration of the arginine analog.

While any number of arginine analogs having an anti-hypotensive effect may be used in conjunction with the described therapeutic regimen, those arginine analogs most preferred include N^{ω}-methyl-L-arginine, N^{ω}-amino-L-arginine, N^{ω}-nitro-L-arginine, or a mixture thereof. Most preferably, the arginine analog of choice for use in the described therapeutic regimen is N^{ω}-methyl-L-arginine or N^{**ω**}-amino-L-arginine. The therapeutically effective amount of these particular analogs may be defined as between 0.1 mg/kg and 100 mg/kg. In a more narrowly defined aspect of the invention, the therapeutically effective amount of N^{ω}-methyl-L-arginine is a dose of between 10 mg/kg and 30 mg/kg. The dose of arginine analog, such as N^{ω}-methyl-L-arginine most preferred is about 20 mg/kg. These dose ranges may also be used for other arginine analogs with nitric oxide inhibiting activity in the practice of the present invention.

The arginine-free parenteral formulation of the invention may be defined further as including a mixture of essential and nonessential amino acids as specified in claim 1. This mixture of essential and nonessential amino acids include thse described *infra*. Again, the parenteral formulation of the therapeutic regimen is arginine-free. The pharmacologically acceptable excipient of the parenteral formulation is most preferably a Ringers solution or saline. Of these, saline is the most preferred excipient. Ornithine (1-2 g/l) and/or citrulline may be added to the formulation to enhance and maintain metabolic requirements of the urea cycle in the animal.

While the arginine analogs of the therapeutic regimen may be administered according to any administration route known to those of ordinary skill in the medical art, such as through oral, parenteral or enteral routes, the arginine analog is most preferably administered via a parenteral route, such as by an IV, IP or subcutaneous administration. Of these, intravenous administration of the arginine analog is most preferred, particularly through IV bolus treatment.

The parenteral formulation is to be administered in an amount sufficient to reduce plasma concentrations of arginine in the animal, most preferably to an amount less than normal physiological levels. For example, the amount of the parenteral formulation sufficient to reduce plasma concentrations of arginine in an animal may constitute a continuous intravenous feeding of the formulation described herein (arginine-free) for at least 2 hours prior to administration of the arginine analog. In a most preferred aspect of the regimen, the arginine analog is administered *via* an intravenous route to the animal after an initial parenteral feeding has been established. Alternatively, the arginine analog may be administered as a separate treatment concurrently with establishment of the animal on the above-described parenteral formulation.

A method for treating hypotension in an animal requires the following. In one embodiment, this method comprises identifying an animal having a systolic blood pressure of less than about 100 mm Hg, administering to said animal an anti-hypotensive parenteral formulation comprising an arginine-free mixture of essential and nonessential amino acids, administering currently with the formulation or subsequent to the formulation a therapeutically effective amount of an arginine analog capable of inhibiting nitric oxide, monitoring the blood pressure of said animal over a period of at least 24 hours, and maintaining the animal on the formulation and the arginine analog until a systolic blood pressure of at least about 100 mm Hg is detected. Detecting and monitoring blood pressure in an animal having a systolic blood pressure of less than 100 mm Hg may be achieved using standard blood pressure monitoring techniques, such as use of a blood pressure cuff for measurement of blood pressure in a human, for example, or through use of an indwelling arterial catheter connected to a pressure transducer (known to those of skill in the medical arts as an "arterial line").

In the described method, the arginine analog is most preferably N^{ω}-methyl-L-arginine, N^{ω}-amino-L-arginine, N^{**ω**}-nitro-L-arginine or a mixture thereof. Most preferably, the arginine analog of choice to be used in conjunction with the described method is N^{ω}-methyl-L-arginine. The anti-hypotensive parenteral formulation used for the method most preferably comprises the concentrations of essential and nonessential amino acids described *infra*.

Where the arginine analog of choice is N^{ω}-amino-L-arginine, the therapeutically effective concentration of the analog may preferably constitute a dose of between 0.1 mg/kg and 100 mg/kg. An even more preferred concentration range of the arginine analog, N^{ω}-amino-L-arginine, is between 10 mg/kg and 30 mg/kg. Even more preferably, the therapeutically effective concentration of the arginine analog N^{ω}-amino-L-arginine is about 20 mg/kg. According to a most preferred embodiment of the described method, the arginine analog is to be administered intravenously, such as in a single bolus dose.

A method for treating chemotherapeutic agent-related hypotension in an animal receiving a chemotherapeutic agent requires the following. This method in one embodiment comprises monitoring an animal receiving a chemotherapeutic agent for a decrease in systolic blood pressure to less than 100 mm Hg to detect an animal with systemic hypotension, treating the animal with a therapeutic regimen comprising an arginine-free parenteral formulation concurrently with or followed by the administration of a therapeutically effective concentration of an arginine analog capable of inhibiting nitric oxide synthase, and maintaining the animal on the therapeutic regimen until an increase of systolic blood pressure to at least about 100 mm Hg is detectable.

Detecting and monitoring blood pressure in an animal may be accomplished using those techniques well known to those of skill in the medical arts, such as *via* a blood pressure cuff or arterial line as described herein.

It is contemplated that the aforedescribed method may be used in the treatment of a human receiving a chemotherapeutic agent. By way of example, chemotherapeutic agents associated with at least a risk of the development of systemic hypotension include tumor necrosis factor, interleukin-2 and interleukin-1 alone or in combination with interferons or each other.

In a most preferred aspect of the aforedescribed method, the arginine-free parenteral formulation comprises the mixture of essential and non-essential amino acids already described herein together in a pharmacologically acceptable excipient. It is contemplated that the therapeutically effective concentration of the arginine analog capable of inhibiting nitric oxide production to be used in conjunction with the method is between 0.1 mg/kg and 100 mg/kg. A more preferred and narrowly defined range of arginine analog to be used in the described method is between 10 mg/kg and 30 mg/kg. Most preferably, the therapeutically effective concentration of the arginine analog constitutes a dose of about 20 mg/kg.

Those arginine analogs most preferred in conjunction with the herein described methods include N^{ω}-methyl-L-arginine, N^{ω}-amino-L-arginine, or N^{ω}-nitro-L-arginine, or a mixture thereof. Among these, N^{ω}-methyl-L-arginine is most particularly preferred.

A method for treating hypotension attendant septic shock requires the following. In one embodiment of the method, a therapeutically effective amount of an arginine-free parenteral formulation as described herein is administered to an animal concurrently with or prior to the administration of a therapeutically effective amount of an arginine analog capable of inhibiting nitric oxide synthase, and maintaining the animal on the arginine-free parenteral formulation until a systolic blood pressure of at least about 100 mM Hg is detectable in the animal. By way of example, hypotension attendant septic shock is noted in animals with bacterial endotoxin-related septic shock. While any of a variety of animals may be treated for hypotension attendant septic shock according to the aforedescribed method, it is contemplated that the method may find particular applicability in the treatment of humans suffering from such a condition. In particularly preferred embodiments of the described method, the arginine analog preferred is N^{ω}-methyl-L-arginine, N^{ω}-amino-L-arginine, N^{ω}-nitro-L-arginine or a mixture thereof. Of these arginine analogs, N^{ω}-methyl-L-arginine or N^{ω}-amino-L-arginine is most preferred.

A method for providing nutritional support for an animal with hypotension or at risk of developing hypotension requires the following. This method comprises administering to the animal a nutritionally supportive arginine-free parenteral formulation concurrently with or prior to treatment with a hypotension inhibiting concentration of a nitric oxide synthase inhibitor. Subjects at risk of developing hypotension include subjects in shock or having had experienced some sort of trauma, subjects exposed to endotoxic agents or a chemotherapeutic agent having hypotensive activity.

The nitric oxide inhibitors which may be comprised in the formulation of the present invention may be even more specifically described as nitric oxide synthase inhibitors.

A TPN regimen of low or essentially arginine-free formulations is proposed in conjunction with a treatment regimen of nitric oxide synthase inhibitors to reduce, if not eliminate, the risk of hypotension and septic shock in patients with bacterial infections. Clinical regimens which typically require the administration of a TPN formulation include, for example, nutritional support of cancer patients and others who have no or limited ability to tolerate oral feeding.

Preferred N^{ω}-substituted arginine analogs of the L configuration for uses as described herein include N^{ω}-aminoarginine, N^{ω}-nitroarginine, and N^{ω}-alkyl arginines such as N^{ω}-methylarginine, N^{ω}-ethylarginine, N^{ω}-propylarginine or Nω-butylarginine. Therapeutically effective amounts of the substituted or disubstituted arginine analogs inhibit production in the animal or patient of nitric oxide from arginine, thus obviating its hypotensive effects. Notwithstanding the accumulated evidence supporting synthesis of NO·, it is understood by those skilled in the art that other nitrogen oxides may be present and may be active in reducing blood pressure.

The present invention also contemplates the use of additional guanidino-based inhibitors of nitric oxide synthesis for the manufacture of a medicament for the treatment treatment of hypotension, septic shock, and related conditions, in combination with the arginine-free parenteral formulations of the present invention. These guanidino-based inhibitors comprise molecules that are smaller than L-arginine (molecules containing 5 or fewer carbon atoms). The inventors have found that guanidino-containing amino acids that include a carbon chain of one less carbon than arginine do not function as nitric oxide synthase inhibitors, while guanidino containing amino acids that have a carbon chain length one carbon longer than arginine have some, albeit reduced, nitric oxide synthase inhibiting activity. Guanidino containing amino acids which include a carbon chain two carbons longer than L-arginine were found not to have nitric oxide inhibiting activity. The present inventors have also found that arginine analogs that may inhibit nitric oxide synthesis (or nitric oxide synthase) do not require a carboxyl group or one of the amino groups of the native arginine molecule to retain nitric oxide synthase inhibiting activity. Therefore, the present invention is intended to encompass nitric oxide synthase inhibitors and arginine analogs that include these guanidino-based compounds. Examples of these guanidino-based compounds include amino guanidine and N^{G}-alkyl-N^{G'}-amino guanidines, where the alkyl group contains 1 to 3 carbons.

The inventors herein also propose uses of anti-TNF antibodies, anti-endotoxin antibodies, and cytokine receptor antagonists (e.g., IL-1 receptor antagonist) in combination with arginine-free formulations in conceptually distinct approaches for the manufacture of medicaments for the treatment of septic and cytokine-induced shock. The arginine-free formulations may be conveniently supplied as either a parenteral formulation (to be administered intravenously) or as an oral formulation (to be administered enterally). The most preferred embodiment of the parenteral formulation comprises a parenteral formula.

Since antibodies and receptor antagonists intervene early in the pathological response to cytokines, their optimal use requires anticipation of shock. While administration of these agents could affect important biological events after the initiation of the biological response to endotoxin or cytokines, these agents do not block NO-synthesis once the enzyme is induced. Because NO-production, once induced in smooth muscle cells, appears to persist for more than 16 hours after transfer to cytokine-free medium, antibodies and receptor antagonists are less likely to be effective at blocking or slowing NO synthesis once it has begun. The recently reported potential benefit of a human monoclonal antibody against endotoxin in patients with septic shock associated with gram-negative bacteremia (Ziegler *et al.* (1991) *New England journal of Medicine*, *324*:429) is best attributed to the prevention of further induction of cytokine mediators rather than to direct improvement of the existing cardiovascular profile. In contrast, the inventors own work has demonstrated that nitric oxide synthase inhibitors or treatments designed to act to limit nitric oxide synthase substrate availability, act within minutes, even in severe shock.

The rapidity of the response to nitric oxide synthase inhibitors and to treatments designed to limit nitric oxide synthase substrate availability, and the fact that their efficacy is independent of the precipitating factor (i.e., endotoxin, TNF, IL-1 or IL-2) are important clinical considerations that the present inventors have focused in the combination therapies and regimens described in the present application.

According to one aspect of the present invention, a therapeutically effective amount of an arginine-free formulation is to be administered concurrently with or followed by a therapeutically effective amount of an anti-endotoxin antibody. The formulation is further defined as comprising a mixture of essential and nonessential amino acids together in a pharmacologically acceptable excipient while the formulation may be prepared as either a parenteral or enteral formulation; a parenteral formulation is most preferred. The anti-endotoxin antibody may comprise either a polyclonal antibody or a monoclonal antibody. Most preferably, however, the anti-endotoxin antibody is a monoclonal antibody. By way of example, such a monoclonal antibody is HA-1A.

The parenteral formulation of the therapeutic regimen is described *infra*, again constituting a mixture of essential and non-essential amino acids.

In a most preferred embodiment, the parenteral formulation is to be administered concurrently (simultaneously) with the administration of the anti-endotoxin antibody. The therapeutically effective amount of the formulation is most particularly defined as an amount sufficient to reduce plasma or serum concentrations of arginine in the animal. A reduction in plasma or serum concentrations of arginine in the animal is anticipated to be accomplishable by administering to the animal a continuous intravenous feed of the arginine-free formulation as a parenteral intravenous feed for at least 2 hours. It is anticipated that a preferred practice of using the therapeutic regimen will include the administration of the arginine-free formulation concurrently with the administration of the anti-endotoxin antibody. Simultaneous or concurrent administration is preferred primarily due to the need for the various treatments to act so as to provide the most rapid control of the hypotensive condition.

While the anti-endotoxin antibody may be administered to the animal *via* a number of routes known to those of skill in the medical arts, intravenous administration of the anti-endotoxin antibody is most preferred.

In still another aspect of the present invention, a therapeutically effective amount of an arginine-free formulation is administered concurrently with or followed by a therapeutically effective amount of an interleukin-1 receptor antagonist. Again, the parenteral formulation may be described as comprising a mixture of essential and nonessential amino acids together in a pharmacologically acceptable excipient. The parenteral formulation of this therapeutic regimen will include the amino acids and concentrations thereof already recited, and may also include ornithine or citrulline, or both.

The most preferred route of administration of the interleukin-1 receptor antagonist is *via* intravenous administration. However, other routes of administration may be used with equal efficacy in the practice of the claims therapeutic regimen. A most particularly preferred interleukin-1 receptor antagonist to be used in the present invention is IL 1ra. Most preferably, the arginine-free parenteral formulation is to be administered concurrently with the interleukin-1 receptor antagonist.

The present invention also includes a therapeutically effective amount of an arginine-free formulation to be administered concurrently with or followed by a therapeutically effective amount of an anti-tumor necrosis factor antibody. Again, the arginine-free formulation is to comprise a mixture of essential and nonessential amino acids together in a pharmacologically acceptable excipient, and is most preferably to be administered concurrently with the anti-tumor necrosis factor antibody. While the formulation may be either a parenteral or enteral formulation, parenteral formulations are most preferred.

The anti-tumor necrosis factor antibody may comprise either a monoclonal antibody or a polyclonal antibody, with the monoclonal antibody for tumor necrosis factor being most preferred. These antibodies may be prepared according to methods well known to those of skill in the art, including standard immunization protocols and/or hybridoma technologies. Tumor necrosis factor may be obtained from commercial sources for such methods. For example, tumor necrosis factor may be obtained from Amgen Biologicals (Thousand Oaks, California). By way of example, an anti-tumor necrosis factor monoclonal antibody which may be used in the practice of the invention is CB0006, which is described in Exley *et al.* (1990) Lancet, 335:1275-1277.

It is contemplated that the therapeutically effective amount of the anti-tumor necrosis factor antibody to be used in the described therapeutic regimen is 0.1 mg/kg to 20 mg/kg. Most preferably, it is anticipated that a therapeutically effective amount of anti-tumor necrosis factor will be provided to the animal at a dose of about 10 mg/kg. The most preferred route of administration of the anti-tumor necrosis factor antibody is *via* intravenous administration. Most preferably, the parenteral formulation is to be administered concurrently with the administration of the anti-tumor necrosis factor antibody, and in an amount sufficient to reduce plasma or serum concentrations of arginine.

It is contemplated that the amount of arginine-free parenteral formulation that will be sufficient to reduce plasma or serum concentrations of arginine in the animal constitutes an intravenous feed of the arginine-free parenteral formulation as described herein for at least 2 hours. In a preferred aspect, the arginine-free parenteral formulation is to be administered concurrently with the administration of the anti-tumor necrosis factor antibody.

A method for treating hypotension attendant to septic shock requires the following: administering to the animal a therapeutically effective amount of an arginine-free formulation concurrently with or prior to administering a therapeutically effective amount of an anti-endotoxin antibody, periodically monitoring blood pressure in the animal until a systolic blood pressure of at least 100 mm Hg is detectable in the animal, and maintaining the animal on the arginine-free parenteral formulation until a systolic blood pressure of at least 100 mm Hg for at least 24 hours is established. This method is anticipated to be most particularly preferred in the treatment of hypotension attendant that septic shock which is a bacterial endotoxin-related septic shock. The arginine-free formulation is most preferably prepared as a parenteral formulation.

The anti-endotoxin antibody of the described method may be either a monoclonal antibody or a polyclonal antibody, with monoclonal antibodies to endotoxin being most particularly preferred. By way of example, such an anti-endotoxin monoclonal antibody is designated HA-1A, which is described in detail in the examples included herein. A therapeutically effective concentration of the anti-endotoxin antibody as part of the herein-described method is defined as constituting a concentration of between 0.1 mg/kg to 20 mg/kg.

Another aspect of the method comprises a method for treating chemotherapeutic agent-related hypotension. In a most preferred embodiment, the method comprises monitoring an animal receiving a chemotherapeutic agent for a decrease in systolic blood pressure to less than about 100 mm Hg to detect an animal with systemic hypotension, treating the animal having systemic hypotension with a therapeutic regimen comprising a therapeutically effective amount of an arginine-free formulation sufficient to reduce plasma or serum arginine concentrations administered concurrently with or followed by the administration of a therapeutically effective concentration of an interleukin-1 receptor antagonist or an anti-tumor necrosis factor antibody, and maintaining the animal on the therapeutic regimen until an increase of systolic blood pressure to at least about 100 mm Hg is detectable. Most preferably, the arginine-free formulation is a parenteral formulation. The formulation is most preferably administered concurrently with an antagonist or antibody.

It is contemplated that the described method may be useful in the treatment of a human receiving a chemotherapeutic therapeutic agent associated with the development of hypotension. By way of example, chemotherapeutic agents associated with a decrease in blood pressure, i.e. hypotension, include tumor necrosis factor, interleukin-1 and interleukin-2.

Where the chemotherapeutic agent being administered to the animal is tumor necrosis factor, the therapeutic regimen will include the administration of an interleukin-1 receptor antagonist, such as IL 1ra. In contrast, where the chemotherapeutic agent being used is interleukin-1 or interleukin-2, the therapeutic regimen will include an anti-TNF antibody.

Ordinarily, patients receiving TNF would not preferentially receive anti-TNF antibodies. However, if severe refractory hypotension occurs, then anti-TNF antibody treatment combined with an arginine-free TPN may be useful even where TNF is the chemotherapeutic agent being used. Such constitute potential rescue strategies in treating patients with refractory hypotensive conditions.

Again, the arginine-free parenteral formulation to be used in conjunction with either the interleukin-1 antagonist or anti-tumor necrosis factor antibody includes the amino acids and concentrations thereof already described herein, together in a pharmacologically acceptable excipient. The formulation may also further include ornithine, citrulline, or both, for the reasons described herein. The therapeutically effective concentration of the interleukin-1 receptor antagonist is more particularly defined as constituting a concentration of between 1 mg/kg to 100 mg/kg. The anti-tumor necrosis factor antibody most preferred for use in the described method is a monoclonal antibody, and will constitute a therapeutically effective concentration thereof of between 0.1 mg/kg to 20 mg/kg.

A method for treating septic shock-related hypotension in an animal exposed to endotoxin, in one preferred embodiment, comprises treating the animal with a therapeutically effective amount of an arginine-free formulation sufficient to reduce plasma or serum arginine levels in the animal concurrently with an interleukin-1 receptor antagonist or an anti-endotoxin antibody, or both, monitoring the blood pressure of the animal, and maintaining the animal on the arginine-free parenteral formulation until a systolic blood pressure of at least 100 mm Hg is detected. Most preferably, the formulation is a parenteral formulation.

A method for treating hypotension in an animal with endotoxic related septic shock or cytokine-induced shock comprises administering to an animal a therapeutically effective amount of an arginine-free formulation sufficient to reduce plasma or serum arginine levels in the animal, administering the formulation concurrently with or prior to the administration of a therapeutically effective amount of an anti-tumor necrosis factor antibody, monitoring blood pressure of the animal, and maintaining the animal on the arginine-free formulation until a systolic blood pressure of at least 100 mm Hg is detected. While the anti-tumor necrosis factor antibody may be a monoclonal or a polyclonal antibody, monoclonal antibodies are most preferred. Again, the arginine-free formulation is most preferably a parenteral formulation.

The arginine-free parenteral formulation of the method includes the amino acids and amounts thereof previously described herein together in a pharmaceutically acceptable excipient. Again, the formulation may include ornithine, citrulline, or both. It is contemplated that the therapeutically effective amount of the anti-tumor necrosis factor antibody to be used in conjunction with the described method constitutes an amount of between 0.1 mg/kg to 20 mg/kg body weight of the animal. Of this range, a dose of about 10 mg/kg of the anti-tumor necrosis factor may constitute the most effective anti-hypotensive dose when used in conjunction with the arginine-free parenteral formulation. So implemented, it is anticipated that the herein described methods may be useful in the treatment of endotoxin-related septic shock or cytokine-induced shock in a human.

While the anti-tumor necrosis factor antibody or interleukin-1 receptor antagonist may be administered to the animal *via* any route known to those of skill in the medical arts, it is most preferred that these agents be administered intravenously, most preferably as a separate bolus dose to the animal. The aforedescribed method is anticipated to provide a most preferred embodiment for treating cytokine-induced shock in an animal.

Notwithstanding the accumulated evidence supporting synthesis of NO, it is understood by those skilled in the art that other nitrogen oxides may be present and may be active in reducing blood pressure. Within this specification, the acronym NO· will be understood to represent nitric oxide and any additional vasoactive nitrogen oxides.

Abbreviations used in the drawings and other places in the present disclosure include the following.
ACh = acetylcholine
BAEC = bovine aortic endothelial cells
B.P. = blood pressure
CO = Cardiac output
EDRF = Endothelium-Derived Relaxing Factor
ET = endotoxin
GP = guinea pig
HIST = histamine
IFN = gamma-interferon
IV = Intravenous
L-Arg = L-arginine
LPS = lipopolysaccharide (endotoxin)
L-NMA = L-NMMA = N^{w}-methyl-L-arginine
MAP = mean arterial pressure
MBEC = murine brain endothelial cells
NAA = N^{w}-L-amino-L-arginine
NNA = N^{w}-nitro L-arginine
NO = Nitric Oxide
PAF = Platelet Activating Factor
PPS = Platelet - poor, plasma-derived serum
SAP = Systemic arterial pressure
SNP = sodium nitroprusside
SVR = Systemic vascular resistance
TNF = Tumor Necrosis Factor
TPN = Total Parenteral Formulation

### BRIEF DESCRIPTION OF THE DRAWINGS

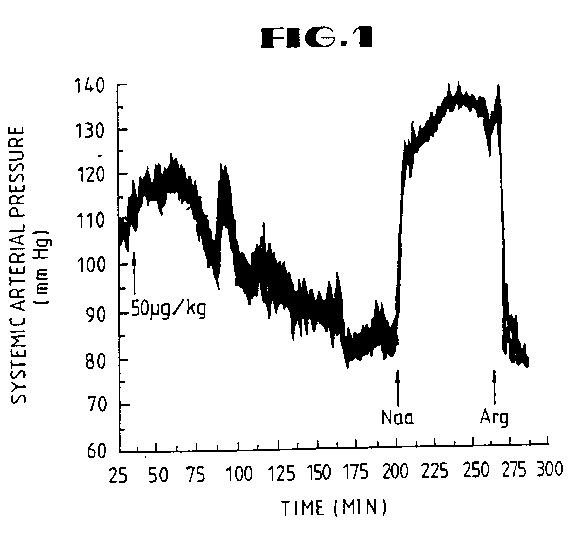

FIGURE 1 - Reversal of hypotension associated with the administration of IL-1 to dogs. The tracing shown is a representative experiment that was repeated four times. Each dog was anesthetized and incubated as described herein, and baseline cardiovascular data were monitored for 30 minutes. IL-1 was dissolved in phosphate-buffered saline (pH 7.4) containing 1 mg/ml of dog albumin, and the resulting solution was infused intravenously for three minutes to deliver a dose of 50 µg/kg. MAP was continuously monitored; when a stable nadir was reached and maintained for more than 10 minutes or when MAP dropped below 50 mm Hg, NAA (20 mg/kg in 10 ml phosphate-buffered saline) was administered by intravenous infusion for 30 seconds. Sixty minutes after MAP was restored to baseline or higher values, L-arginine (400 mg/kg in 20 ml phosphate-buffered saline) was administered by slow intravenous infusion for 5 minutes. Anesthetized dogs given IL-1 (50 µg/kg) developed a progressive, moderately severe hypotension, with MAP decreasing from a baseline of 111 +/- 7 mm Hg to 80 +/- 2 mm Hg within about 3 hours. Hypotension was rapidly reversed by the intravenous administration of a single bolus dose of NAA (20 mg/kg, 88 umol/kg), with MAP increasing by from 46 +/- 9 mm Hg (58%_ to 125 +/- 8 mm Hg.

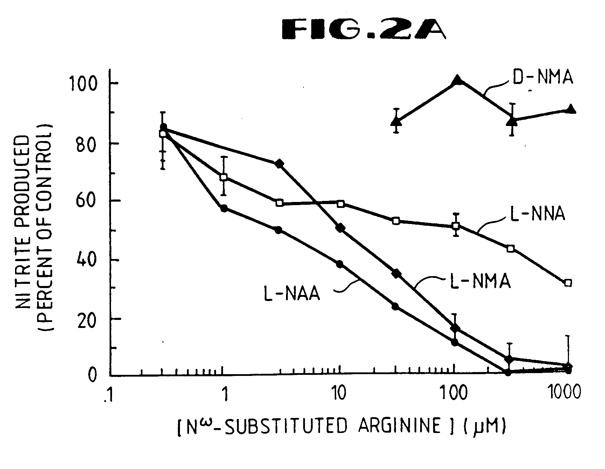

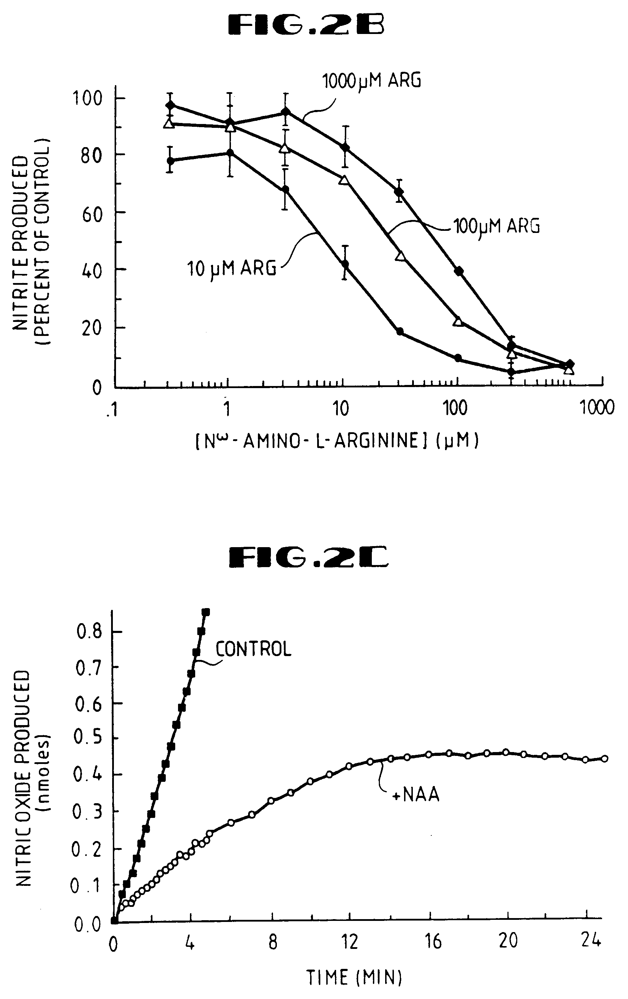

FIGURES 2A, 2B, 2C - Inhibition of IL-1-activated NO· production by N^{ω}-substituted arginine analogues. Figure 2A: Comparison of nitrite synthesis inhibition in smooth muscle cells by the L-isomers of NAA, NMA, and NNA and by the D-isomer of NMA. Figure 2B: Concentration dependence of nitrite synthesis inhibition in smooth muscle cells by NAA as a function of different L-arginine concentration in the culture medium. Confluent monolayers of smooth muscle cells in 96-well microtiter plates were treated with a combination of 100 ng/ml IL-1 and 50 ng/ml IFN-γ for 24 hours. Cells were then washed with Hanks' balanced salt solution, and fresh medium containing the indicated concentration of L-arginine was added. Points indicate means ± SD of nitrite production, expressed as percent of control, during a 16-hour exposure to arginine analogues (n = 3-4). Figure 2C: Effect of NAA on smooth muscle cell NO· synthase. Cytosol from cytokine-induced smooth muscle cells (∼2 µg protein) was incubated with 1 mM L-arginine, 0.5 mM NADPH, 1 mM dithiothreitol, 10 µM flavin-adenine dinucleotide, 10 µM tetrahydrobiopterin, and 0.1 U/ml dihydropteridine reductase in a total volume of 100 µL. The time course of Fe²⁺-myoglobin oxidation was measured in the absence (control) or presence of 300 µM NAA (+NAA). The inventors have also observed that myoglobin oxidation showed an absolute dependence on L-arginine and NADPH. Points indicate the time course of change in rate of NO· synthesis in a representative experiment which was repeated three times.
FIGURE 3 - Illustrates the effects of the sequential administration of NMMA and L-arginine on moderately low blood pressure of a dog treated with TNF.
FIGURE 4A - Illustrates the effects of sequential administration of NMMA and L-arginine on severe hypotension in a canine administered TNF.
FIGURE 4B - Illustrates control experiments where NMMA was administered to previously untreated dogs.
FIGURE 4C - Illustrates the effects of NMMA on nitroglycerin-induced canine hypotension.
FIGURE 5 - Shows the time course of changes in mean systemic arterial pressure (SAP) in a pentobarbital-anesthetized dog following the IV administration of endotoxin (LPS; Lipopolysaccharide) (ET), N^{ω}-methyl-L-arginine (L-NMA), and L-arginine (L-Arg).
FIGURE 6 - Shows the time course of changes in mean systemic arterial pressure (SAP) in a pentobarbital-anesthetized dog following the i.v. administration of endotoxin, L-NMA (2 doses) and L-Arg.
FIGURE 7 - Shows the time course of TNF-mediated canine systemic hypotension and reversal by Nω-aminoarginine.
FIGURE 8 - Shows the reversal of endotoxin-induced systemic hypotension by N^{ω}-aminoarginine (3 doses).

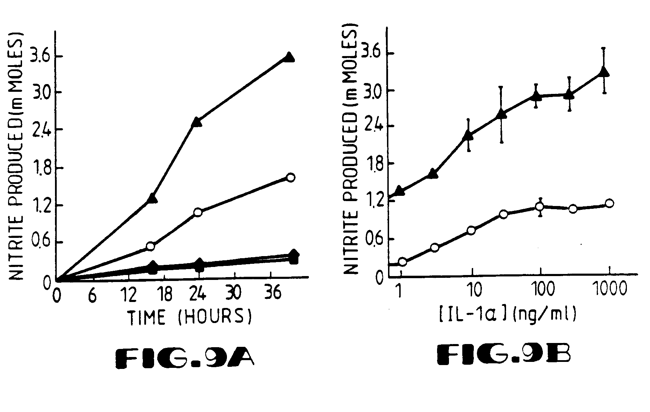

FIGURE 9A-B - IL-1 activates nitrite production by rat aortic smooth muscle cells in culture. **FIGURE A:** Time course of nitrite synthesis elicited by 40 ng/mL IL-1 alone (open circles) and by IL-1 in the presence of 50 ng/ml IFN-γ (open triangles). In the absence of IL-1, IFN-γ did not elicit nitrite production. Inclusion of IL-1 receptor antagonist (40 µg/mL; filled symbols) in the culture medium inhibited nitrite production stimulated by both IL-1 and IL-1 plus IFN-γ. **FIGURE B:** IL-1 concentration dependence for smooth muscle cell nitrite production. Data points indicate nitrite produced during a 29-hour incubation with the indicated concentration of IL-1 alone (open circles) or in the presence of IL-1 plus 50 ng/mL IFN-γ (open triangles). All values are means ± SD for nitrite production by confluent smooth muscle cell monolayers grown in 96-well microtiter plates (60-80 X 10³ cells per well; n = 3-4).

### DESCRIPTION OF THE PREFERRED EMBODIMENTS

Clinical studies of biologic response modifiers such as certain cytokines have shown that a major dose-limiting toxicity associated with administration of such agents is hypotension. Nitrite, the predominant spontaneous oxidation product of NO, is readily assayed and used herein for assays of NO production. Nitric oxide (NO) is a highly reactive compound which spontaneously decomposes to nitrate and nitrite in a culture medium.

The present invention implements the inventors' findings that IFN (100 U/ml) in combination with either TNF (500 U/ml), IL-1 (10 U/ml), or endotoxin (1 µg/ml.), can induce MBEC's to accumulate nitrite in a culture medium (15 to 80 µM) within 48 hours. TNF, IL-1 or endotoxin alone was found to induce the production of minimal levels of nitrites (1-3 µM).

The release of vasoactive factors such as NO by endothelial cells may play a role in the development of hypotension associated with the administration of these agents *in vivo*. This invention relates to a demonstration that cultured MBEC's produce NO in response to various combinations of cytokines and the potential role of NO in the pathophysiological causes of hypotension and cardiovascular collapse.

The present inventors have found that increased concentrations of nitrites were not associated with MBEC exposed to TNF and IFN in arginine-free culture medium. The nitrite concentration was found to increase in a dose-dependent manner upon addition of L-arginine back to the medium of a culture of MBEC cells. The inventors have also found that nitrite accumulates when cultured mouse endothelial cells are exposed to immunomodulators and endotoxin¹⁷.

The present invention relates to the use of a specific formulation for the manufacture of medicament for inhibiting or preventing hypotension as well as septic shock through the reduction of nitric oxide synthesis. Most particularly, an arginine-free parenteral formulation is administered to effect a reduction in serum arginine levels. This reduction in serum arginine levels is postulated to provide for a reduction in nitric oxide synthesis, as arginine is the substrate from which nitric oxide is formed. Nitric oxide has recently been demonstrated by the inventors to be derived from the amino acid L-arginine.

As nitric oxide is a potent vasodilator linked to the development of hypotension and septic shock, the claimed methods propose the inhibition and prophylaxis of these conditions in an animal through the control of serum arginine concentrations and nitric oxide synthesis. The invention provides a reduction in nitric oxide synthesis by eliminating or limiting the arginine concentration in an animal's serum. A reduction in an animal's serum concentration of arginine is accomplished through restricting the amount of arginine given to the animal to not more than 0.1% arginine in a non-hypotensive TPN formulation.

Particular embodiments of the claimed formulations may include ornithine and citrulline, ornithine alone, or citrulline alone to supplement urea cycle substrate physiological requirements in the animal.

For purposes of the present invention, the term "cytostatic" is defined as that physiological state of a cell characterized by a lack of active cell division. Thus, a culture or group of cells which are in a "cytostatic" state are not actively dividing or which are growth inhibited (i.e., virtually no cell growth).

The terms "anti-hypotensive" and "non-hypotensive" are used interchangeably in defining the present invention. These terms are intended to denote the nature of the formulation as limiting the onset of hypotension through decreased arginine concentrations and thus availability in the serum of arginine.

For purposes of the present invention, a "pressor" agent is defined as a pharmacological agent which causes an increase in blood pressure when administered to an animal. By way of example, phenylephrine is a "pressor" agent. However, other pressor agents would be expected to provide similar effects in the systems described herein. Other examples of pressor agents include dopamine, epinephrine, norepinephrine and phenylephrine.

The present invention also provides for the use of a therapeutically useful formulation for the manufacture of a medicament for treating hypotension, such as that attendant septic shock or treatment with certain chemotherapeutic agents such as TNF, IL-1 or IL-2. The various formulations described in conjunction with the present invention include the arginine-free parenteral formulation. The formulation is to be administered either concurrently with or followed by the treatment of the animal with an arginine analog capable of inhibiting nitric oxide-production. Agents which are capable of inhibiting nitric oxide-production include arginine analogs capable of inhibiting nitric oxide synthase, and include NMA, NAA, NNA, or a mixture thereof.

Human recombinant IL-1-α (hereafter referred to as IL-1; specific activity, 2 x 10⁷ lymphocyte-activating factor units/mg) was produced by Dainippon Pharmaceutical Co. LTD. and provided by the National Cancer Institute. Human recombinant IL-1 receptor antagonist was produced by Synergen, Co. and was provided by Drs. Lyle Moldawer and Stephen Lowry, Department of Surgery, Cornell University Medical College. Rat interferon-γ (IFN-γ) was obtained from Amgen Biologicals (Thousand Oaks, CA).

N^{ω}-methyl-L-arginine (NMA) and NAA, hydrochloride salt, were synthesized as reported previously by Gross *et al.* (1990) (*Biochem. Biophys. Res. Commun., 170*:96-103) and Corbin (1974) (*Anal. Biochem., 57*:310-312). Except where otherwise indicated, all biochemical reagents were obtained from Sigma Chemical Co. (St. Louis, MO). Cell culture media and reagents, unless otherwise noted, were from Whittaker Bioproducts (Walkersville, MD).

### Cell Culture

Mouse A375 melanoma cells were provided by Dr. E. Kleinerman, The University of Texas M.D. Anderson Cancer Center. Cells were maintained in Dulbecco's modified Eagle medium and Ham's F-12 medium (1:1) containing 10 m*M* HEPES buffer (pH 7.4) and 10% fetal bovine serum. All tissue culture reagents contained less than 0.25 ng/ml endotoxin as measured by the limulus amebocyte assay. Murine D10 T cells were obtained from the American Type Culture Collection (Rockville, MD).

Aortic smooth muscle cells were cultured by explanting segments of the medial layer of aortas from adult male Fischer 344 rats. Aortas were removed aseptically and freed of adventitial and endothelial cells by scraping both the luminal and abluminal surfaces. Medial fragments were allowed to attach to Primaria 25-cm² tissue culture flasks (Becton-Dickinson, Lincoln Park, NJ) which were kept moist with growth medium until cells emerged. Cultures were fed twice weekly with medium 199 containing 10% fetal bovine serum, 25 m*M* HEPES buffer (pH 7.4), 2 m*M* L-glutamine, 40 µg/ml endothelial cell growth supplement (Biomedical Technologies, Inc. (Stoughton, MA), and 10 µg/ml gentamicin (GIBCO). When primary cultures became confluent, they were passaged by trypsinization, and explants were discarded. For these studies, cells from passages 12-14 were seeded at 20,000/well in 96-well plates and were used at confluence (60,000-80,000 cells/well). The cells exhibited the classic SMC phenotype with hill and valley morphology and stained positively for smooth-muscle actin.

### Cell Respiration Assay

Rat aortic smooth muscle cells in 96-well microtiter plates were incubated for 90 minutes in RPMI-1640 medium containing 0.2 mg/ml 3-(4,5-dimethylthiazol-2-yl)-2,5-diphenyltetrazolium bromide (MTT), washed with Hands' balanced salt solution, and solubilized in 100 µl of dimethyl sulfoxide. The extent of reduction of MTT to formazan within cells, quantitated by measurement of the optical density at 550 nm (OD₅₅₀), was taken as an indicator of cellular respiration. The assay is more thoroughly described by Klostergaard *et al*. (1987) (*J. Immunol. Methods, 101*:97-108).

### IL-1-Induced Cell Proliferation Assay

Murine D10 cells, an IL-1-dependent T-cell line, were used to measure IL-1 mitogenic activity. Cell proliferation in the presence of IL-1 was assessed by incorporation of [³H]thymidine as previously described by Bakouche *et al*. (1987) (*J. Immunol., 138*: 4249-4255).

### IL-1-Induced Cytotoxicity Assay

IL-1-induced cytotoxicity was studied using A375 tumor cells plated at a density of 6000 cells per well in 96-well microtiter plates. After overnight attachment, IL-1 (3-300 ng/ml) was added in the presence or absence of NAA or NMA. After cells were incubated for 3 days, [³H]thymidine was added (1 µCi per well) for an additional 2 hours. Cells were harvested onto glass fiber disks (PHD Cell Harvester; Cambridge Technology, Inc., Watertown, MA). Disks were air dried overnight, and radioactivity was determined with a Model 1900TR Scintillation Counter (Packard Instrument Division, Downers Grove, IL).

### Induction and Assay of Nitrite Synthesis in Smooth Muscle Cells

Rat aortic smooth muscle cells were incubated with RPMI-1640 medium containing 10% bovine calf serum, 25 m*M* HEPES buffer (pH 7.4), 2 m*M* glutamine, 80 U/ml: fungizone, and IL-1, IFN-γ, and various inhibitors at the concentrations indicated in the figure legends. At the desired times, nitrite concentration in the culture medium was measured using the standard Griess assay adapted to a 96-well microtiter plate reader.³⁶ The Griess assay is also described in Gross *et al*.³⁷ (1991). Thus, 100 µL of Griess reagent (0.5% sulfanilic acid, 0.05% naphthalenediamine, and 2.5% phosphoric acid) was added to an equal volume of culture medium, and the OD₅₅₀ was measured and related to nitrite concentration by reference to a standard curve. The background OD₅₅₀ of medium incubated in the absence of cells was subtracted from experimental values.

### Preparation and Assay of Smooth Muscle Cell NO· Synthase

Rat aortic smooth muscle cells were incubated with RPMI-1640 medium containing 10% bovine calf serum, 25 m*M* HEPES buffer (pH 7.4), 2 m*M* glutamine, 80 µg/ml penicillin, 80 µg/ml streptomycin, 2 µg/ml fungizone, 30 µg/ml lipopolysaccharide (*Escherichia coli* 0111:B4), and 50 U/ml IFN-γ. Cells were harvested after 24 hours, and cytosol was prepared as described in the above cited Gross *et al*. (1991)⁴¹ article. Cytosolic NO· synthase activity was assayed by the Fe²⁺-myoglobin method described previously.

### Studies of IL-1-Induced Hypotension in Dogs

Experiments were conducted on healthy mongrel dogs that were free of microfilaria and weighed 25-28 kg each. All protocols were approved by The University of Texas Animal Welfare Committee, and animal care met all standards prescribed in the "Guide for the Care and Use of Laboratory Animals" (Department of Health, Education and Welfare, Guide for the Care and "Use of Laboratory Animals Pub. No. 78-23, Washington, DC, Health Department (1978). After an overnight fast, the dogs were anesthetized with pentobarbital (25 mg/kg given intravenously), orotracheally incubated, and ventilated with an animal respirator (Harvard Apparatus, South Natick, MA), using room air at a tidal volume of 15 ml/kg delivered at 14 breaths per minute and adjusted to normal pH and CO₂ tension. A catheter was placed percutaneously in the femoral artery, and a flow-directed thermodilution catheter was inserted through the jugular vein and positioned with the distal port in the pulmonary artery. Mean systemic arterial pressure (MAP), heart rate, cardiac output, and systemic vascular resistance were measured as previously described in Kilbourn *et al*. (1990) (*Biochem. Biophys. Res. Commun., 172*:1132-1138).

The therapeutic regimens and methods described herein by the inventors provide a novel and potentially more effective method for treating a variety of conditions where hypotension is a typical consequence. For example, hypotension is typically seen in patients suffering from septic shock and in those receiving certain chemotherapeutic agents (TNF, interleukin-1, interleukin-2). The innovative combination of an arginine-free parenteral formulation together with anti-endotoxin antibodies, anti-tumor necrosis factor antibodies, and/or interleukin-1 receptor antagonist may allow for the use of chemotherapeutic agents without the risk of the development of life-threatening hypotension in the animal.

Recombinant IL-1 or TNF may be obtained from Amgen (Thousand Oaks, California) and used in standard immunization techniques to provide polyclonal antibodies to these antigens. Alternatively, spleen or other tissue from the immunized animals may be used to prepare hybridoma cell lines when fused with an immortal cell line. Such hybridomes constitute a source of monoclonal antibodies specific for their respective antigens.

The treatment of hypotension, such as in a patient or animal with systemic sepsis, involves that the following symptoms will be monitored: fever or hypothermia (temperature > 38.3°C [101°F] or <35.6°C [96°F]; tachycardia (>90 beats per minute in the absence of beta-blockade) and tachypnea (respiratory rate >20 breaths per minute or the requirement of mechanical ventilation); and either hypotension (systolic blood pressure ≤90 mm Hg or a sustained drop in systolic pressure ≥40 mm Hg in the presence of an adequate fluid challenge and the absence of antihypertensive agents) or two of the following six signs of systemic toxicity or peripheral hypoperfusion: unexplained metabolic acidosis (pH ≤7.3 base deficit of >5 mmol per liter, or an elevated plasma lactate level); arterial hypoxemia (partial pressure of oxygen ≤75 mm Hg or ratio of the partial pressure of oxygen to the fraction of inspired oxygen <250); acute renal failure (urinary output of less than 0.5 ml per kilogram of body weight per hour); elevated prothrombin or partial-thromboplastin time or reduction of the platelet count to less than half the base-line value or less than 100,000 platelets power cubic millimeter; sudden decrease in mental acuity; and cardiac index of more than 4 liters per minute per square meter of body-surface area with systemic vascular resistance of less than 0.8 kg/s·cm⁴ (800 dyn sec cm⁻⁵).

### EXAMPLE 1

### Correlation Between Serum Arginine Levels and Blood Pressure

The present example is provided to demonstrate the correlation between plasma arginine levels and blood pressure. More specifically, the present example demonstrates a correlation between low plasma arginine levels and increased blood pressure in endotoxin-treated animals.

The present example also demonstrates the utility for employing a parenteral formulation which is essentially arginine free or low enough in arginine to lower plasma arginine levels, to prevent or alleviate synthesis of nitric oxide, which in turn is shown to result in an increase in blood pressure. Maintenance of an animal on an arginine-free parenteral formulation and a nitric oxide synthase inhibitor, such as the arginine analogs NMA, NAA or NNA or a combination thereof, would therefore, according to the present invention, provide a method for even further augmenting the anti-hypotensive effect of both components of such a regimen, using significantly lower doses of arginine analogs than would otherwise be required in animals with normal or high physiological concentrations of arginine. The hypertensive effect of NAA and other nitric oxide inhibitors *in vivo* alone is also described in the present disclosure. The onset of life-threatening levels of low blood pressure, such as that typically attendant to cytokine-induced hypotension and septic shock would thus be prevented or alleviated in an animal. The onset of life-threatening levels of low blood pressure, such as that typically attendant to hypotension and septic shock would thus be prevented or alleviated in an animal.

The enzyme arginase was used to reduce plasma arginine levels in Spraque-Dawly rats (Weight per rat = 250-300 gm). Arginase is an enzyme that irreversibly converts L-arginine to L-ornithine + urea. The rats were anesthetized with ethyl ether and then pithed as described Shiply and Tilden³⁰. The animals were pithed prior to use in the present study so as to eliminate any neurological control of blood pressure.

Arginase was dissolved in sterile saline (1000 I.U./ml) and was administered by intravenous infusion at a rate of 300 I.U./min. for 20 min. One I.U. is the amount of arginase that converts 1 µmol of arginine to products per minute. Blood pressure was determined using a pressure transducer connected to an indwelling catheter placed in the carotid artery as described.¹²

### Serum arginine concentrations

The administration of arginase to pithed rats with or without exposure to endotoxin (15 mg/kg dose, ip), according to the dose outlined above, resulted in a decrease in plasma arginine levels of from 150 µM to ≤ 4 µM within a few minutes. Plasma arginine remained at levels ≤ 4 µM for at least 1 hour after the arginase infusion was stopped.

### Blood pressure recording in the pithed rat.

To record blood pressure, a tracheotomy was first performed on each rat, after which the rats were artificially respired with room air. The left common carotid artery was then cannulated in each rat for blood pressure measurement *via* a Statham pressure transducer (Hato Rey, Puerto Rico) and displayed on a physiogram (Grass Instruments, Qunicy MA). Heart rate was measured from the lead III electrocardiogram.

Two separate groups of animals were examined. The first group of animals, designated the "control" group, received no endotoxin. The blood pressure of the "control" group animals was measured at two different times, once before the administration of arginase and once after the administration of arginase.

The second group of animals, designated the endotoxin group, received a single dose of endotoxin of 15 mg/kg body weight, which was administered at least 6 hours prior to any subsequent arginase treatment. The blood pressure of all animals in both treatment groups was then measured at two different times, again once before arginase treatment and once after arginase treatment.

The results from this example are presented in Table 1.

**TABLE 1**

| **EFFECT OF REDUCED PLASMA ARGINASE ON BLOOD PRESSURE** | | | | |
|---|---|---|---|---|
| Control Rats | No Arginase | | Arginase | |
| | B.P.(mm Hg) | Average B.P. | B.P.(mm Hg) | Average |
| 1 | 61 | 59.8 ± 1.3 | 61 | 63.25 |
| 2 | 60 | | 60 | |
| 3 | 60 | | 64 | |
| 4 | 58 | | 68 | |

| Endotoxin Rats (15 mg/Kg) | No Arginase | | Arginase | |
|---|---|---|---|---|
| | B.P.(mm Hg) | Average B.P. | B.P.(mm Hg) | Average |
| 1 | 36 | 33.2 ± 3.3 | 44 | 38.4 |
| 2 | 34 | | 40 | |
| 3 | 28 | | 28 | |
| 4 | 32 | | 36 | |
| 5 | 36 | | 44 | |

Blood pressure readings for 4 control pithed rats were 61, 60, 60, and 58 mm Hg (average 59.8 ± 1.3 mm Hg) (See Table 1). Following administration of arginase, blood pressure was unchanged in two rats and increased by 4 and 10 mm Hg in 2 other rats (average increase 3.5 ± 4.7 mm Hg, not statistically significant).

Blood pressure readings for 5 rats at 6 hours after giving 15 mg/kg lipopolysaccharide (endotoxin) by intravenous injection was 36, 34, 28, 32, and 36 mm Hg (average 33.2 ± 3.3 mm Hg, See Table 1). Note that the endotoxin-treated rats were clearly hypotensive relative to the controls.

Following administration of arginase, blood pressure in the endotoxin-treated rats increased by 8, 6, 0, 4, and 8 mm Hg (average increase 5.2 ± 3.3 mm Hg). The average blood pressure increase following arginase treatment of the endotoxic, pithed rats was 15.7% (statistically significant, p < 0.05).

Overall, this study shows that reducing plasma arginine levels has no significant effect on blood pressure in control animals, but did have a significant effect on blood pressure readings in endotoxic animals. The lack of a demonstrated effect in control animals may be due to the slow rate of NO formation in control animals, so as to negate any requirement for exogenous (i.e. plasma) arginine. Thus, a reduction in plasma arginine levels in such animals would not be a limiting factor for generating NO.

In contrast, the rate of NO formation in endotoxic animals is much faster than in control (non-endotoxic animals), and results in the development of hypotension. In these endotoxic animals, the cells making NO must obtain extra arginine from the plasma. When plasma arginine is very low in endotoxic animals (i.e. after arginase administration), there is not enough arginine available to sustain a pathologically high rate of NO synthesis by cells associated with blood vessel walls (i.e., endothelial cells). Thus, the concentration of NO is reduced, resulting in a concomitant reduction in the level of blood pressure reduction in the vasculature of the animal. Thus, depletion of serum arginine levels could be used to effect an increase in blood pressure in hypotensive animals.

Thus, use of arginine-free TPN solutions, or solutions sufficiently low in arginine concentration so as to effect a sufficient reduction in plasma arginine levels adequate to limit nitric oxide synthesis, for example, to about 4 µM arginine or less (i.e., 4 nM/ml serum arginine), are expected to have a beneficial effect comparable to that of arginase administration for preventing or treating hypotension, particularly hypotension in animals in septic shock.

### EXAMPLE 2

### CORRELATION BETWEEN PLASMA ARGININE LEVELS AND RESPONSE TO PRESSOR AGENTS

The present example is provided to demonstrate the correlation between low plasma arginine levels and increased response to "pressor" agents *in vivo* in endotoxin-treated animals.

Animals were pithed as described in Example 1. Blood pressure measurements were obtained also as described in Example 1. Arginase was also prepared according to the method described in Example 1.

Both left and right jugular veins were cannulated for drug administration; and left jugular was used for bolus administration of phenylephrine and the right jugular vein was used for continuous infusion of arginase. All animals from both groups (Control and Endotoxin) received phenylephrine in sequential doses of 0.3, 1.0, 2.0, or 6.0 ug/Kg.

The results from this example are presented in Table 2.

**TABLE 2**

| **EFFECT OF ARGINASE AND ENDOTOXIN ON BLOOD PRESSURE** | | | | |
|---|---|---|---|---|
| **Phenylephrine dose** | **Blood Pressure Increase (mm Hg)** | | | |
| | **Without Arginase** | | **With Arginase** | |
| | **Raw Data** | **Ave. ± S.D.** | **Raw Data** | **Ave. ± S.D.** |
| | | | | |

| **Study #1: Control Animals** | | | | |
|---|---|---|---|---|
| 0.3 µg/Kg | +16,14,32,9 | 17.8 ± 5.0 | +4,9,20,10 | 13.3 ± 2.5 |
| 1.0 µg/Kg | +40,25,36,24 | 31.3 ± 4.0 | +12,17,40,21 | 22.5 ± 6.1 |
| 2.0 µg/Kg | +64,48,58,34 | 51.0 ± 6.6 | +28,30,66,31 | 38.8 ± 9.1 |
| 6.0 µg/Kg | +84,92,122,74 | 93.0 ± 10.3 | +64,72,120,63 | 79.8 ± 13.4 |

| **Study #2: Endotoxic Animals** | | | | |
|---|---|---|---|---|
| 0.3 µg/Kg | +2,0,4,2,3 | 2.2 ± 0.7 | +2,0,4,2,2 | 2.0 ± 0.6 |
| 1.0 µg/Kg | +6,4,9,10,6 | 7.0 ± 1.1 | +8,6,16,9,6 | 9.0 ± 1.8 |
| 2.0 µg/Kg | +12,10,24,12+10 | 13.6 ± 2.6 | +19,14,44,20,20 | 23.3 ± 5.3 |
| 6.0 µg/Kg | +25,26,72,26,20 | 33.7 ± 9.6 | +36,46,78,44,38 | 48.5 ± 7.6 |

This example shows the effects of endotoxin and of arginase on mean systolic blood pressure response to phenylephrine in pithed rats. Endotoxin (15 mg/kg body weight) was given by intravenous injection 6 hrs before the experiment began; arginase (300 I.U./Min. for 20 min.) was given intravenously to each rat after the "Without Arginase" measurements were made.

Table 2 shows the maximum increase in blood pressure following the phenylephrine dose indicated for each rat ("Raw Data"); note that the data is in pairs since each rat was tested first without arginase (at 0.3, 1.0, 2.0 and 6.0 ug/Kg phenylephrine, in sequence) and was then retested with phenylephrine in the same dose and sequence after arginase treatment. Thus, the first line of data in the Table shows that four control rats were each tested with 0.3 µg/Kg of phenylephrine. The first control rat showed a blood pressure of 16 mm Hg without arginase, but only 4 mm Hg after arginase administration ("with arginase"). For the second, third and fourth rats, the blood pressure increments were 14, 32 and 9 before arginase, respectively, and 9, 20 and 10 after arginase, respectively.

The data in Table 2 demonstrates that reducing plasma arginine levels through arginase treatment, enhances the "pressor" agent (such as phenylephrine), response in endotoxic animals, reducing the difference observed between endotoxic and control animal blood pressure increases at the same pressor agent dose. Moreover, endotoxic animals pretreated with arginase demonstrated an enhanced "pressor" effect (a statistically greater increase in blood pressure), compared to the pressor response observed in endotoxic animals receiving no arginase (See Table 2).

Endotoxin decreases an animals ability to present the normal hypertensive response (i.e., increase in blood pressure) to phenylephrine. Thus, compare the "Without Arginase" data of control and endotoxic rats at each dose of phenylephrine (Table 2). This effect occurs because the endotoxic animals are making large amounts of nitric oxide from arginine, and that causes hypotension and blunting of the response to phenylephrine.

Arginase administration improves the hypertensive response to a pressor agent, such as phenylephrine, dose. Smaller differences were observed between control and endotoxic animals given arginase (indicating only a small loss of responsiveness) relative to the larger differences between control and endotoxic animals not given arginase (indicating a large loss in responsiveness).

For example, at a phenylephrine dose of 6 µg/Kg, in animals not given arginase (i.e., having a higher serum arginine concentration), the pressor response to phenylephrine drops from 93.0 ± 10.3 mm Hg (a pharmacologically useful pressor agent response) in control animals to 33.7 ± 9.6 mm Hg (a poor pressor agent response) in endotoxic animals, a difference in pressor response of 59.3 mm Hg. In contrast, at the same phenylephrine dose in animals given arginase (i.e., decreased serum arginine levels), the pressor response in control and endotoxic animals was 79.8 ± 13.4 and 48.5 ± 7.6, respectively, a difference of only 31.3 mm Hg. Thus, depletion of plasma arginine with arginase very significantly restores the "normal" (hypertensive) response to pressor drugs, such as phenylephrine.

The data herein thus demonstrates that hypotension may be controlled, particularly in endotoxic animals, by manipulating an animal's serum arginine concentration. As serum arginine levels may be controlled in part through an animal's nutrition as they are through arginase administration, the present data provides a mechanism whereby hypotension (low blood pressure) may be corrected by maintaining the animal on a low/anti-hypotensive concentration or essentially arginine-free nutritional regimen. As such, the presently disclosed technique may also be used to prevent the development of hypotension in a patient at risk, either through administration of the TPN formulation alone, or through administration of a combination therapy with a pressor agent or one of the nitric oxide inhibitors, such as NMA, NNA, or a combination, thereof, as described herein.

### EXAMPLE 3

### Anti-hypotensive Arginine-free TPN formulation

The present example defines an anti-hypotensive TPN formulation of the present invention.

A sterile, non-pyrogenic, stable solution for parenteral administration to a patient having hypotension or at risk of hypotension or systemic shock, particularly those receiving immunomodulatory agents, is prepared from pure crystalline amino acids, which are dissolved in a glucose solution (5% to 20%) in the following concentrations to provide a 2X concentrate TPN or a ready-to-feed TPN formulation, as indicated:

**TABLE 3**

| Amino Acids | 2x concentrate mg/100 ml formulation | Final Concentration (Feeding Formulation)g/l |
|---|---|---|
| isoleucine | 600-800 | 3-4 |
| leucine | 800-1200 | 4-6 |
| valine | 600-800 | 3-4 |
| phenylalanine | 200-400 | 1-2 |
| methionine | 200-400 | 1-2 |
| lysine | 600-800 | 3-4 |
| histidine | 200-400 | 1-2 |
| threonine | 400-600 | 2-3 |
| tryptophan | 100-300 | 0.5-1.5 |
| tyrosine | 50-150 | 0.25-0.75 |
| alanine | 800-1000 | 4-5 |
| aspartic acid | 400-600 | 2-3 |
| glycine | 800-1000 | 4-5 |
| proline | 600-800 | 3-4 |
| serine | 200-400 | 1-2 |

To obtain the preferred TPN formulation concentration suitable as a feeding formulation, a volume of 500 ml of the 2X concentrate (defined in Table 3) is mixed with 500 ml of a dextrose solution, for the production of 1 liter of the feeding formulation (i.e., 500 ml of a 2X concentrate of AA and 500 ml of dextrose solution). Most preferably, the dextrose solution is supplemented with a physiologically acceptable concentration of vitamins and minerals.

The TPN of the present invention may also include glutamic acid (400-600 mg/100 ml of a 2x conc., or 2-3 g/l in a final concentration) and/or taurine (50-100 mg/100 ml of a 2-fold concentrate; 0.25-0.5 g/l final concentration).

The solution is then filter-sterilized into appropriate containers for intravenous fluids. To prepare for administration, the volume is then brought to the desired feeding solution concentration with an equal volume of sterile glucose solution. The TPN as a ready to feed formulation is then to be kept cool. The solution may then be administered to a patient intravenously (I.V.). The pH of the TPN solution must also be adjusted to a physiologically acceptable pH, between 7.0 and 7.4. The formulation is arginine-free.

### Example 4

### Anti-Hypotensive Formulation with Low Arginine

If a formulation of amino acids for a patient having hypotension, or at risk of developing hypotension or septic shock, is desired which includes arginine, the formulation as outlined in Example 3 may be utilized after supplementation with arginine. Arginine will be added to constitute not more than 0.1% final concentration by weight of the formulation (≤1 g of arginine/liter of the TPN feeding formulation). The same amounts of the essential and non-essential amino acids (leucine, isoleucine, valine, phenylalanine, lysine, valine, isoleucine, threonine, tryptophan, histidine, lyrosine, alanine, glycine, proline and serine) as defined in Example 3 will be present; and the solution prepared in the same manner.

### Example 5

### Anti-Hypotensive Arginine-Free TPN Formulation

If a formulation of amino acids for patients at risk of hypotension or septic shock, or receiving pharmacological agents which may cause such a condition, such as TNF (tumor necrosis factor), etc., is desired which is essentially arginine-free and contains ornithine and citrulline, the formula as outlined in Example 3 supplemented with ornithine and citrulline can be utilized.

Ornithine will then be added to constitute 1-2 grams ornithine per liter of the TPN feeding solution (0.1% to 0.2% by weight ornithine). Citrulline will be added to constitute about 1 gram citrulline per liter of the TPN feeding solution (about 0.1% by weight citrulline). The same amounts of the amino acids of Example 3 will be present, and the solution prepared in the same manner. The anti-hypotensive formulation is arginine-free.

### PROPHETIC EXAMPLE 6

### Formulations and Methods for Inhibiting Hypotension and Septic Shock

The present prophetic example provides methods whereby the particularly defined arginine-free and low-arginine formulations may be used in the treatment of patients at risk of developing hypotension and septic shock, or whom may require parenteral nutritional support and have already developed hypotension or septic shock.

The proposed formulations and proposed methods may be used most particularly in the clinical management of patients requiring total parenteral nutritional support and receiving immunomodulators. By way of example, the term "immunomodulator" refers to such agents as interferon, interleukin-2, and tumor necrosis factor.

Many of the class of substances recognized as immunomodulators are used as anti-cancer chemotherapeutic agents. Thus, it is envisioned that the presently described methods would be effective for the clinical management of patients being maintained on parenteral nutritional support and receiving chemotherapeutic agents with immunomodulatory action.

According to the present invention, the use of a non-hypotensive formulation comprising a mixture of amino acids in a pharmaceutically acceptable diluent for the manufacture of a medicament prophylaxis or treatment of systemic hypotension related to the elevated production of nitric oxide in an animal is provided. The formulation may be essentially arginine-free, in a most preferred embodiment. A non-hypotensive formulation which includes a low arginine-concentration may also be employed according to another embodiment.

The formulation is to be administered to the patient until a physiologically acceptable blood pressure in the animal is reached and maintained. For a human, a physiologically acceptable systolic blood pressure level is about 100 mm Hg.

More particularly, the present invention relates to an essentially arginine-free or low-arginine (not more than 0.1%) formulation comprising a mixture of amino acids. The formulation should be prepared so as to be physiologically suitable as an intravenous hyperalimentation (total parenteral nutrition) solution for patients requiring such solutions.

Stated as a range of concentrations for the most preferred mixture of amino acids, the composition of the preferred arginine-free formulations is defined in Table 4. Most preferably, the proposed concentrations to be included in such a formulation appear in Table 4.

**TABLE 4**

| **PREFERRED RANGES OF AMINO ACIDS IN NON-HYPOTENSIVE FORMULATIONS** |
|---|
| 3-4 g/l isoleucine (0.3-0.4%); |
| 4-6 g/l leucine(0.4-0.6%); |
| 3-4 g/l lysine (0.3-0.4%); |
| 1-2 g/l methionine (0.1-0.2%); |
| 1-2 g/l phenylalanine (0.2-0.2%); |
| 2-3 g/l threonine (0.2-0.3%); |
| 0.5-1.5 g/l tryptophan (0.05-0.15%); |
| 3-4 g/l valine (0.3-0.4%); |
| 4-5 g/l alanine (0.4-0.5%); |
| 1-2 g/l histidine (0.1-0.2%); |
| 3-4 g/l proline (0.3-0.4%); |
| 1-2 g/l serine (0.1-0.2%); |
| 0.25-0.75 g/l tyrosine (0.025-0.075%); |
| 4-5 g/l glycine (0.4-0.5%); and |
| 2-3 g/l aspartic acid (0.2-0.3%). |

The formulation may also include ornithine. Where ornithine is part of the particular formulation, it is to be included at a concentration of 1-2 grams/l of the TPN feeding formulation (0.1-0.2% ornithine).

Where the formulation is a parenteral formulation, the mixture should be adjusted so as to be physiologically compatible for parenteral administration.

The described non-hypotensive parenteral nutritional formulations may alternatively include low concentrations of arginine found not to provide sufficient substrate for nitric oxide production in hypotensive animals. A low concentration of arginine for purposes of the present invention is defined as less than or equal to 0.1% arginine in the feeding formulation ready to be administered to the patient. Most preferably, the formulation may include between 0.01% to 0.1% arginine.

An additional most preferred embodiment of the claimed invention is essentially arginine-free. In one particularly defined embodiment of the essentially arginine-free formulation, the amino acids ornithine and citrulline are included. Ornithine and citrulline contribute to the urea cycle substrate requirements of the animal. Where ornithine and citrulline are included in the formulation, the concentration of these ingredients most preferred comprise 0.1-0.2% (or 1-2 g/l) ornithine and about 0.1% (or 1 g/l) citrulline.

**Table 5**

| **Arginine-Free Formulation Mixture of Amino Acids** | | |
|---|---|---|
| Amino Acid | 2X Concentrate (mg/100 ml) | Final Feeding Concentration (g/l) |
| Isoleucine | 600 | 3 |
| Leucine | 1,000 | 5 |
| Lysine | 1,000 | 5 |
| Methionine | 200 | 1 |
| Phenylalanine | 300 | 1.5 |
| Threonine | 400 | 2 |
| Tryptophan | 200 | 1 |
| Valine | 500 | 2.5 |
| Alanine | 900 | 4.5 |
| Histidine | 300 | 1.5 |
| Proline | 700 | 3.5 |
| Serine | 400 | 2.0 |
| Tyrosine | 450 | 2.0 |
| Glycine | 800 | 4.0 |
| Aspartic acid | 600 | 3 |
| Ornithine | 400 | 2 |

The ornithine content described for the formulation above may be omitted and replaced with citrulline at a concentration of about 2 g/l. The amino acids concentrate (2X) is mixed with a pharmaceutically acceptable diluent, such as for example, a glucose solution in a proportion of 1 to 1 (1 part amino acid solution to 1 part of a dextrose solution). In addition, trace elements, vitamin supplements and essential salts (Na⁺, K⁺, PO₄⁻, Ca⁺⁺, Mg⁺⁺) may be added.

The anti-hypotensive formulations as part of a method for treating or preventing hypotension may be administered as a parenteral nutritional formulation according to parenteral feeding methods well known to those of skill in the medical arts.

In practicing these methods, a physiological benchmark will be referred to in reference to determining at what point the administration of the arginine-free formulation should be terminated. For example, the patient's systolic blood pressure level may be monitored so as to determine when the patient has reached a physiologically acceptable level. A return to normal systolic pressure may then be used to indicate the point at which nitric oxide-production was being reduced, and had escaped risk of a greater reduction in peripheral vascular resistance or arterial blood pressure.

As generally defined according to the claimed method, hypotension (low blood pressure) is defined as an adult human systolic blood pressure level of less than about 100 mm Hg. A physiologically acceptable systolic blood pressure in an adult human is at least about 100 mm Hg systolic blood pressure.⁴⁰

It has been observed that serum arginine levels increase upon the administration of a standard TPN formulation.⁴¹ Therefore, by eliminating arginine as an ingredient in a TPN formulation, the inventors propose that serum arginine-levels will be significantly reduced in patients receiving such an arginine-free TPN formulation as compared to similarly situated patients whom had instead been receiving a standard TPN solution. A standard TPN solution which includes greater than 0.1% arginine would not be expected to constitute a hypotensive formulation.

### EXAMPLE 7

### IN VIVO EFFECTS OF NITRIC OXIDE INHIBITOR NAA ON IL-1 INDUCED HYPOTENSION

The present example is provided to demonstrate the utility of NAA (N^{ω}-amino-L-arginine) and other nitrogen analogs for increasing blood pressure, most particularly in a cytokine-induced hypotensive animal.

### Effect of NAA on IL-1-Induced Hypotension

As summarized in Table 6 and illustrated in Fig. 1, anesthetized dogs (n = 4) given IL-1 (50 µg/kg) developed a progressive, moderately severe hypotension, with MAP decreasing from a baseline of 111 ± 7 mm Hg to 80 ± 2 mm Hg within about 3 hours. The IL-1-induced hypotension was rapidly reversed by the intravenous administration of a single bolus dose of NAA (20 mg/kg, 88 µmol/kg); MAP increased by 46 ± 9 mm Hg (58%) to 125 ± 8 mm Hg. In all cases, MAP following NAA administration was significantly higher than the pre-IL-1 baseline MAP (average increase = 14.3 ± 7.7 mm. In control dogs not given IL-1, administration of NAA also increased MAP above baseline; the average increase in MAP was 20.1 ± 3.8 mm Hg, a value not significantly different from the increase above baseline observed in the dogs given IL-1 (*p* = 0.254). Thus, whereas NAA had a much greater pressor effect in dogs previously made hypotensive by administration of IL-1, the final MAP achieved after NAA administration was similar in control and IL-1-treated dogs.

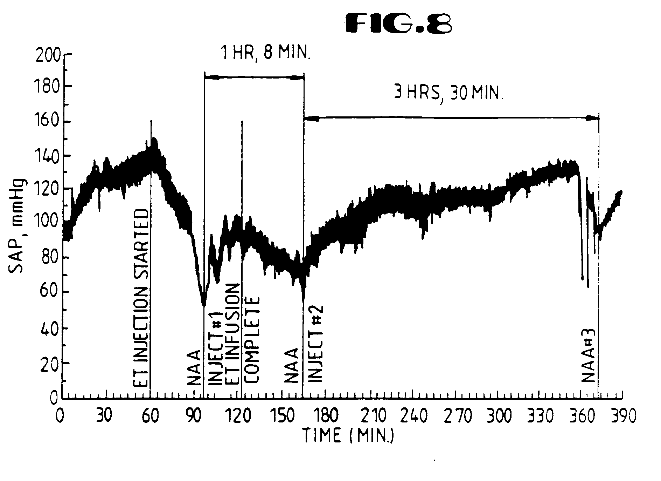

Specificity of the NAA pressor effect in both IL-1-treated and control dogs was demonstrated by its rapid and complete reversal by L-arginine (400 mg/kg, 2.3 mmol/kg); after arginine administration, MAP averaged 66 ± 27 mm Hg in IL-1-treated dogs, but it was essentially at baseline levels in control dogs. In other studies with IL-1 and other cytokines (e.g., TNF and IL-2), the present inventors determined the duration of the NAA pressor effect in the absence of L-arginine. Single injections of NAA (20 mg/kg) in cytokine-treated dogs were found to maintain MAP at baseline or higher levels for up to about 60 minutes; second and third injections of NAA (20 mg/kg) had longer effects and maintained physiologically acceptable MAP readings for up to 7 hours (Figure 8).

The central role of vasodilation in IL-1-mediated hypotension is exemplified by the observation that the decrease in MAP was associated with a 33.5% decrease in systemic vascular resistance (Table 6). Following administration of NAA, systemic vascular resistance increased in parallel with the restoration of MAP; systemic vascular resistance values after injection of NAA averaged 43% higher than baseline systemic vascular resistance measurements, although there was significant animal-to-animal variation. Following administration of arginine, systemic vascular resistance decreased substantially in all IL-1-treated animals (average decrease = 61%). In control dogs, the NAA-mediated pressor effect was also associated with an increase in systemic vascular resistance.

Cardiac output was noted to decrease by 29.2% (*P* = .046) in dogs treated with NAA alone. This decrease was completely reversed by the subsequent administration of L-arginine (Table 6). In dogs treated with IL-1, the cardiac output increased slightly, but not significantly (*P* = .371). Administration of NAA caused a 25.2% reduction in cardiac output compared with that obtained after the administration of IL-1 (*P* = .037); however, this value was not statistically different from the baseline cardiac output (1.93 versus 2.25 L/minute; *P* = .204). These changes were reversed by administration of L-arginine (Table 6). None of the compounds administered caused a significant change in heart rate.

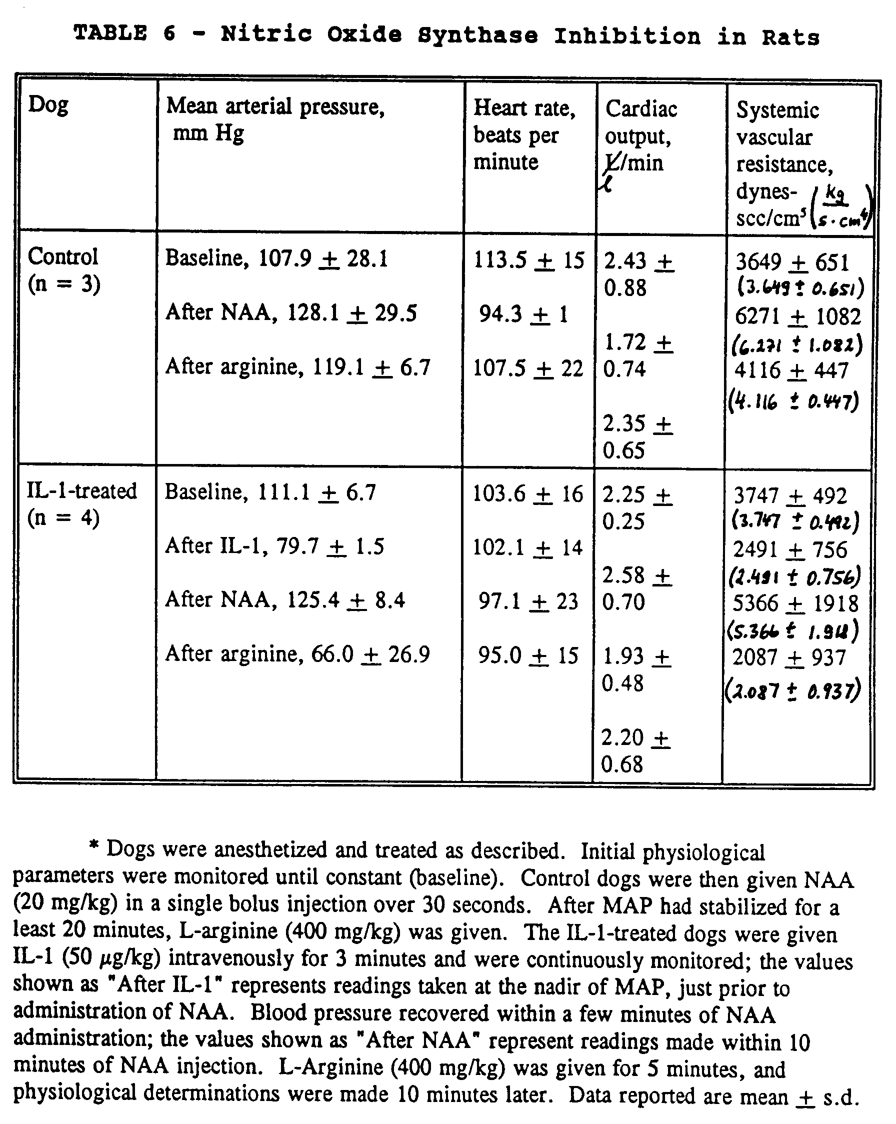

### EXAMPLE 8

### NMMA IN VIVO AND BLOCKAGE OF TNF-INDUCED HYPOTENSION

The present example is provided to demonstrate the *in vivo* utility of nitric oxide inhibitors, such as NMMA, for inhibiting hypotension. More specifically, the present example demonstrates that hypotension associated with the administration of TNF in the dog can be blocked by subsequent administration of NMMA. Furthermore, this increase in blood pressure in hypotensive animals is demonstrated to be reversible upon the administration of arginine. NMMA in its free base form has the structural formula: 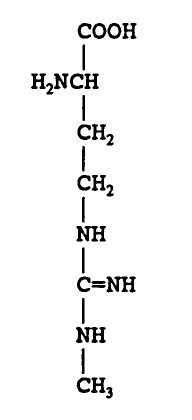 Furthermore, this inhibition of hypotension can be reversed by administration of an excess of arginine. These results show that NO· is the mediator of hypotension induced by TNF. Furthermore, activation of NO· synthesis may be involved in the pathogenesis of septic shock. This study also demonstrates the hypertensive action of arginine in a TNF-treated animal previously made normotensive by administration of NMMA.

### Reagents

Recombinant human TNF, specific activity 2 x 10⁷ units/mg, was from the Dainippon Chemical Corporation, Tokyo, Japan. TNF was administered at a dose of 10 µg//kg in a volume of 10 ml of phosphate buffered saline containing 17 mg/ml of dog albumin. NMMA was synthesized by adaptation of the method of Corbin and Reporter (Anal. Biochem. 57: 310-312, 1974). The NMMA was dissolved in 5 ml of phosphate-buffered saline for administration at a dose of 20 mg/kg. Arginine was obtained from Sigma Chemical Company, St. Louis, Mo.

### Animals

Four conditioned mongrel dogs, 2 males and 2 females, weighing 28 to 30 kg, were studied. Care of the animals were in accordance with the recommendations of the American Association for Accreditation of Laboratory Animals [DHEW(DHHS) publication no. (NIH) 78-23, revised, 1978]. On the day of the experiment, the dogs were fasted overnight. They were anesthetized with phenobarbital (25 mg/kg). The animals were then intubated orally with a #10 fr. endotracheal tube and ventilated with a Harvard pump ventilator at a rate of 14 breaths per minute and a tidal volume of 15 ml/kg. An arterial line was percutaneously placed in the femoral artery on the day of the experiment.

### Physiological measurements

Mean (electronic) and phasic systemic arterial pressures (SAP) were continuously recorded on a Hewlett-Packard recording system (model 7758B) using strain gauge manometers (Hewlett-Packard model 1290A) which were connected to the arterial line. Heart rate (HR) was determined from an EKG tracing and continuously recorded on the Hewlett-Packard recording system. Oxyhemoglobin saturation (SaO₂) was obtained using a pulse oximeter (BIOX 111, Boulder, CO). Continuous time-series records of SAP, HR, and SaO₂ were obtained using a Lab Master analog-to-digital convertor (16 channel, 12 bit, 30 kHz; Scientific Solutions, Inc.) sampling at 55 Hz and storing the 6 sec averages on a magnetic disk.

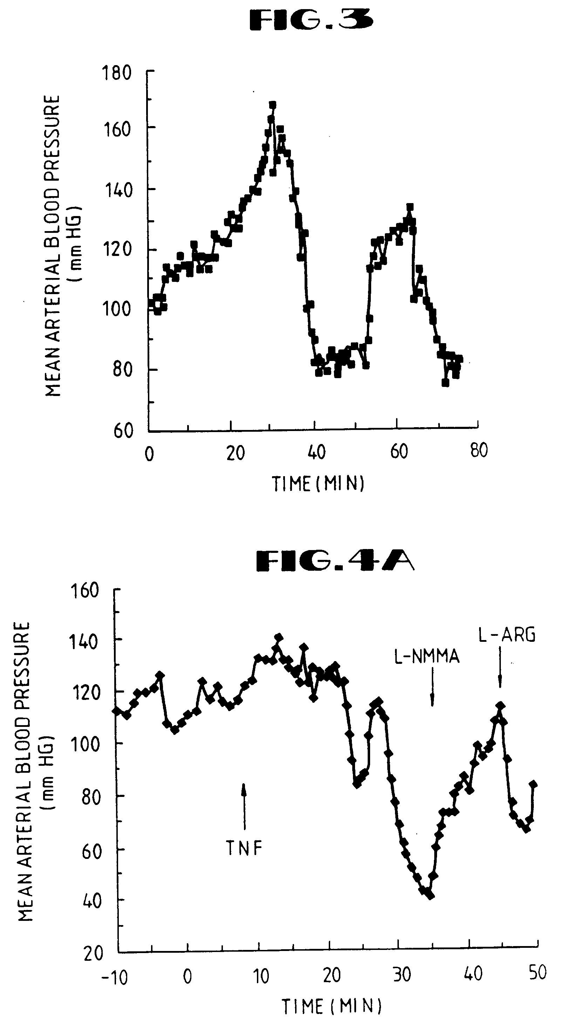

NMMA was found to reverse the hypotension associated with the administration of TNF (Figure 3). The pressor effect of NMMA occurred rapidly (within 2 minutes) and could be antagonized by administration of an excess of L-arginine. The antagonism of the NMMA pressor effect was stereospecifis for the L-form of arginine.

The data shown in Figure 3 is representative of several animal experiments. There were some variations noted in the degree of hypotension as well as the time of onset of hypotension after TNF administration. An example of severe hypotension resulting from administration of TNF is shown in Figure 4a. Ten µg TNF/kg body weight was intravenously administered at the ten minute time point; 4.4 mg NMMA/kg at about 52 minutes; and 3 g L-arginine at about 63 minutes. The onset of hypotension was found to occur between 30 to 60 minutes after TNF. The SAP dropped rapidly from 106 to 36. The administration of NMMA resulted in the rapid increase in blood pressure to an SAP of 116. This represents an 80 mm Hg increase in blood pressure after administration of NMMA.

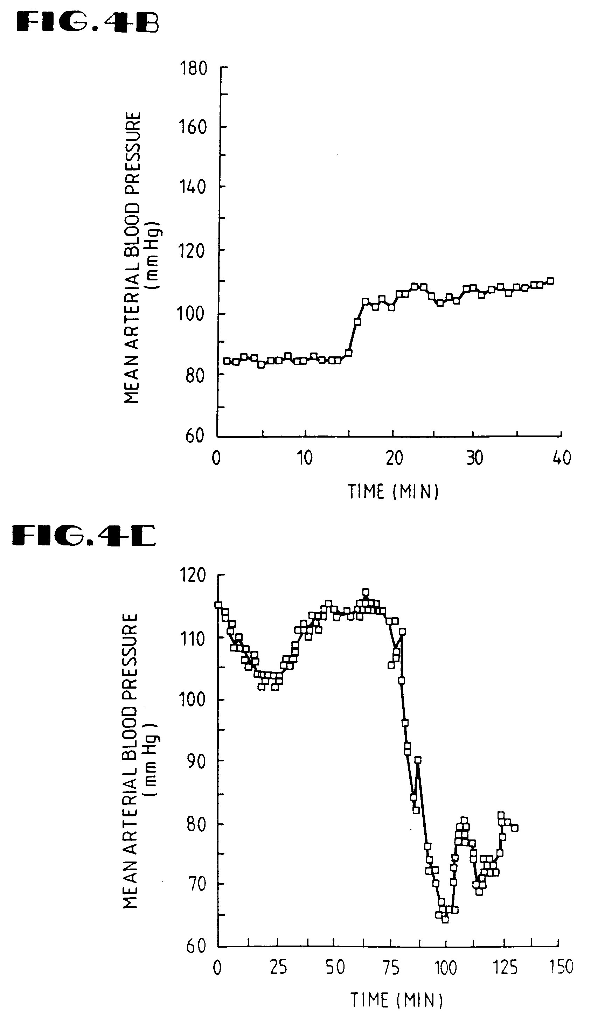

The administration of NMMA alone to untreated dogs (n=3) was also tested. Within 2 minutes after NMMA infusion, the blood pressure increased by 12 mm Hg. This was followed by a compensatory decrease in the HR with a return of the BP to baseline. The NMMA-induced bradycardia lasted 31 minutes. This response was not observed in animals which had been previously treated with TNF (Figure 4b). Subsequent administration of L-arginine reversed these small changes observed in systemic arterial pressure. In a second control study nitroglycerin was infused at a rate of 28 µg/kg/minute, IV, to lower the blood pressure to the same level as that observed with tumor necrosis factor (Figure 4c). After administration of NMMA in nitroglycerin infused dogs, the blood pressure increased only 14 mm. Subsequent administration of L-arginine reversed this modest effect.

The administration of L-arginine to cytokine treated dogs reversed the antihypotensive effects of NMMA. Blood pressure was not affected by the administration of L-arginine to previously untreated normotensive dogs.

The dose-limiting toxicity of TNF administered to patients is hypotension. These experiments support that proposition that NO·, also known as EDRF, is the mediator of the hypotension. Furthermore, these hemodynamic changes can be antagonized by an N^{ω}-substituted arginine derivative and subsequently restored by the addition of excess arginine, supporting a role for arginine as the substrate for NO· synthesis. The present inventors have shown that NMMA can increase the resting blood pressure in the guinea pig. Therefore, NO· may play a role in normal arterial pressure homeostasis. This also appears to be true in the dog.

The pressor response to NMMA is much more dramatic in dogs with TNF-induced hypotension than in normotensive dogs. This suggests that TNF induced hypotension is due to an excess production of a vasoactive factor (i.e., NO·) which acts to regulate normal resting blood pressure.

TNF is also involved in the development of the toxicity observed in septic shock. Septic shock is most commonly caused by endotoxin, a component of the cell wall of Gram negative organisms. The administration of anti-TNF antibodies after TNF exposure does not protect against hypotension. However, administration of anti-TNF antibodies may protect against hypotension where administered before TNF exposure. This implies that endotoxin may induce other mediators of hypotension. The results presented herein indicate that NO· is the true mediator of that response.

### EXAMPLE 9

### L-NMA IN VIVO AND ENDOTOXIN-INDUCED HYPOTENSION

This example is provided to demonstrate the utility of nitric oxide synthase inhibitors, such as the arginine analog L-NMA, in treating hypotension in an animal.

In the present study, the effect of L-NMA on endotoxin-induced shock in dogs was examined. The present findings indicate that NO· is an important mediator of endotoxin-induced hypotension and that inhibitors of NO· synthesis should be of value in the treatment of septic shock.

**Reagents:** Nω-Methyl-L-arginine was synthesized as previously described by Corbin, *et al*. (1974) (Nω-Methylated Arginines: convenient Preparation of Nω-Methylarginines, *Anal. Biochem*., 57, 310-312), and purified by crystallization as the monoflavianate salt. A solution of the free amino acid was obtained by stirring a suspension of the salt with Dowex-1 (OH); after neutralization with HCl, the concentration of L-NMA was determined by amino acid analysis using the crystalline monoflavianate salt as standard. Endotoxin (Escherichia coli; B0128:B12) and all other reagents were purchased from Sigma chemical Company, St. Louis, Missouri. Nitroglycerin was purchased from DuPont Pharmaceuticals, Wilmington, D.E.

**Animals:** Studies were carried out on 12 conditioned mongrel dogs (9 males and 3 females) weighing 22-32 kg (avg=25.3 kg). Animal care was in accordance with the recommendations of the American Association for Accreditation of Laboratory Animal Care, and met all standards prescribed by the Guide for the Care and Use of Laboratory Animals (Guide for the Care and Use of Laboratory Animals (1978) Dept. of Health, Education and Welfare, Washington, D.C. (Publ. No. 78-23). Animal protocols were approved by The University of Texas Animal Welfare committee. The dogs were fasted overnight prior to the day of experimentation. They were anesthetized with sodium pentobarbital (25 mg/kg i.v.). Dogs were then endotracheally intubated and ventilated with a piston-driven respirator (Harvard instruments) using room air at a tidal volume of 15 ml/kg and at a rate of 12 to 14 breaths per minute, adjusted to achieve a normal arterial pH and pCO₂ (Instrumentation Laboratories lL1302 pH/Blood Gas Analyzer). Catheters were placed percutaneously into the femoral and pulmonary arteries; In the latter, a flow-directed thermal-dilation catheter was used (Abbott Critical Care Systems).

**Physiologic measurements:** Mean SAP and heart rate were continuously monitored (Parametron 7048 Monitoring System, Roche) and stored on a magnetic disk using an analog-to-digital converter (Scientific Solutions, Inc.). Cardiac output (CO) was determined as the mean of six measurements by thermal-dilution. Systemic vascular resistance was calculated as (SAP X80)/CO and expressed as dynes-sec/cm⁵ (kg/s·cm⁴).

**Protocol:** After the blood pressure and heart rate stabilized, endotoxin (40 µg/kg, in 10 ml of phosphate-buffered saline (PBS), pH 7.4) was infused i.v. over 2 minutes. This dose of endotoxin typically induces severe and often lethal cardiovascular collapse in the dog. Blood pressure was monitored, and when either SAP fell below 60 mm Hg or a stable nadir in systemic arterial pressure (SAP) was maintained for 10 minutes, L-NMA was administered (20 mg/kg in 5 ml of PBS i.v. over 1 min.). In most experiments, L-arginine (400 mg/kg in 20 ml PBS) was administered ten minutes later by i.v. infusion over 2 minutes. In control experiments, dogs without prior exposure to endotoxin received L-NMA alone. To simulate the hypotension observed in dogs receiving endotoxin, one group of dogs received a continuous i.v. infusion of nitroglycerin (2 mg/ml) at a rate adjusted to maintain the SAP at 60-70 mm Hg. Nitroglycerin-treated dogs then received L-NMA (20 mg/kg) and 20 minutes later L-arginine was administered (400 mg/ml).

**Statistics:** Statistical significance was evaluated using Student's test and either a one-tailed or two-tailed analysis as appropriate for comparisons.

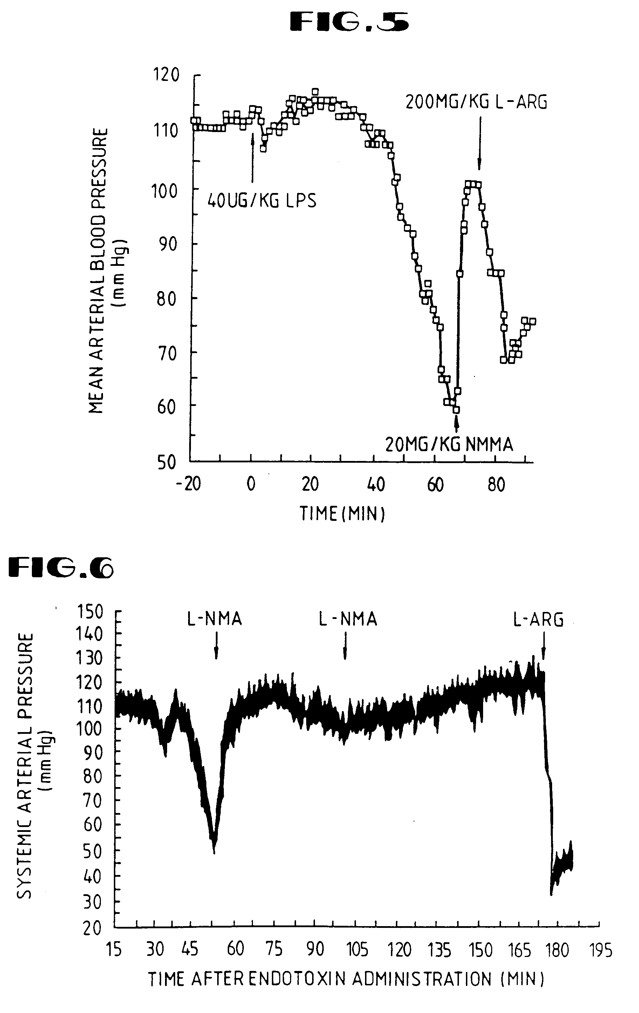

A representative blood pressure tracing which depicts the effect of endotoxin on systemic arterial pressure in the anesthetized dog is shown in Figure 5. Cardiovascular parameters for this and 3 additional dogs are summarized in Table 7.

**Table 7**

| Hemodynamic Effects of L-NMA during Hypotension | | | | |
|---|---|---|---|---|
| Type of Evaluation | Systemic Arterial Pressure (mmHg) | Heart Rate (beats/min) | Cardiac Output (l/min) | Systemic Vascular Resistance (dynes-sec/cm⁵) [Kg/s·cm⁴] |
| | | | | |

| Study 1: Endotoxin-treated (n-4) | | | | |
|---|---|---|---|---|
| Baseline | 128.3 ± 9.4 | 119.5 ± 6.0 | 2.99 ± 0.32 | 3565 ± 454 [3.565 ± 0.454] |
| After Endotoxin | 59.5 ± 3.1** | 124.0 ± 7.6 | 2.17 ± 0.44 | 2403 ± 352 [2.403 ± 0.352] |
| After L-NMA | 107.3 ± 9.6** | 123.3 ± 4.8 | 2.03 ± 0.32 | 4462 ± 552** [4.462 ± 0.552] |
| After L-Arginine | 128.3 ± 9.4 | 119.5 ± 6.0 | 2.99 ± 0.32 | 3565 ± 454 [3.565 ± 0.454] |

| Study 2: Nitroglycerin-treated (n=3) | | | | |
|---|---|---|---|---|
| Baseline | 129.3 ± 10.2 | 143.7 ± 12.1 | 3.14 ± 0.21 | 3294 ± 74 [3.294 ± 0.074] |
| During Nitroglycerin | 64.7 ± 2.7** | 137.3 ± 5.0 | 2.72 ± 0.27 | 1924 ± 132** [1.924 ± 0.132] |
| After L-NMA | 81.8 ± 3.5* | 191.7 ± 35.0 | 3.85 ± 0.80 | 1858 ± 399 [1.858 ± 0.399] |
| After L-Arginine | 57.0 ± 13.0 | 148.7 ± 19.9 | 5.15 ± 1.08 | 1088 ± 491 [1.088 ± 0.491] |
| For study 1, dogs were anesthetized, instrumented, and baseline cardiovascular measurements were recorded (Pretreatment). Endotoxin (40 µg/kg) was then administered and cardiovascular parameters were monitored. When blood pressure either reached a stable nadir or declined below 60 mm Hg (After endotoxin), L-NMA (20 mg/kg) was administered, and cardiovascular parameters were again determined (After L-NMA). After an additional ten min, L-arginine (400 mg/kg) was administered and cardiovascular measurements were determined 2 min. later (After L-Arginine). Results are reported as means ± S.E., (n=4). Study 2 was carried out similarly, except that endotoxin was not administered. Instead, dogs received a continuous infusion of nitroglycerin (2 mg/ml) titrated to maintain SAP AT 65 mm Hg, (n=3). Asterisks indicate statistically significant difference (*p<0.005, **p<0.001) from the immediately proceeding condition. | | | | |

ET (40 µg/kg) produced a marked decrease in blood pressure within 120 min. (ΔSAP=-69 ± 16 mm Hg, p<0.05). Untreated, this dose of endotoxin typically causes lethal cardiovascular collapse in the dog. L-NMA largely reversed the hypotension within 1.5 minutes, increasing SAP by 47.8 ± 6.8 mm Hg (p<0.01) and SVR by 2.06 ± 0.338 Kg/s·cm⁴ (2060 ± 338 dynes-sec/cm⁵)(p<0.01); HR and CO were unchanged (Table 7). L-arginine reversed the effect of L-NMA and restored the endotoxin-induced hypotension, decreasing both SAP (p<0.0.01) and SVR (p<0.01) to values similar to those observed before administration of L-NMA. As illustrated in Figure 5, after L-arginine, blood pressure decreased to similar levels compared to those observed prior to L-NMA administration. Figure 5 shows the time course of changes in mean systemic arterial pressure (SAP) in a pentobarbital-anesthetized dog following the i.v. administration of endotoxin (ET), Nω-methyl-L-arginine (L-NMA), and L-arginine (L-Arg). Data from this and additional experiments are summarized in Table 7.

In view of the potential clinical use of NO·-synthesis inhibitors in endotoxin- and cytokine-induced shock, it is important to establish that L-NMA can provide long-term reversal of hypotension. It was found that a single i.v. dose of L-NMA (20 mg/kg) restored normal blood pressure for 30-60 minutes. If an additional dose of L-NMA (20 mg/kg) was given when the blood pressure began to decrease again, normal blood pressure could be maintained for at least 2 hours in the endotoxin-treated dog. Results of a typical study are shown in Figure 6. The maintenance of normal blood pressure continued to be dependent on L-NMA even after 2 hours, since L-arginine could still restore endotoxic hypotension at this time (i.e., a decline in blood pressure to less than 45 mm Hg). Figure 6 shows the time course of changes in mean systemic arterial pressure (SAP) in a pentobarbital-anesthetized dog following the i.v. administration of endotoxin. After 53 min. blood pressure declined to 47 mm Hg (ΔSAP=-61 mm Hg). Administration of L-NMA (20 mg/kg) resulted in a rapid reversal of the severe hypotension (73 mm Hg increase in SAP within 10 min). Blood pressure was maintained for 48 min by the first dose of L-NMA then started to decline. A second dose of L-NMA restored the blood pressure to a level equivalent to the first dose and maintained the SAP greater than 100 mm Hg for 2 hrs. To demonstrate that the potential for hypotension was still retained, the effect of L-NMA was reversed with an excess of L-arginine (400 mg/ml). This resulted in a decline in blood pressure to 43 mm Hg (ΔSAP=-77mm Hg).

As shown in Table 8, L-NMA alone had a significant but modest hypertensive effect in control dogs not treated with endotoxin; L-NMA increased SAP by only 24.8 ± 2.7 mm Hg (p,0.01) with an associated increase in SVR (p=0.01), and decreases in heart rate (HR) and cardiac output (CO) that did not reach statistical significance. L-arginine (400 mg/kg) fully reversed the pressor effect of L-NMA.

**TABLE 8**

| HEMODYNAMIC EFFECTS OF L-NMA IN CONTROL DOGS | | | | |
|---|---|---|---|---|
| | Systemic Arterial Pressure (mm Hg) | Heart Rate (beats/min) | Cardiac Output (l/min) | Systemic Vascular Resistance (dynes-sec/cm⁵⁾ [Kg/cm⁴·s] |
| Baseline | 129.0±10.9 | 121±17.9 | 3.54±0.68 | 3115±347 [3.115±0.347] |
| After L-NMA | 153.8±11.4** | 82.5±6.1 | 2.12±0.26 | 5967±523*[5.967±0.523] |
| Experiments were as described in Figure 5, except that endotoxin was not administered. Results are reported as means±S.E., (n=4). Asterisks indicate significant differences from baseline (*p,0.005, **p,0.001). L-NMA = Nω-methyl-L-arginine. | | | | |

In an additional series of experiments, blood pressure was reduced to 65 mm Hg by continuous i.v. infusion of nitroglycerin, a hypotensive agent that forms NO· by an L-arginine and NO· synthase-independent mechanism. Administration of L-NMA (20mg/kg) to those dogs resulted in only a 17.1±5.0 mm Hg change without significant alteration in HR, CO, or SVR.

The pathogenesis of the cardiovascular collapse that occurs during septic shock is poorly understood. Current treatment includes i.v. fluid administration and use of pressor drugs to increase peripheral vascular resistance and cardiac output. Very recently, endotoxin-binding agents including polymyxin B (Hanasawa, et al., 1989, New Approach to Endotoxic and Septic Shock by Means of Polymyxin B Immobilized Fiber *Surg. Gynecol. Obstet.* 168:232.) and antibodies which neutralize TNF (Tracey, et al., 1987, Anti-cachectin/TNF monoclonal antibodies prevent septic shock during lethal bacteremia *Nature* 330:662-664.) have been used in an attempt to modify the sequelae of septic shock. Although the latter approaches may have prophylactic value, there is no evidence that septic shock can be easily or rapidly reversed by removal of endotoxin or TNF.

Therapy of patients already in septic shock requires intervention at secondary and tertiary steps in the cascade of events initiated by endotoxin. Because the development of hypotension and other changes associated with septic shock may depend on complex interactions between cytokines, eicosanoids, PAF, activated complement components, and other factors, it is not surprising that several interventions have been found to be at least partially effective in some animal models.

Inhibitors of prostaglandin synthesis and PAF receptor antagonists are two major classes of compounds that may have therapeutic potential (8-9). Although these agents appear to be effective, they have been tested primarily in animals administered very large doses of endotoxin (e.g., 1 to 40 mg/kg, or about 1000 times larger than the dose used here). The onset of hypotension occurs within a few minutes in such animals and may not accurately reflect the cytokine-mediated processes characteristic of clinical septic shock.

In the present study with endotoxin and in a previous study with TNF, microgram doses of ET or TNF were administered, and the hypotensive response occurred after a delay of 30 to 90 min.

The present inventors' demonstration that dogs given TNF exhibit a severe hypotension that can be substantially reversed by administration of L-NMA suggested that overproduction of NO· is a major factor in TNF-induced shock. The data in Table 8 show that L-NMA has a rapid and strong anti-hypotensive effect in the endotoxemic dog.

The effects of L-NMA on cardiac output and SVR in the four control dogs showed considerable variation. In two dogs, cardiac output decreased markedly (Δ≥1.5 l/min.) and calculated SVR increased dramatically, i.e., ΔSVR was ≽ 3.5 Kg/s·cm⁴ (Δ≥3500 dynes-sec./cm). In contrast, major changes in cardiac output after L-NMA administration were not seen in any of the ET-treated dogs or in the other two control dogs; in the latter, SVR increased by only about 1.4 Kg/s·cm⁴ (1400 dynes-sec./cm⁵). Although these results suggest the possibility that L-NMA may have a direct effect on cardiac output under control conditions, additional studies are required. It is likely that activation of the arterial baroreceptor reflex mechanism accounts for the L-NMA-induced decrease in HR and CO under control conditions. In support of this view, it was observed that control dogs given phenylephrine at a dose that elevated SAP to a level similar to that produced by L-NMA alone also showed similar decreases in HR and CO. The lack of effect of L-NMA on HR or CO in hypotensive dogs may be because the level of hypotension was below the range of baroreceptor reflex sensitivity.

In view of the multiple mediators reported to contribute to septic shock, it was the expectation that even complete inhibition of NO· formation could not fully reverse the hypotension of ET-induced shock. Indeed, that blood pressure was not *fully* restored to pretreatment values by 20 mg/kg L-NMA suggests that mediators other than NO· contribute modestly to hypotension in the endotoxemic dog. The possibility that NO· synthesis was not fully inhibited by the administered dose of L-NMA provides an alternative explanation for the failure to fully restore blood pressure to pretreatment levels. Although direct determination of the extent of NO· synthesis inhibition is not possible in vivo, limited dose response studies indicate that L-NMA doses greater than 20 mg/kg do not have a significantly greater pressor effect. The ET-induced hypotension escaping blockade by 20 mg/kg L-NMA may be due to mediators other than NO·. While it may be that long-term inhibition by L-NMA may be self-limited by conversion to L-Arginine (Salvemini, et al., 1990, Immediate Release of a Nitric Oxide-Like Factor from Bovine Aortic Endothelial Cells by Escherichia coli Lipopolysaccharide. *Proc. Natl. Acad. Sci*. 87:2593.), such metabolism would not be expected to diminish the short-term pressor effect of L-NMA which is shown in Figure 5. Nevertheless, the finding that L-NMA restores blood pressure to normal or near normal values indicates that overproduction of NO· is a major, and perhaps *the* major, cause of hypotension in endotoxic shock.

In one study, a single injection of L-NMA (20 mg/kg) was able to reverse endotoxin-elicited hypotension for 30 to 60 min. As shown in Figure 6, normotension could be maintained for at least 2 hours by a subsequent dose of L-NMA. The long-term reversal of endotoxin-induced hypotension with L-NMA demonstrates the potential clinical utility of this agent. In conclusion, these results suggest that NO· synthesis inhibitors should be of considerable value in the treatment of septic shock. Since administration of arginine reverses the pressor effect of L-NMA (Figure 5, Figure 6), it is clear that high concentrations of plasma arginine antagonise and may completely abrogate the beneficial effects of L-NMA. L-NMA and related nitric oxide synthase inhibitors should therefore be particularly useful in hypotensive animals maintained at low plasma arginine levels such as with a low arginine dietary source (an arginine-free TPN).

### EXAMPLE 10

### NMMA AND NAA IN VIVO AND TNF CYTOTOXICITY

NMMA does not inhibit the anti-tumor activity of TNF and IL-2, *in vitro*. TNF bioactivity was measured by the cytotoxicity towards murine L929 cells, *in vitro*. Addition of NMMA or Nωaminoarginine did not alter the cytolytic effect of TNF towards tumor cells *in vitro* (Table 10).

**TABLE 10**

| **Effects of NMMA on the Cytolytic Activity of rh-TNF Against Actinomycin D-Treated L929 Cells** | |
|---|---|
| [NMMA] (mM) | TNF Activity (Units/ml) |
| 0 | 594.5 |
| 0.125 | 536.9 |
| 0.250 | 538.2 |
| 0.500 | 562.4 |
| 0.750 | 404.7 |
| 1.0 | 415.7 |

Similarly, NMMA did not alter either the proliferation phase or the lytic phase of human LAK cells exposed to IL-2, *in vitro* (Table 10).

**TABLE 11**

| **Effects of NMMA on IL-2 Mediated Lymphokine Activated Killer Cell Activity** ***in vitro*** | |
|---|---|
| [NMMA] (mM) | % Target Cell Lysis* |
| 0 | 66.1 ± 9.5 |
| 0.25 | 63.3 ± 11.8 |
| 0.5 | 67.7 ± 10.8 |
| 1.0 | 59.3 ± 7.5 |
| 2.0 | 75.1 ± 4.1 |

| | |
|---|---|
| * % Lysis calculated from the % of release of radioactivity from ⁵¹Cr-labeled Raji Target cells minus spontaneous release. Effector cells were human blood lymphocytes cultured for 4 days in the presence of 40 U/ml of IL-2 (E:T=80:1). | |

Aminoarginine is the most potent inhibitor of nitric oxide production reported thus far. Since NMMA is metabolized to citrulline which can subsequently serve as a precursor for arginine biosynthesis, other arginine analogs were tested for their ability to inhibit nitric oxide production (Table 12).

**TABLE 12**

| **Comparison of the ED**_{**50%**}***** **values of Nω-Substituted Arginine Analogs** | |
|---|---|
| Analog | ED_{50%} |
| NMMA | 336.7 |
| Aminoarginine | 109.5 |
| Nitro-L-Arginine | 2115 |
| Nitro-D-Arginine | >4500 |
| Nitro-L-Arginine benzyl ester | >1200 |
| Nitro-L-Arginine methyl ester | 1826 |
| Nitro-D-Arginine methyl ester | >4500 |

| | |
|---|---|
| *ED_{50%} + The effective dose of drug that inhibited 50% of the nitrite production by murine endothelial cells exposed to Gamma-Interferon (100U/ml) and TNF (500 U/ml) *in vitro*. | |

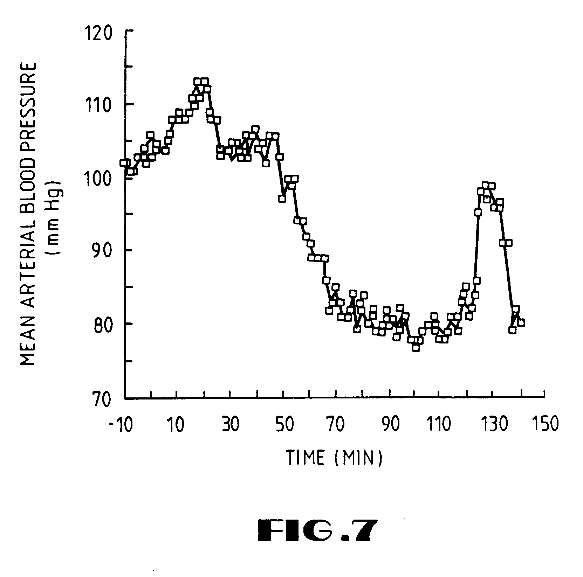

The most potent derivative tested was N^{ω}-aminoarginine. Subsequent testing *in vivo*, showed that aminoarginine was as effective as NMMA in reversing the hypotension associated with TNF administration in the dog (Figure 7).

The reversal of ET shock (lethal dose) by Nω-aminoarginine (NAA) for 4 hrs. 38 min. was demonstrated using multiple doses of aminoarginine (NAA). Figure 8 depicts systemic arterial pressure (SAP) versus time (min). ET (2 mg/kg), a lethal dose, was infused over 60 min. and NAA administered at 97, 165, and 374 min. to maintain blood pressure. The animal was survived for 24 hours and then autopsied. No pathological changes were observed in liver, lungs, heart, brain, bowel or kidney.

### EXAMPLE 11

### ANTI-HYPOTENSIVE ARGININE-FREE TPN FORMULATION

The present example defines an anti-hypotensive TPN formulation of the present invention. This arginine-free formulation is intended to be used to reduce plasma arginine levels in an animal. The reduced arginine levels in the animal will then augment the anti-hypotensive effect of the nitric oxide synthase inhibitors described herein. This combination therapy can thus be used in the treatment of conditions where hypotension is the sole or an attendant symptom. For example, this regimen may be used in the treatment of an animal in septic shock, an animal treated with chemotherapeutic agents which may reduce blood pressure, or an animal which is generally experiencing hypotension due to trauma.

A sterile, non-pyrogenic, stable solution for parenteral administration to a patient having hypotension or at risk of hypotension or systemic shock, particularly those receiving immunomodulatory agents, is prepared from pure crystalline amino acids, which are dissolved in a glucose solution (5% to 20%) in the following concentrations to provide a 2X concentrate TPN or a ready-to-feed TPN formulation, as indicated:

**TABLE 13**

| Amino Acids | 2x concentrate mg/100 ml formulation | Final Concentration (Feeding Formulation) g/l |
|---|---|---|
| isoleucine | 600-800 | 3-4 |
| leucine | 800-1200 | 4-6 |
| valine | 600-800 | 3-4 |
| phenylalanine | 200-400 | 1-2 |
| methionine | 200-400 | 1-2 |
| lysine | 600-800 | 3-4 |
| histidine | 200-400 | 1-2 |
| threonine | 400-600 | 2-3 |
| tryptophan | 100-300 | 0.5-1.5 |
| tyrosine | 50-150 | 0.25-0.75 |
| alanine | 800-1000 | 4-5 |
| aspartic acid | 400-600 | 2-3 |
| glycine | 800-1000 | 4-5 |
| proline | 600-800 | 3-4 |
| serine | 200-400 | 1-2 |

To obtain the preferred TPN formulation concentration suitable as a feeding formulation, a volume of 500 ml of the 2X concentrate (defined in Table 13) is mixed with 500 ml of a 50% dextrose solution, for the production of 1 liter of the feeding formulation (i.e., 500 ml of a 2X concentrate of AA and 500 ml of dextrose solution). Most preferably, the dextrose solution is supplemented with a physiologically acceptable concentration of vitamins and minerals.

The TPN of the present invention may also include glutamic acid (400-600 mg/100 ml of a 2x conc., or 2-3 g/l in a final concentration) and/or taurine (50-100 mg/100 ml. of a 2-fold concentrate; 0.25-0.5 g/l final concentration).

The solution is then filter-sterilized into appropriate containers for intravenous fluids. To prepare for administration, the volume is then brought to the desired feeding solution concentration with an equal volume of sterile glucose solution. The TPN as a ready to feed formulation is then to be kept cool. The solution may then be administered to a patient intravenously (IV). The pH of the TPN solution must also be adjusted to a physiologically acceptable pH, between 7.0 and 7.4. The formulation is arginine-free.

### PROPHETIC EXAMPLE 12

### FORMULATIONS AND METHODS FOR INHIBITING HYPOTENSION AND SEPTIC SHOCK

The present prophetic example provides methods whereby the particularly defined arginine-free formulations described herein may be used in the treatment of patients at risk of developing hypotension or septic shock, or whom may require parenteral nutritional support and are at risk of developing or who have already developed hypotension or septic shock.

The proposed formulations are most particularly useful in the clinical management of patients requiring total parenteral nutritional support and receiving immunomodulators. The term "immunomodulator" refers to such agents as interferon, interleukin-2, interleukin-1 and tumor necrosis factor as used in the description of the present invention.

Many of the class of substances recognized as immunomodulators are used as anti-cancer chemotherapeutic agents or immunorestorative agents in cancer patients receiving standard cytotixic drugs. Thus, it is envisioned that the presently described methods would be effective for the clinical management of patients being maintained on parenteral nutritional support and receiving chemotherapeutic agents with immunomodulatory action or immunorestorative action (eg., interleukin-1).

Thus, a method for prophylaxis or treatment of systemic hypotension related to the elevated production of nitric oxide in an animal is described comprising administering to the animal a non-hypotensive formulation which is arginine-free comprising a mixture of amino acids in a pharmaceutically acceptable diluent. The formulation is again to be essentially arginine-free.

The formulation is to be administered to the patient until a physiologically acceptable blood pressure in the animal is reached and maintained. For a human, a physiologically acceptable systolic blood pressure level is about 100 mm Hg.

More particularly, the present invention relates to an essentially arginine-free formulation comprising a mixture of amino acids. The formulation should be prepared so as to be physiologically suitable as an intravenous hyperalimentation (total parenteral nutrition) solution for patients requiring such solutions.

Stated as a range of concentrations for the most preferred mixture of amino acids, the composition of the specially tailored arginine-free formulations is defined in Table 14. Most preferably, the proposed concentrations to be included in such a formulation appear in Table 14.

**TABLE 14**

| **PREFERRED RANGES OF AMINO ACIDS IN NON-HYPOTENSIVE FORMULATIONS** |
|---|
| 3-4 g/l isoleucine (0.3-0.4%); |
| 4-6 g/l leucine(0.4-0.6%); |
| 3-4 g/l lysine (0.3-0.4%); |
| 1-2 g/l methionine (0.1-0.2%); |
| 1-2 g/l phenylalanine (0.2-0.2%); |
| 2-3 g/l threonine (0.2-0.3%); |
| 0.5-1.5 g/l tryptophan (0.05-0.15%); |
| 3-4 g/l valine (0.3-0.4%); |
| 4-5 g/l alanine (0.4-0.5%); |
| 1-2 g/l histidine (0.1-0.2%); |
| 3-4 g/l proline (0.3-0.4%); |
| 1-2 g/l serine (0.1-0.2%); |
| 0.25-0.75 g/l tyrosine (0.025-0.075%); |
| 4-5 g/l glycine (0.4-0.5%); and |
| 2-3 g/l aspartic acid (0.2-0.3%). |

The formulation may also include ornithine. Where ornithine is part of the particular formulation, it is to be included at a concentration of 1-2 grams/l of the TPN feeding formulation (0.1-0.2% ornithine).

Where the formulation is a parenteral formulation, the mixture should be adjusted so as to be physiologically compatible for parenteral administration.

The described non-hypotensive parenteral nutritional formulations may alternatively include low concentrations of arginine found not to provide sufficient substrate for nitric oxide production in hypotensive animals. A low concentration of arginine for purposes of the present invention is defined as less than or equal to 0.1% arginine in the feeding formulation ready to be administered to the patient. Most preferably, the formulation may include between 0.01% and 0.1% arginine.

An additional preferred embodiment of the claimed invention is an essentially arginine-free formulation. In a particularly preferred embodiment of the essentially arginine-free formulation, the amino acids ornithine and citrulline are included. Ornithine and citrulline contribute to the urea cycle substrate requirements of the animal. Ornithine or citrulline, or both are to be considered optional components of the formulation. These additional ingredients are to be included to support nutritional requirements of the urea cycle. Where ornithine and citrulline are included in the formulation, the concentration of these ingredients most preferably is 0.1-0.2% (or 1-2 g/l) ornithine and about 0.1% (or 1 g/l) citrulline.

**Table 15**

| **Arginine-Free Formulation Mixture of Amino Acids** | | |
|---|---|---|
| Amino Acid | 2X Concentrate (mg/100 ml) | Final Feeding Concentration (g/l) |
| Isoleucine | 600 | 3 |
| Leucine | 1,000 | 5 |
| Lysine | 1,000 | 5 |
| Methionine | 200 | 1 |
| Phenylalanine | 300 | 1.5 |
| Threonine | 400 | 2 |
| Tryptophan | 200 | 1 |
| Valine | 500 | 2.5 |
| Alanine | 900 | 4.5 |
| Histidine | 300 | 1.5 |
| Proline | 700 | 3.5 |
| Serine | 400 | 2.0 |
| Tyrosine | 450 | 2.0 |
| Glycine | 800 | 4.0 |
| Aspartic acid | 600 | 3 |
| Ornithine | 400 | 2 |

The ornithine content described for the formulation above may be omitted or it may be replaced with citrulline at a concentration of about 2 g/l. The amino acids concentrate (2X) is mixed with a pharmaceutically acceptable diluent, such as for example, a glucose solution in a proportion of 1 to 1 (1 part amino acid solution to 1 part of a dextrose solution). In addition, trace elements, vitamin supplements and essential salts (Na⁺, K⁺, PO₄⁻, Ca⁺⁺, Mg⁺⁺) may be added.

The anti-hypotensive formulations useful for treating or preventing hypotension may be administered as a parenteral nutritional formulation according to parenteral feeding methods well known to those of skill in the medical arts.

In practicing such method, a physiological benchmark will be referred to in order to determine at what point the administration of the arginine-free formulation should be terminated. For example, the patient's systolic blood pressure level may be monitored so as to determine when the patient has reached a physiologically acceptable level (defined as about 100 mm Hg). A return to normal systolic pressure may then be used to indicate the point at which nitric oxide production was reduced, and the animal being treated had escaped risk of a greater reduction in peripheral vascular resistance or arterial blood pressure.

Hypotension (low blood pressure) is defined as an adult human systolic blood pressure level of less than about 100 mm Hg. A physiologically acceptable systolic blood pressure in an adult human is at least about 100 mm Hg systolic blood pressure.

It has been observed that serum arginine levels increase upon the administration of a standard TPN formulation. Therefore, by eliminating arginine as an ingredient in a TPN formulation, the inventors propose that serum arginine levels will be significantly reduced in patients receiving such an arginine-free TPN formulation as compared to similarly situated patients whom had instead been receiving a standard TPN solution. A standard TPN solution which includes greater than 0.1% arginine would not be expected to constitute a hypotensive formulation within the meaning of the present invention.

### PROPHETIC EXAMPLE 13

### THERAPEUTIC REGIMEN OF ARGININE-FREE FORMULATIONS AND NITRIC OXIDE INHIBITORS FOR THE TREATMENT OF HYPOTENSION

The present prophetic example is provided to outline a method for treating the hypotension attendant a variety of pathologies, such as septic shock, chemotherapy-related hypotension, etc., in an animal. Most particularly, the herein described methods are provided to outline those most preferred methods for treating hypotension in an animal, such as a human, with the herein described arginine-free parenteral formulations and arginine analogs. The arginine analogs described herein have also been shown by the present inventors to be nitric oxide inhibitors, most specifically nitric oxide synthase inhibitors. Therefore, these terms may be used interchangeably in the description of the present methods and regimens.

One of ordinary skill in the medical arts armed with the *in vivo* and *in vitro* results disclosed herein regarding the effect of lowered physiological arginine in an animal and the effects of various arginine analogs and nitric oxide synthase inhibitors, will be apprised of sufficient teaching to employ the herein described therapeutic regimens and methods to treat hypotension in a patient. Particular physiological guidelines for the adaptation of doses and physiologically compatible solutions for use in a patient may be obtained through reference to a variety of medical text books, including *Remington's Pharmaceutical Sciences* (1990) (Mack Publishing Company, Easton Pennsylvania 18042, (18th edition)). These guidelines are to be considered by the clinician in preparing physiologically compatible and non-toxic formulations in treating a hypotensive condition in a patient.

The present inventors have demonstrated that a decrease in the physiological levels of arginine in an animal, particularly in a hypotensive animal, will enhance the pressor response to a pressor agent in the animal to provide an increase in blood pressure *in vivo*. In addition, the inventors have demonstrated herein that administration of a variety of arginine analogs, including NMA, NAA and NNA, provide an effective means of increasing blood pressure in hypotensive animals. This effect was demonstrated, even more specifically, in endotoxin-induced hypotensive animals. The studies presented in the present disclosure have also demonstrated that hypotensive animals which were not treated in any manner to reduce physiological arginine levels demonstrated a blunted response to blood pressure increasing agents (pressor agents). These studies, taken together, provide a reasonable scientific expectation that a combination therapeutic regimen which decreased physiological levels of arginine in a hypotensive animal, and which provided for the concurrent administration of a arginine analog capable of increasing blood pressure, or which provided for the administration of such an arginine analog subsequent to an effective decrease in physiological arginine levels, will provide an effective treatment for improving and/or eliminating a hypotensive condition in a patient. A reasonable correlation may be made between the results reported herein using laboratory animals and those responses which may be expected upon treatment of a human patient.

Accordingly, hypotension in a patient may be effectively treated by initially maintaining the patient on a supportive dietary source which is arginine-free. It is contemplated that reduced physiological concentrations of arginine may be obtained in a patient by maintaining the patient on an arginine-free parenteral formulation (TPN). Elimination of arginine from the parenteral formulation will function to decrease available arginine in the animal. As demonstrated in the examples provided herein, a decrease in available arginine levels in a hypotensive animal will function to enhance the response of the animal to a pressor agent. Most preferably, it is contemplated that a sufficiently lowered arginine concentration in the patient may be obtained after the patient has been maintained on the arginine-free parenteral formulation for at least 2-6 hours. The patient should be monitored by the attending physician for a decrease in arginine concentrations. An initial plasma or serum arginine level should be obtained as a baseline from which to gauge relative increases or decreases in physiological arginine levels. Most preferably, plasma or serum levels of arginine in the patient will be reduced to less than 4 µM arginine prior to the administration of an arginine analog to the patient. Lesser reductions in serum arginine will, however, be acceptable and even more preferred in the practice of the described method.

While results between patients will vary, it is contemplated that maintenance of a patient on an arginine-free parenteral formulation for at least 2-24 hours will reduce physiological arginine levels in the patient sufficiently to provide the enhanced pressor response with the arginine analog described herein. As used in the present description, the terms arginine analog and nitric oxide inhibitor and nitric oxide synthase inhibitor are used interchangeably.

Upon the reduction of physiological arginine levels in the patient to an acceptable level (defined as between trace and one-half of the original concentration of arginine) an arginine analog, such as NMA, NAA, NNA, or a combination thereof is to be administered to the patient. Most preferably, the arginine analog is to be administered *via* an intravenous route in a single bolus dose. The arginine analog is to be prepared most preferably in a sterile saline solution at a concentration sufficient to provide the patient with a dose of the arginine analog of the between 0.1 mg/kg to 100 mg/kg. Again, most preferably, the dose of the arginine analog most preferred for use in the herein described method is between 10 mg/kg to 30 mg/kg, or about 20 mg/kg.

The patient should be monitored for relative increases or decreases in blood pressure continuously at least once every hour during the treatment, and especially after administration of the arginine analog. The arginine analog of choice most preferred is NMA. Where no increase in blood pressure is obtained upon administration of the arginine analog, the dose of arginine analog should be increased and the increased dose provided to the patient in a subsequent treatment within 30 minutes of the initial arginine analog treatment.

### EXAMPLE 14

### PREPARATION OF ANTI-ENDOTOXIN ANTIBODIES

The present example is provided to demonstrate a proposed method for preparing anti-endotoxin antibodies to be used in the herein described combination therapeutic regimens and methods with an arginine-free parenteral formulation.

Polyclonal antibodies to endotoxin may be prepared through use of an immunization protocol, wherein an animal, for example a mouse, may be injected with a commercial preparation of endotoxin in an amount sufficient to establish anti-endotoxin antibody titer levels in the animal. By way of example, a commercial source of endotoxin may be obtained from Sigma Chemical Co. (St. Louis, Missouri). Endotoxin includes a polypeptide which is common to both gram negative bacterial and *Escherichia coli* cell wall materials.

HA-1A (Centoxin) is a human monoclonal IgM antibody that binds to the lipid A domain of endotoxin and is produced by the stable heteromyeloma cell line A6(H4C5) developed by Teng, Kaplan, and Braude. (Teng *et al*. Proc. Natl. Acad. Sci. U.S.A. 82:1790-4, 1985). HA-1A has been shown to bind specifically to many endotoxins and to a broad range of clinical isolates of gram-negative bacteria. In various animal models of gram-negative bacteremia and endotoxemia, the administration of HA-1A after challenge prevents the development of the dermal Shwartzman reaction and death. (Teng *et al*. Proc. Natl. Acad. Sci. U.S.A. 82:1790-4, 1985; Ziegler *et al*. A. Clin. Res. 35:619A, 1987).

HA-1A is produced by continuous-perfusion cell culture and is purified from the supernatant fluid by a series of steps involving selective precipitation and column chromatography. (Ziegler *et al*. N. Engl. J. Med. 324:429-436, 1991). The cell line that produces HA-1A has been tested extensively and has been shown to be free of human viruses. Furthermore, the purification process for HA-1A includes specific viral-inactivation procedures, and tests are performed to confirm the absence of viruses. None of the lots contained detectable endotoxin in an assay with a sensitivity of 3 pg per milliliter.

Two monoclonal antibodies to endotoxin, E5 and HA-1A, await approval from the Food and Drug Administration. E5, which was developed from murine splenocytes immunized with J5 mutant *E. coli* cells, is an IgM antibody with reactivity to lipid A. (Greenman *et al*., JAMA 266:1097-1102, 1991). HA-1A is a human IgM antibody (also derived from immunization with J5 mutant cells) that binds specifically to lipid A (Ziegler *et al*., N. Engl. J. Med. 324:429-436, 1991). Both E5 and HA-1A have been shown to bind to endotoxin from a wide variety of gram-negative bacteria, and both have been evaluated in randomized, double blind, controlled trials.

In an HA-1A study, a single 100-mg intravenous dose of antibody was administered to 262 patients; 281 received placebo. (Ziegler *et al*. N. Engl. J. Med. 324:429-436, 1991). Active treatment produced a 39% decrease in 28-day mortality among the 200 patients with gram-negative bacteremia (37% of all study patients). Treatment benefit extended to the 101 patients with gram-negative bacteremia who were in shock (defined as a systolic blood pressure of <90 mm Hg or the use of vasopressors to maintain blood pressure) at study entry; in this subgroup, active treatment reduced 28-day mortality by 42%. Among patients with gram-negative bacteremia, active treatment increased the organ failure resolution. All evidence of organ failure disappeared within 7 days in 38 of 61 patients (62%) given HA-1A and in 26 of 62 patients (42%) given placebo.

Some investigators have reported negative results with anti-endotoxin core antiserum. Most of their studies were performed in mice. Although the reasons for discrepant results are not fully understood, several factors may contribute. (McCabe *et al*. J. Infect. Dis. 158:291-300, 1988; Ziegler, E.J. J. Infect. Dis. 158:286-290, 1988). These include the relative resistance of rodents to endotoxin, the need to compromise host defenses severely in order to establish satisfactory animal models of gram-negative infection and endotoxemia, and the rather low affinity of cross-reactive anti-endotoxin core antibodies as compared with type-specific antibodies. Recently, Baumgartner *et al*. (J. Exp. Med. 171:889-96, 1990) reported a lack of protection by a human monoclonal antibody derived from cells isolated from the same original clone as HA-1A, but the antibody was not produced or purified by the laboratory that produced HA-1A. when species resembling humans in endotoxin sensitivity are studied, protection from J5 antibody can be demonstrated (Spier *et al*. Circ. Shock 28:235-48, 1989). The negative results of Calandra *et al*. with J5 immunoglobulin in patients with gram-negative septic shock (Calandra *et al*. J. Infect. Dis. 158:312-9, 1988) may have been due to the absence of IgM in their preparation.

HA-1A has been administered in phase I trials to 15 patients with cancer (Khazaeli *et al*. J. Biol. Response Mod. 9:178-84, 1990) and in unblinded fashion to 34 patients with sepsis, (Fisher *et al*. Crit. Care Med. 18:1311-5, 1990) as well as to the 291 patients who received it in the aforedescribed trial. In all these patients, HA-1A was safe, well tolerated, and nonimmunogenic.

Nitric oxide synthase inhibitors administered concomitantly with the anti-endotoxin antibody may include an arginine analog having inhibitory activity toward nitric oxide synthase as described in previous examples. It is expected that lower doses of the anti-endotoxin antibody will be therapeutically effective when administered concomitantly or subsequent to an arginine-free parenteral formulation.

### EXAMPLE 15

### PREPARATION OF ANTI-TUMOR NECROSIS FACTOR ANTIBODIES

The present example is provided to demonstrate a proposed method for preparing anti-tumor necrosis factor antibodies for use in the herein described therapeutic regimens and methods. Either a polyclonal antibody or monoclonal antibody specific for tumor necrosis factor may be prepared according to methods known to those of skill in the art. For preparation of a polyclonal antibody, immunization techniques wherein an animal is immunized with tumor necrosis factor, may be utilized. Alternatively, monoclonal antibodies may be prepared according to standard hybridoma protocols, wherein the spleen of a tumor necrosis factor-immunized animal is fused to an immortal tumor cell line to provide a hybridoma which produces anti-tumor necrosis factor monoclonal antibodies.

The administration of antibodies to TNFα protects against the lethal effects of subsequent endotoxin challenge in animals (Beutler *et al*. Science 229:869-871, 1985; Tracey *et al*. Nature 330:662-664, 1987; Opal *et al*. J. Infect Dis. 161:1148-1152, 1990; Silva *et al*. J. Infect. Dis. 162:421-427, 1990); this protection holds even if anti-TNFα antibodies are administered 30 minutes *after* endotoxin challenge (Hinshaw *et al*. Circ. Shock. 30:279-292, 1990; Bahrami *et al*. *In:* Program and Abstracts of the Second International Congress on the Immune ConsequenceS of Trauma, Shock, and Sepsis: Mechanisms and Therapeutic Approaches; March 6-9, 1991; Munich, Germany, Abstract). Thus, TNFα is believed to play a central role in the development of sepsis, and administration of anti-TNFα antibodies appears to be an attractive method for improving outcome, particularly when used in conjunction with an arginine-free parenteral formulation.

The efficacy of anti-TNF antibodies for treating hypotension in humans was demonstrated in humans by Exley *et al*. (1990). The anti-TNF antibodies described in Exley *et al*. (1990) therefore constitute a most particularly preferred monoclonal antibody for use in the present invention. In a phase 1 study, Exley *et al*. (Lancet 335:1275-1277, 1990) administered murine IgG monoclonal antibodies to recombinant human TNFα (CB0006) to 14 patients with severe septic shock. Mean arterial pressure increased markedly after CB0006 antibody infusion, and there were no adverse reactions to treatment. A controlled clinical trial of anti-TNFα antibodies is now under way.

A major potential benefit of monoclonal antibodies to TNFα is that such treatment may improve outcome in both gram-negative and gram-positive sepsis. Several factors may limit the success of this agent, however. First, TNFα levels have been detected in only about one third of patients with septic shock (Marks *et al*. Am. Rev. Respir. Dis. 141:94-97, 1990), possibly because of the short half-life of TNFα in humans (14 to 18 minutes). Thus, anti-TNFα antibodies may be administered too late (or too early) for them to have any effect. Second, several studies have demonstrated that elevated TNFα levels alone are insufficient to produce shock (Silva *et al*. J. Infect. Dis. 162:454-459, 1990; Sun *et al*. Am. J. Pathol. 136:949-956, 1990). Third, anti-TNFα antibodies may not be effective against all causes of sepsis.

However, it is contemplated that anti-TNF antibody, when used in conjunction with an arginine-free parenteral formulation described herein, would provide an effective method and regimen for the treatment of hypotension, particularly that hypotension attendant septic shock.

### PROPHETIC EXAMPLE 16

### ANTI-ENDOTOXIN ANTIBODIES AND ARGININE-FREE TPN FOR TREATMENT OF HYPOTENSION IN SEPTIC SHOCK

This example provides interleukin 1 receptor antagonist (particularly IL 1ra) in combination with an arginine-free parenteral formulation nitric oxide synthase inhibitors for treatment of hypotension.

An IL-1 specific inhibitor has been produced from human monocytes (Seckinger *et al*. J. Immunol. 139:1541-1545, 1987; Arend *et al*. J. Immunol. 134:3868-3875, 1985); this inhibitor blocks the binding of IL-1 to its cell surface receptors (Seckinger *et al*. J. Immunol. 139:1541-1545, 1987). The inhibitor has been cloned, and sequence analysis reveals 40% conserved amino acid homology with IL 1β. It has been renamed the IL-1 receptor antagonist (IL 1ra)(Hannum *et al*. Nature 343:336-340, 1990; Eisenberg *et al*. Nature 343:341-346, 1990; Carter *et al*. Nature 344:633-638, 1990), and it competes with IL-1 for occupancy of surface receptors without agonist effects.

Wakabayashi *et al*. (FASEB Journal 5:338-343, 1991) have blocked endogenous IL-1 activity by pretreatment with human recombinant IL 1ra in a model of *E. coli*-induced shock in rabbits. The IL 1ra-treated group received IL 1ra intravenous injection as a 10-ml bolus (10 mg/kg) at t = -15 min, followed by a constant rate (15 µg · kg⁻¹ · min⁻¹) for 4 h. In saline-treated controls, hypotension was sustained for 4 h and death occurred for two of five rabbits; in rabbits treated with the IL 1ra, however, blood pressure was only transiently decreased, returned to pre-*E. coli* levels, and no deaths occurred. The associated leukopenia was also reduced by treatment with the antagonist (P < 0.05). Histological examination of lung tissues showed reduced infiltrating neutrophils in the IL 1ra treatment group. Despite the attenuated responses in animals treated with the IL 1ra, circulating TNF and IL 1 levels were nearly identical in both groups. The specific blockade of IL 1 at the receptor level demonstrates an essential role for this cytokine in the pathogenesis of septic shock.

An aspect of the present invention is the IL 1ra in combination with an arginine-free parenteral formulation in the treatment of hypotension. The Wakabayashi *et al*. (1990) article describes a method for preparing an IL-1 receptor antagonist which may be used in the practice of the present invention.

### PROPHETIC EXAMPLE 17

### ANTI-ENDOTOXIN ANTIBODIES AND ARGININE-FREE TPN FOR TREATMENT OF HYPOTENSION IN SEPTIC SHOCK

The present example is provided to demonstrate the proposed use of a therapeutic regimen of an arginine-free parenteral formulation and anti-endotoxin antibodies in the treatment of hypotension. More specifically, it is proposed that the present methods and compositions may be used in the treatment of hypotension in patients exposed to endotoxin and/or who have developed septic shock.

The earliest possible intervention in endotoxin (septic) shock involves the use of antibodies directed against endotoxin itself or against cytokines participating in the cascade initiated by endotoxin (i.e., tumor necrosis factor and IL-1) Although antibodies are of no use in studies such as those of Ochoa et al., (1992) *J. Natl. Cancer Inst*., 84:854-867, where a cytokine is used therapeutically, antibodies show some clinical activity in septic shock (Ziegler *et al*. (1991) *N. Eng. J. Med*. 324:429-437); Baumgartner *et al*. (1990) *J. Exp. Med*. 171:889-896; Calandra *et al*. (1988) *J. Infect. Dis*. 158:312-319. The advantages of antibody therapy are that all adverse effects of endotoxin can, in principle, be avoided and that constitutive nitric oxide synthase is not inhibited. Antibodies should most preferably be given before nitric oxide synthase is induced. Once induced, nitric oxide synthase may remain active for 24-48 hours. In a clinical setting, NO mediated shock cannot be anticipated with the necessary degree of certainty.

Antibodies directed against endotoxins will be prepared as described herein using immunization protocols of an animal injected with endotoxin, or by standard hybridoma technology well known to those of skill in the art. One potential method for the preparation of these antibodies is described herein. The most preferred anti-endotoxin antibody for use in the invention is HA-1A, previously described. A commercial source of endotoxin is available from suppliers such as Sigma Chemical Co. (St. Louis, Missouri).

The anti-endotoxin antibody would preferably be administered to the patient concurrently with the administration of an arginine-free TPN. The arginine-free TPN is described in Example 1, and most preferably will include citrulline, ornithine, or both, to even further satisfy urea cycle requirements in the animal.

A therapeutically effective concentration of the anti-endotoxin antibody will be determined on the basis of patient response as an improvement of the hypotensive condition. An improvement in the hypotensive condition as defined for purposes of describing the present invention, is demonstrated by an increase in systolic pressure to at least 100 mm Hg in the patient. A systolic blood pressure of at least 100 mm Hg is defined as a normotensive condition. The antibodies are most preferably to be administered before nitric oxide synthase induction to be fully effective.

### PROPHETIC EXAMPLE 18

### INTERLEUKIN-1 RECEPTOR ANTAGONISTS AND ARGININE-FREE TPN FOR TREATMENT OF HYPOTENSION

The present example is provided to outline a proposed method for the treatment of hypotension in an animal, particularly that hypotension attendant exposure to endotoxin or septic shock, through the use of an arginine-free parenteral formulation and an interleukin-1 receptor antagonist. The most preferred interleukin-1 receptor antagonists for use in conjunction with the present invention may be obtained from, or prepared according to the method outlined in Wakabayashi *et al*. (1991).

By way of example, the specific interleukin-1 receptor antagonist to be used is IL 1ra. This interleukin-1 receptor antagonist is described in Wakabayashi *et al*. (1991)⁵⁰, and is most preferably to be administered *via* a bolus intravenous infusion of between 1 mg/kg to 100 mg/kg, with the most preferred dose being about 10 mg/kg.

The arginine-free parenteral formulation is to be prepared as described herein. A patient having a systolic blood pressure of less than about 100 mm Hg will be targeted for the present treatment. Such a patient is to be placed on a continuous feed of an arginine-free formulation which includes a mixture of essential and nonessential amino acids as described herein. The formulation, in one embodiment, may be supplemented with 1-2 g/l ornithine and/or 1-2 g/l citrulline. The patient is to be maintained on the arginine-free TPN concurrently with the interleukin-1 antagonist. Blood samples are to be obtained from the patient and arginine levels in the serum or plasma fraction are to be determined. The patient is to be given an intravenous bolus dose of interleukin-1 receptor antagonist, most preferably IL-1 ra. This particular interleukin-1 receptor antagonist is described in Wakabayashi *et al*. (1991)⁵⁰.

Tachycardia (>90 beats per minute in the absence of beta-blockade) and tachypnea (respiratory rate >20 beats per minute or the requirement of mechanical ventilation) have been characterized as signs of systemic toxicity (Ziegler *et al*. (1991) *The New England Journal of Medicine*, 324(7):429-436). With the combination therapeutic regimen described herein, the treated patient may also be free of these systemic toxicity symptoms as well as exhibiting normotensive systolic blood pressure.

The blood pressure of the patient may be monitored, for example using a cuff-blood pressure monitoring device, after the interleukin-1 receptor antagonist is administered. The patient is to be maintained on the continuous parenteral feed of the arginine-free TPN both before and after the antagonist is administered. A return to normotensive blood pressure levels (at least 100 mm Hg systolic blood pressure) may result in the patient upon treatment according to the claimed method. Reduction in serum/plasma arginine levels in combination with the antagonist may provide relief from other signs of systemic toxicity, unlike previous reports using the antagonist alone (IL 1ra).

### PROPHETIC EXAMPLE 19

### ANTI-TUMOR NECROSIS FACTOR AND ARGININE-FREE TPN FOR TREATMENT OF HYPOTENSION

The present example is provided to demonstrate one preferred method by which the herein described therapeutic regimen and methods may be employed for the treatment of hypotension.

Anti-tumor necrosis factor antibodies may be prepared as described by Calandra *et al*. (1991), *In*: Bacterial Endotoxins: Cytokines Mediators and New Therapies for Sepsis, pp. 141-159). By way of example, such anti-tumor necrosis factor antibodies include a polyclonal anti-TNF antibody described by Beutler *et al*. (Beutler *et al*. (1985), Science, 229:869-871). An anti-TNF monoclonal antibody may be used for the herein described methods as well, and is more particularly preferred. Such a monoclonal antibody for TNF is described by Tracey *et al*. (1987), *Nature*, 330:662-664. The Tracey *et al*. article describes a method for preparing the anti-TNF monoclonal antibodies which may be used in conjunction with the herein claimed combination therapeutic regimens and methods. Such an anti-TNF monoclonal antibody preferred is CB0006.

A patient is first to be identified as having hypotension, a condition which is described for purposes of the present invention as a systolic blood pressure of less than about 100 mm Hg. Once a patient has been determined to have a hypotensive condition, he or she is to be started on an parenteral formulation which is arginine-free. The arginine-free TPN is defined compositionally herein. This formulation may also include citrulline and/or ornithine so as to insure metabolic requirements of the urea cycle in the animal. The animal or patient is to be maintained on the arginine-free TPN until serum or plasma arginine levels have been reduced. For this reason, the patient or animal is to be monitored continuously for changes in arginine concentrations in serum and/or plasma samples.

A bolus dose of the anti-TNF antibody, most preferably a monoclonal anti-TNF antibody as described in Exley *et al*. (1989) (Murine monoclonal antibody to recombinant human tumor necrosis factor in the treatment of patients with severe septic shock. Abstract No. 324. Program and abstracts of the 29th interscience conference on anti-microbial agents and chemotherapy, 155) is administered to the patient simultaneously with the arginine-free parenteral formulation. Thus, during administration of the anti-TNF antibody, the patient and/or animal is to be maintained on the arginine-free parenteral formulation. Upon such treatment, the patient and/or animal may demonstrate an increase in systemic blood pressure levels to normotensive levels (i.e., at least about 100 mm Hg).

Treatment of patients with severe systemic shock with a murine monoclonal antibody to recombinant human TNF (anti-rhTNF) was reported in the Exley *et al*. (1989) study, with a reversal of hypotension in nine of ten patients. However, 50% of these patients reported died within 7 days of attaining normotensive levels. Such a mortality rate is reported by those authors as relatively the same as mortality rates in patients with septic shock receiving no treatment (see Calandra *et al*. (1988). Therefore, no protection against death is observed when anti-TNF antibodies are used alone after an infectious challenge. Administration of an arginine-free total parenteral formulation to the patient prior to and concurrently with the antibody treatment may provide for an enhancement in patient survival and improvement in patient mortality.

### EXAMPLE 20

The present example demonstrates the effects of interleukin-1 receptor antagonists on the induction of nitric oxide synthase by IL-1 or IL-1 plus IFN-α. while the results demonstrate *in vitro* results, the data is also indicative of those effects to be expected from the use of interleukin-1 receptor antagonist *in vivo*.

This example demonstrates inhibition of IL-1-α-induced nitric oxide synthase in vascular smooth muscle.

### Materials

Human recombinant, IL-1-α (hereinafter referred to as IL-1; specific activity, 2 x 10⁹ lymphocyte-activating factor units/mg) was produced by Dainippon Pharmaceutical Co., Ltd. (Osaka, Japan) and provided by the National Cancer Institute. Human recombinant Il-1 receptor antagonist was produced by Synergen (Boulder, Co.). Rat interferon-γ (IFN-γ) was obtained from Amgen Biologicals (Thousand Oaks. Ca.) Except where indicated, all biochemical reagents were obtained from Sigma Chemical Co. (St. Louis, Mo.). Cell culture media and reagents, unless otherwise noted, were from Whittaker Bioproducts (Walkersville, Md.).

### Cell Culture

Mouse A375 melanoma cells were provided by Dr. E. Kleinerman, The University of Texas M.D. Anderson Cancer Center. Cells were maintained in Dulbecco's modified Eagle medium and Ham's F-12 medium (1:1) containing 10 mM HEPES buffer (pH 7.4) and 10% fetal bovine serum. All tissue culture reagents contained less than 0.25 ng/mL endotoxin as measured by the limulus amebocyte assay. Murine D10 T cells were obtained from the American Type Culture Collection (Rockville, Md.).

Aortic smooth muscle cells were cultured by explanting segments of the medial layer of aortas from adult male Fischer 344 rats. Aortas were removed aseptically and freed of adventitial and endothelial cells by scraping both the luminal and abluminal surfaces. Medial fragments were allowed to attach to Primaria 25-cm² tissue culture flasks (Becton-Dickinson, Lincoln Park, N.J.) which were kept moist with growth medium until cells emerged. Cultures were fed twice weekly with medium 199 containing 10% fetal bovine serum, 25 mM HEPES buffer (pH 7.4), 2mM L-glutamine, 40 µg/mL endothelial cell growth supplement (Biomedical Technologies, Inc., Stoughton, MA) and 10 µg/ml gentamicin (GIBCO BRL, Grand Island, NY). When primary cultures became confluent, they were passaged by trypsinization, and explants were discarded. For these studies, cells from passages 12-14 were seeded at 20,000 per well in 96-well plates and were used at confluence (60,000-80,000 cells per well). The cell exhibited the classic smooth muscle cell phenotype with hill and valley morphology, and they stained positively for smooth muscle actin.

### Cell Respiration Assay

Rat aortic smooth muscle cells in 96-well microtiter plates were incubated for 90 minutes in RPMI-1640 medium containing 0.2 mg/mL 3-(4,5 dimethylthiazol-2-γl)-2,5-diphenyltetrazolium bromide (MTT), washed with Hanks' balanced salt solution, and solubilized in 100 µL of dimethyl sulfoxide. The extent of reduction of MTT to formazan within cells, quantitated by measurement of the optical density at 550 nm (OD₅₅₀), was taken as an indicator of cellular respiration (Klostergaard, J., *et al*. J. Immunol. Methods 101:97-108, 1987).

### IL-1-Induced Cell Proliferation Assay

Murine D10 cells, an IL-1 dependent T-cell line, were used to measure IL-1 mitogenic activity. Cell proliferation in the present of IL-1 was assessed by incorporation of (³H) thymidine as previously described (Bakouche, O., *et al*. J. Immunol. 138:4249-4255, 1987).

### IL-1-Induced Cytotoxicity Assay

IL-1-induced cytotoxicity was studied using A375 tumor cells plated at a density of 6000 cells per well in 96-well microliter plates. After overnight attachment, IL-1 (3-300 ng/mL) was added in the presence or absence of NAA or NMA. After cells were incubated for 3 days, (³H) thymidine was added (1 µCi per well) for an additional 2 hours. Cells were harvested onto glass fiber disks (PHD Cell Harvested; Cambridge Technology, Inc., Watertown, Ma.) Disks were air dried overnight, and radioactivity was determined with a Model 1900TR Scintillation Counter (Packard Instrument Division, Downers Grove, Il.)

### Induction and Assay of Nitrite Synthesis in Smooth Muscle Cells

Rat aortic smooth muscle cells were incubated with RPMI-1640 medium containing 10% bovine calf serum, 25 mM HEPES buffer (pH 7.4), 2mM glutamine, 80 U/mL penicillin, 80 µg/mL streptomycin, 2 µg/mL fungizone, and IL-1, IFN-γ, and various inhibitors at the concentrations indicated in the figure legends. At the desired times, nitrite concentration in the culture medium was measured using the standard Griess assay (Green, L., *et al*. Anal. Biochem. 126:131-138, 1982) adapted to a 96-well microtiter plate reader (Gross, S.S., *et al*. Biochem. Biophys. Res. Commun. 178:823-829, 1991). Thus, 100 µL of Griess reagent (0.5% sulfanilic acid, 0.05% naphthalenediamine, and 2.5% phosphoric acid) was added to an equal volume of culture medium, and the OD₅₅₀ was measured and related to nitrite concentration by reference to a standard curve. The background OD₅₅₀ of medium incubated in the absence of cells was subtracted from experimental values.

### Preparation and Assay of Smooth Muscle Cell NO Synthase

Rat aortic smooth muscle cells were incubated with RPMI-1640 medium containing 10% bovine calf serum, 25 mM HEPES buffer (pH 7.4), 2 mM glutamine, 80 µg/mL penicillin, 80 µg/mL steptomycin, 2 µg/mL fungizone, 30 µg/mL lipopolysaccharide (*Escherichia coli* 0111:B4), and 50 U/mL IFN-γ. Cells were harvested after 24 hours, and cytosol was prepared (Gross, S.S., *et al*. Biochem. Biophys. Res. Commun. 178:823-829, 1991). Cytosolic NO synthase activity was assayed by the Fe²⁺ - myoglobin method described previously (Gross, S.S., *et al*. Biochem. Biophys. Res. Commun. 178:823-829, 1991).

### RESULTS

### Induction of Nitric Oxide Synthase in Vascular Smooth Muscle Cells

When grown in the absence of biological response modifiers, rat aortic smooth muscle cells showed no evidence of nitric oxide synthase activity. When cultured in the presence of human recombinant IL-1 (40 ng/mL), however, these cells formed and released substantial amounts of nitrite, a stable degradation product of NO. Nitrite synthesis was evident within 14 hours and continued to increase for at least 40 hours (Fig. 28). These observations are in accord with recent reports that IL-1 induces rat (Beasley, D.*, et al*. J. Clin. Invest. 87:602-608, 1991) and rabbit (Busse & Mulsch Febs Lett. 275:87-90, 1990) aortic smooth muscle cells in culture to release a factor that activates guanylate cyclase and decays to nitrite. As shown in Fig. 28, IFN-γ, which does not itself induce nitric oxide synthase in smooth muscle cells, significantly enhanced the induction of synthase by IL-1. IL-1 receptor antagonist inhibited the induction of nitric oxide synthase by IL-1 or IL-1 plus IFN-γ by more than 98%. Concentration levels of IL-1 as low as 1 ng/mL induced detectable nitric oxide synthase activity in smooth muscle cells; the ED₅₀ (i.e., dose which gives a response that is 50% of maximum) values for IL-1 alone and for IL-1 plus IFN-γ were about 5 ng/mL and 1 ng/mL, respectively (Fig. 28, Panel B).

Induction of nitric oxide synthase was dependent on both RNA and protein synthesis. Thus, control monolayers of smooth muscle cells cultured for 14 hours in the presence of IL-1 (100 ng/mL) and IFN-γ (50 ng/mL) produced 2.52 = 0.28 mnol (mean ± SD) of nitrite when transferred to cytokine-free medium and cultured for an additional 16 hours. Equal numbers of smooth muscle cells cultured similarly in medium supplemented with either 0.5 mg/mL of dactinomycin or 1 mg/mL of cycloheximide produced less than 0.1 nmol of nitrite. Neither dactinomycin nor cycloheximide adversely affected cell viability under these conditions, as judged by reduction of MTT, a measure of mitochondrial respiration. (MTT reduction [mean ± SD] by dactinomycin- and cycloheximide-treated IL-1-activated cells was 101% ± 6% and 95% ± 7%, respectively, of that measured in control cells that were treated with IL-1 alone.)

Those of skill in the pharmaceutical and/or neurophysiological arts will be able to practice the present invention with the aid of the disclosure provided here, the following references may facilitate practice or enhanced understanding of certain aspects.

### Bibliography

1. Parrillo, J.E., (1989), *Septic Shock in Humans: Clinical Evaluation, Pathogenesis, and Therapeutic Approach in*: Textbook of Critical Care, 2nd ed.,
2. Natanson *et al*., (1989), *J. Exp. Med., 169*:823.
3. Halusha *et al*., (1985), *Crit. Care Med., 13:*451.
4. Smedegard *et al*., (1989), *Am. J. Pathol., 135*:489.
5. Buetler *et al*., (1985), *Science, 229:*869.
6. Casals-Stenzel, (1987), *European. Pharmacology, 135*:117.
7. Wise *et al*., (1985), *Circ. Shock, 17*:59.
8. Hanasawa *et al*., (1989), *Surg. Gynecol. Obstet., 168*:232.
9. Tracey *et al*., (1987), *Nature, 330*:662-664.
10. Furchgott *et al*., (1980), *Nature, 288*:373-376.
11. Sakuma *et al*., (1988), *PNAS, 85*:8664-8667.
12. Aisaka *et al*., (1989) *BBRC*, 160:881-886.
13. Stuehr *et al*., (1989), *J. Exp. Med., 169*:1011-1020.
14. Stuehr *et al*., (1989), *BBRC, 161*:420-426.
15. Wagner *et al*., (1983), *PNAS, 80*:4518-4521.
16. Bevilaqua, (1986), *PNAS, 83*:4533-5437.
17. Rossi, (1985), *Science, 229*:174.
18. Pober, (1987), *J. Immunol., 138*:2149-2154.
19. Goldblum *et al*., (1989), *Infect. Immunol., 57*:1218-1226.
20. Tracey *et al*., (1986), *Science, 234*:470.
21. Rosenberg *et al*., (1986), *Clinical Res., 34*(2):413A.
22. Dinarello *et al*., (1989), *Progress in Clinical and Biological Res., 286*:243-263.
23. Kilbourn *et al*., (1990), *Proc. Natl. Acad. Sci., 87*:3629-3632.
24. Kilbourn *et al*, (1990), B.B.R.C., 172:1132-1138.
25. Kilbourn, (1990), *J.N.C.I*., 82(9):792-796.
26. Lehninger *et al*., Eds., *Biochemistry*, 2nd ed. (1975), Chpt. 25:693-723.
27. Wagner *et al*., (1983), *PNAS, 80*:4518-4521.
28. Tracey *et al*., (1986), *Science, 234*:470.
29. Cavender, (1987), *J. Immunol*., 138:2149-2154.
30. Shipley *et al* (1947), *Proc. Soc. Exp. Biol. Med, 65*:453-455.
31. Li *et al*, (1989), *Chinese Medical Journal*, 102:922-925.
32. Hesse *et al*., (1988), *Surg. Gynecol. Obstet., 166*:147.
33. Etienne *et al*., (1986), *Pharmacol. Res. Commun., 18*:71.
34. Salvemini *et al*., (1990), *Proc. Natl. Acad. Sci., 87*:2593.
35. *Cecil's Textbook of Medicine*, (1982), *Cardiovascular Disease*, Eds., pp. 155-168.
36. Vanzee *et al*., (1990), J. Immunol., 146:3478-3482.
37. Ignarro, L.J.: Ann Rev Pharmacol. Toxicol 30:535-60, 1990.
38. Hibbs, J., and Taintor, R. (1986), Methods in Enzymology, 132, 508-520.
39. Aisaka, K., S. Gross, O. Griffith and R. Levi: Biochem Biophys Res. Commun 160:881-886, 1989.
40. Rees, D., R. Palmer and S. Moncada: Proc Natl Acad Sci USA 86:3375-3378, 1989.
41. Fibbe, W.E., J.W.M. van der Meer, J.H.F. Falkenburg, M.S. Hamilton, P.M. Kluin and C.A. Dinarello: Exp Hematol 17:805-808, 1989.
42. Onozaki, K., K. Matsushima, B. Aggarwal and J. Oppenheim: The Journal of Immunology 135:3962-3967, 1985.
43. Braunschweiger, P., C. Johnson, N. Kumar, V. Ord and P. Furmanski: Cancer Research 48:6011-6016, 1988.
44. Smith, *et al*. (1990), Am Soc Clin Oncol 9:717721.
45. Crown, *et al*. Blood (In press).
46. Steis, *et al*. (1991), Proc Am Soc Clin Oncol 10:211.
47. Dinarello, *et al*. (1989), Pog Clin Biol Res 286:243-263.
48. Calandra, *et al*. (1990), J Infect Dis 161:982-987.
49. Ohlsson, *et al*. (1990), Nature 348:550-552.
50. Wakabayashi, *et al*. (1991), FASEB J 5:338-343.
51. Levi, *et al*. In: Nitric Oxide from L-Arginine: A Bioregulatory System, ed. S. Moncada and a. Higgs. 35-46. Amsterdam: Excerpta Medica, 1990.
52. Gross, *et al*. (1990), Biochemical And Biophysical Research Communications 170:96-103.
53. Fukuto, et al. (1990), Biochem Biophys Res. Commun 168:458-65.
54. Fasehun, *et al*. (1990), FASEB J 4:A309.
55. Gross, *et al*. (1991), Biochem Biophys Res Commun 178:823-829.
56. Lambert, *et al*. (1991), Life Sci 48:-69-75.
57. Nava, *et al*. (1991) Lancet 338:1555-1557.
58. Billar, *et al*. (1990) J. Leuk. Biol. 48:565-569.
59. Green, L., D. Wagner, J. Glogowski, P. Skipper, J. Wishnok and S. Tannenbaum: Analysis of Nitrate, Nitrite, and [¹⁵N] Nitrate in Biological Fluids. Analytical Biochemistry 126:131-138, 1982.
60. Palmer R, Rees D, Ashton D and Moncada S (June 1988), *Biochem. Biophys. Res. Commun., 153*:1251-1256.
61. Hibbs J, Vavrin Z, Tiator RR (Jan. 15, 1987)*, J. Immunol*., 138:550-565.
62. Ziegler EJ, *et al*. (1991)*, The New England Journal of Medicine*, 324(7):429-436.
63. Baumgartner JD, *et al*. (1990), *J. Exp. Med., 171*:889-896.
64. Calandra T, *et al*. (1988), *The Journal of Infectious Diseases, 158*(2):312-319.
65. Roger C. Bone (1991), *JAMA, 266*(12):1686-1691.
66. Opal SM *et al*. (1991), *J. Clin. Invest., 88*:885-890.
67. Wakabayashi GO *et al*. (1991), *FASEB, 5*:338-343.
68. Wolfe SM (1991), *The New England Journal of Medicine, 324*(7):486-488.
69. Calandra T *et al*. (1991), *In Bacterial Endotoxins: Cytokine Mediators and New Therapies for Sepsis*, pp. 141-159.
70. Eisenberg SP *et al*. (1990), *Nature*, 343:341-346
71. Hannum CH *et al*. (1990), *Nature*, 343:336-340.
72. Carter DB *et al*. (1990), *Nature*, 344:633-638.
73. Teng, NNH *et al*. (1985), *Proc. Natl. Acad. Sci USA*, 82:1790-1794
74. Ajani *et al*. U.S. Patent No. 4,988,724 (1991).
75. Ajani *et al*. U.S. Patent No. 4,859,452 (1989).

## Claims (Claims for the following Contracting State(s): AT, BE, CH, DE, DK, FR, GB, IE, IT, LI, LU, MC, NL, SE)

1. An anti-hypotensive parenteral or nutritional support formulation comprising an essentially arginine-free mixture of amino acids, the formulation upon administration to a hypotensive animal being capable of maintaining the animal's blood pressure at a physiologically acceptable level, the mixture of amino acids comprising:
alanine;
aspartic acid;
glycine;
histidine;
isoleucine;
leucine;
lysine;
methionine;
phenylalanine;
proline;
serine;
threonine;
tryptophan;
tyrosine; and
valine.

2. The formulation of claim 1, wherein the mixture includes:
4-5 g/l alanine;
2-3 g/l aspartic acid;
4-5 g/l glycine;
1-2 g/l histidine;
3-4 g/l isoleucine;
4-6 g/l leucine;
3-4 g/l lysine;
1-2 g/l methionine;
1-2 g/l phenylalanine;
3-4 g/l proline;
1-2 g/l serine;
2-3 g/l threonine;
0.5-1.5 g/l tryptophan;
0.25-0.75 g/l tyrosine; and
3-4 g/l valine
together in a pharmacologically acceptable excipient.

3. The formulation of claim 1 or 2, further comprising a therapeutically effective amount of an arginine analog capable of inhibiting nitric oxide production.

4. The formulation of claim 3, wherein the arginine analog is N^{ω}-methyl-L-arginine, N^{ω}-amino-L-arginine, N^{ω}-nitro-L-arginine, or a mixture thereof.

5. The formulation of claim 4, wherein the therapeutically effective amount of the analog is between 0.1 mg/kg and 100 mg/kg, preferably between 10 mg/kg and 30 mg/kg, more preferably about 20 mg/kg.

6. The formulation of any of the preceding claims, wherein hypotension is chemotherapeutic agent-related hypotension or is hypotension attendant to septic shock.

7. The formulation of claim 6, wherein the therapeutic agent is tumor necrosis factor, IL-1, IL-2, or a combination thereof.

8. The formulation of claim 6, wherein the septic shock is a bacterial endotoxin-related septic shock.

9. The formulation of claim 1 or 2, further comprising a therapeutically effective amount of an anti-endotoxin antibody, in particular of the anti-endotoxin antibody HA-1A.

10. The formulation of claim 9, wherein hypotension is attendant to septic shock, particularly endotoxin-related septic shock, more particularly bacterial endotoxin-related septic shock.

11. The formulation of claim 9, wherein the therapeutically effective amount of the anti-endotoxin antibody is between 0.1 mg/kg and 20 mg/kg.

12. The formulation of claim 1 or 2, further comprising a therapeutically effective amount of an interleukin-1 (IL-1) receptor agonist.

13. The formulation of claim 12, wherein hypotension is chemotherapeutic agent-related hypotension, particularly wherein the chemotherapeutic agent is tumor necrosis factor or IL-2, or, septic shock-related hypotension due to exposure to endotoxin.

14. The formulation of claim 12, wherein the therapeutically effective amount is between 1 mg/kg and 100 mg/kg.

15. The formulation of claim 1 or 2, further comprising a therapeutically effective amount of an anti-tumor necrosis factor antibody, in particular of the antibody CB 0006.

16. The formulation of claim 15, wherein hypotension is chemotherapeutic agent-related hypotension, particularly wherein the therapeutic agent is tumor necrosis factor, IL-1 or IL-2, or hypotension is due to endotoxin-related septic shock or cytokine-induced shock.

17. The formulation of claim 15, wherein the therapeutically effective amount is 0.1 mg/kg to 20 mg/kg.

18. The formulation of any of claims 1 to 5, 9, 12, and 15, further comprising ornithine, preferably in an amount of 1-4 g/l.

19. The formulation of any of claims 1, 2, 4, 5, 9, 12, and 15, further comprising citrulline, preferably in an amount of 1-2 g/l.

20. A parenteral formulation which upon administration to an animal, is capable of preventing systemic hypotension induced by tumor necrosis factor or endotoxin in an animal, said parenteral formulation comprising:
alanine;
aspartic acid;
glycine;
histidine;
isoleucine;
leucine;
lysine;
methionine;
phenylalanine;
proline;
serine;
threonine;
tryptophan;
tyrosine;
valine, and an amount of not more than 1g/l of arginine.

21. The formulation of any of claims 1 to 20 for use in human medicine.

22. Use of the formulation of any of claims 1 to 20 for the manufacture of a medicament for the prophylaxis and treatment of hypotension.

## Claims (Claims for the following Contracting State(s): ES, GR)

1. A method for preparing an anti-hypotensive parenteral or nutritional support formulation comprising an essentially arginine-free mixture of amino acids, the formulation upon administration to a hypotensive animal being capable of maintaining the animal's blood pressure at a physiologically acceptable level, the mixture of amino acids comprising:
alanine;
aspartic acid;
glycine;
histidine;
isoleucine;
leucine;
lysine;
methionine;
phenylalanine;
proline;
serine;
threonine;
tryptophan;
tyrosine; and
valine, the method comprising mixing the identified components of said formulation.

2. The method of claim 1, wherein the mixture includes:
4-5 g/l alanine;
2-3 g/l aspartic acid;
4-5 g/l glycine;
1-2 g/l histidine;
3-4 g/l isoleucine;
4-6 g/l leucine;
3-4 g/l lysine;
1-2 g/l methionine;
1-2 g/l phenylalanine;
3-4 g/l proline;
1-2 g/l serine;
2-3 g/l threonine;
0.5-1.5 g/l tryptophan;
0.25-0.75 g/l tyrosine; and
3-4 g/l valine.
together in a pharmacologically acceptable excipient, the method comprising mixing of the identified components of said formulation in the amounts specified.

3. The method of claim 1 or 2, wherein the formulation further comprises a therapeutically effective amount of an arginine analog capable of inhibiting nitric oxide production.

4. The method of claim 3, wherein the arginine analog is N^{ω}-methyl-L-arginine, N^{ω}-amino-L-arginine, N^{ω}-nitro-L-arginine, or a mixture thereof.

5. The method of claim 4, wherein the therapeutically effective amount of the analog is between 0.1 mg/kg and 100 mg/kg, preferably between 10 mg/kg and 30 mg/kg, more preferably about 20 mg/kg.

6. The method of any of the preceding claims, wherein hypotension is chemotherapeutic agent-related hypotension or is hypotension attendant to septic shock.

7. The method of claim 6, wherein the therapeutic agent is tumor necrosis factor, IL-1, IL-2, or a combination thereof.

8. The method of claim 6, wherein the septic shock is a bacterial endotoxin-related septic shock.

9. The method of claim 1 or 2, wherein the formulation further comprises a therapeutically effective amount of an anti-endotoxin antibody, in particular of the anti-endotoxin antibody HA-1A.

10. The method of claim 9, wherein hypotension is attendant to septic shock, particularly endotoxin-related septic shock, more particularly bacterial endotoxin-related septic shock.

11. The method of claim 9, wherein the therapeutically effective amount of the anti-endotoxin antibody is between 0.1 mg/kg to 20 mg/kg.

12. The method of claim 1 or 2, wherein the formulation further comprises a therapeutically effective amount of an interleukin-1 (IL-1) receptor agonist.

13. The method of claim 12, wherein hypotension is chemotherapeutic agent-related hypotension, particularly wherein the chemotherapeutic agent is tumor necrosis factor or IL-2, or, septic shock-related hypotension due to exposure to endotoxin.

14. The method of claim 12, wherein the therapeutically effective amount is between 1 mg/kg to 100 mg/kg.

15. The method of claim 1 or 2, wherein the formulation further comprises a therapeutically effective amount of an anti-tumor necrosis factor antibody, in particular of the antibody CB 0006.

16. The method of claim 15, wherein hypotension is chemotherapeutic agent-related hypotension, particularly wherein the therapeutic agent is tumor necrosis factor, IL-1 or IL-2, or hypotension is due to endotoxin-related septic shock or cytokine-induced shock.

17. The method of claim 15, wherein the therapeutically effective amount is 0.1 mg/kg to 20 mg/kg.

18. The method of any of claims 1 to 5, 9, 12, and 15, wherein the formulation further comprises ornithine, preferably in an amount of 1-4 g/l.

19. The method of any of claims 1, 2, 4, 5, 9, 12, and 15, wherein the formulation further comprises citrulline, preferably in an amount of 1-2 g/l.

20. A method for preparing a parenteral formulation which upon administration to an animal, is capable of preventing systemic hypotension induced by tumor necrosis factor or endotoxin in an animal, said parenteral formulation comprising:
alanine;
aspartic acid;
glycine;
histidine;
isoleucine;
leucine;
lysine;
methionine;
phenylalanine;
proline;
serine;
threonine;
tryptophan;
tyrosine;
valine, and an amount of not more than 1 g/l of arginine, the method comprising mixing of the identified components of said formulation in the amounts specified.

21. A formulation as defined in any of claim 1 to 20.

22. A formulation as defined in any of claims 1 to 20 for use in human medicine.

23. A method for the manufacture of a medicament for the prophylaxis ad treatment of hypotension, characterized in the use, as an essential constituent, of the formulation as defined in any of claims 1 to 20.

## Patentansprüche (Patentansprüche für folgende(n) Vertragsstaat(en): AT, BE, CH, DE, DK, FR, GB, IE, IT, LI, MC, NL, SE)

1. Antihypotonische parenterale oder nährende, unterstützende Formulierung, die ein im wesentlichen Arginin-freies Gemisch von Aminosäuren enthält, wobei die Formulierung bei Verabreichung an ein hypotonisches Lebewesen in der Lage ist, den Blutdruck des Lebewesens auf einem physiologisch akzeptablen Niveau zu halten, wobei das Gemisch von Aminosäuren enthält:
Alanin;
Asparginsäure;
Glycin;
Histidin;
Isoleucin;
Leucin;
Lysin;
Methionin;
Phenylalanin;
Prolin;
Serin;
Threonin;
Tryptophan;
Tyrosin; und
Valin.

2. Formulierung nach Anspruch 1, wobei das Gemisch enthält:
4-5 g/l Alanin;
2-3 g/l Asparginsäure;
4-5 g/l Glycin;
1-2 g/l Histidin;
3-4 g/l Isoleucin;
4-6 g/l Leucin;
3-4 g/l Lysin;
1-2 g/l Methionin;
1-2 g/l Phenylalain;
3-4 g/l Prolin;
1-2 g/l Serin;
2-3 g/l Threonin;
0,5-1,5 g/l Tryptophan;
0,25-0,75 g/l Tyrosin; und
3-4 g/l Valin,
zusammen in einem pharmazeutisch verträglichen Arzneimittelträger.

3. Formulierung nach Anspruch 1 oder 2, weiter enthaltend eine therapeutisch wirksame Menge an einem Arginin-Analogen, das zur Inhibierung einer Stickstoffoxiderzeugung in der Lage ist.

4. Formulierung nach Anspruch 3, wobei es sich bei dem Arginin-Analogen um N^{ω}-Methyl-L-arginin, N^{ω}-Amino-L-arginin, N^{ω}-Nitro-L-arginin oder ein Gemisch davon handelt.

5. Formulierung nach Anspruch 4, wobei die therapeutisch wirksame Menge an dem Analogen zwischen 0,1 mg/kg und 100 mg/kg, vorzugsweise zwischen 10 mg/kg und 30 mg/kg, stärker bevorzugt etwa 20 mg/kg, beträgt.

6. Formulierung nach einem der vorhergehenden Ansprüche, wobei es sich bei der Hypotonie um eine mit einem chemotherapeutischen Mittel verbundene Hypotonie oder um eine einen septischen Schock begleitende Hypotonie handelt.

7. Formulierung nach Anspruch 6, wobei das therapeutische Mittel der Tumor-Nekrose-Faktor, IL-1, IL-2 oder eine Kombination davon ist.

8. Formulierung nach Anspruch 6, wobei es sich bei dem septischen Schock um einen mit einem bakteriellen Endotoxin verbundenen septischen Schock handelt.

9. Formulierung nach Anspruch 1 oder 2, weiter enthaltend eine therapeutisch wirksame Menge an einem Anti-Endotoxin-Antikörper, insbesondere von dem Anti-Endotoxin-Antikörper HA-1A.

10. Formulierung nach Anspruch 9, wobei die Hypotonie eine Begleiterscheinung zu dem septischen Schock ist, insbesondere einen mit einem Endotoxin verbundenen septischen Schock, stärker bevorzugt einen mit einem bakteriellen Endotoxin verbundenen septischen Schock.

11. Formulierung nach Anspruch 9, wobei die therapeutisch wirksame Menge des Anti-Endotoxin-Antikörpers zwischen 0,1 mg/kg und 20 mg/kg liegt.

12. Formulierung nach Anspruch 1 oder 2, weiter enthaltend eine therapeutisch wirksame Menge an einem Interleukin-1 (IL-1)-Rezeptor-Agonisten.

13. Formulierung nach Anspruch 12, wobei es sich bei der Hypotonie um eine mit einem chemotherapeutischen Mittel verbundene Hypotonie handelt, insbesondere wobei das chemotherapeutische Mittel der Tumor-Nekrose-Faktor oder IL-2 ist, oder um eine mit einem septischen Schock verbundene Hypotonie handelt aufgrund eines einem Endotoxin Ausgesetztseins.

14. Formulierung nach Anspruch 12, wobei die therapeutisch wirksame Menge zwischen 1 mg/kg und 100 mg/kg liegt.

15. Formulierung nach Anspruch 1 oder 2, weiter enthaltend eine therapeutisch wirksame Menge an einem Antitumor-Nekrose-Faktor-Antikörper, insbesondere von dem Antikörper CB 0006.

16. Formulierung nach Anspruch 15, wobei es sich bei der Hypotonie um eine mit einem chemotherapeutischen Mittel verbundene Hypotonie handelt, insbesondere, wobei es sich bei dem therapeutischen Mittel um den Tumor-Nekrose-Faktor, IL-1 oder IL-2 handelt, oder die Hypotonie durch einen mit einem Endotoxin verbundenen septischen Schock oder einen durch ein Cytokin induzierten Schock bedingt ist.

17. Formulierung nach Anspruch 15, wobei die therapeutisch wirksame Menge 0,1 mg/kg bis 20 mg/kg beträgt.

18. Formulierung nach einem der Ansprüche 1 bis 5, 9, 12 und 15, weiter enthaltend Ornithin, vorzugsweise in einer Menge von 1-4 g/l.

19. Formulierung nach einem der Ansprüche 1, 2, 4, 5, 9, 12 und 15, weiter enthaltend Citrullin, vorzugsweise in einer Menge von 1-2 g/l.

20. Parenterale Formulierung, die bei Verabreichung an ein Lebewesen in der Lage ist, eine systemische Hypotonie zu verhindern, die durch den Tumor-Nekrose-Faktor oder ein Endotoxin in einem Lebewesen induziert wird, wobei die parenterale Formulierung enthält:
Alanin;
Asparginsäure;
Glycin;
Histidin;
Isoleucin;
Leucin;
Lysin;
Methionin;
Phenylalanin;
Prolin;
Serin;
Threonin;
Tryptophan;
Tyrosin;
Valin;
und eine Menge von nicht mehr als 1 g/l Arginin.

21. Formulierung nach einem der Ansprüche 1 bis 20 zur Verwendung in der Humanmedizin.

22. Verwendung der Formulierung nach einem der Ansprüche 1 bis 20 zur Herstellung eines Arzneimittels zur Prophylaxe und Behandlung von Hypotonie.

## Patentansprüche (Patentansprüche für folgende(n) Vertragsstaat(en): ES, GR)

1. Verfahren zur Herstellung einer antihypotonischen parenteralen oder nährenden, unterstützenden Formulierung, die ein im wesentlichen Argininfreies Gemisch von Aminosäuren enthält, wobei die Formulierung bei Verabreichung an ein hypotonisches Lebewesen in der Lage ist, den Blutdruck des Lebewesens auf einem physiologisch akzeptablen Niveau zu halten, wobei das Gemisch von Aminosäuren enthält:
Alanin;
Asparginsäure;
Glycin;
Histidin;
Isoleucin;
Leucin;
Lysin;
Methionin;
Phenylalanin;
Prolin;
Serin;
Threonin;
Tryptophan;
Tyrosin; und
Valin,
wobei das Verfahren das Vermischen der genannten Komponenten der Formulierung umfaßt.

2. Verfahren nach Anspruch 1, wobei das Gemisch enthält:
4-5 g/l Alanin;
2-3 g/l Asparginsäure;
4-5 g/l Glycin;
1-2 g/l Histidin;
3-4 g/l Isoleucin;
4-6 g/l Leucin;
3-4 g/l Lysin;
1-2 g/l Methionin;
1-2 g/l Phenylalanin;
3-4 g/l Prolin;
1-2 g/l Serin;
2-3 g/l Threonin;
0,5-1,5 g/l Tryptophan;
0,25-0,75 g/l Tyrosin; und
3-4 g/l Valin,
zusammen in einem pharmazeutisch verträglichen Arzneimittelträger, wobei das Verfahren das Vermischen der genannten Komponenten der Formulierung in den spezifizierten Mengen umfaßt.

3. Verfahren nach Anspruch 1 oder 2, wobei die Formulierung weiter eine therapeutisch wirksame Menge an einem Arginin-Analogen enthält, das zur Inhibierung einer Stickstoffoxiderzeugung in der Lage ist.

4. Verfahren nach Anspruch 3, wobei es sich bei dem Arginin-Analogen um N^{ω}-Methyl-L-arginin, N^{ω}-Amino-L-arginin, N^{ω}-Nitro-L-arginin oder ein Gemisch davon handelt.

5. Verfahren nach Anspruch 4, wobei die therapeutisch wirksame Menge an dem Analogen zwischen 0,1 mg/kg und 100 mg/kg, vorzugsweise zwischen 10 mg/kg und 30 mg/kg, stärker bevorzugt etwa 20 mg/kg, beträgt.

6. Verfahren nach einem der vorhergehenden Ansprüche, wobei es sich bei der Hypotonie um eine mit einem chemotherapeutischen Mittel verbundene Hypotonie oder um eine einen septischen Schock begleitende Hypotonie handelt.

7. Verfahren nach Anspruch 6, wobei das therapeutische Mittel der Tumor-Nekrose-Faktor, IL-1, IL-2 oder eine Kombination davon ist.

8. Verfahren nach Anspruch 6, wobei es sich bei dem septischen Schock um einen mit einem bakteriellen Endotoxin verbundenen septischen Schock handelt.

9. Verfahren nach Anspruch 1 oder 2, wobei die Formulierung weiter eine therapeutisch wirksame Menge an einem Anti-Endotoxin-Antikörper, insbesondere von dem Anti-Endotoxin-Antikörper HA-1A, enthält.

10. Verfahren nach Anspruch 9, wobei die Hypotonie eine Begleiterscheinung zu dem septischen Schock ist, insbesondere einen mit einem Endotoxin verbundenen septischen Schock, stärker bevorzugt einen mit einem bakteriellen Endotoxin verbundenen septischen Schock.

11. Verfahren nach Anspruch 9, wobei die therapeutisch wirksame Menge des Anti-Endotoxin-Antikörpers zwischen 0,1 mg/kg bis 20 mg/kg liegt.

12. Verfahren nach Anspruch 1 oder 2, wobei die Formulierung weiter eine therapeutisch wirksame Menge an einem Interleukin-1 (IL-1)-Rezeptor-Agonisten enthält.

13. Verfahren nach Anspruch 12, wobei es sich bei der Hypotonie um eine mit einem chemotherapeutischen Mittel verbundene Hypotonie handelt, insbesondere wobei das chemotherapeutische Mittel der Tumor-Nekrose-Faktor oder IL-2 ist, oder um eine mit einem septischen Schock verbundene Hypotonie handelt aufgrund eines einem Endotoxin Ausgesetztseins.

14. Verfahren nach Anspruch 12, wobei die therapeutisch wirksame Menge zwischen 1 mg/kg bis 100 mg/kg liegt.

15. Verfahren nach Anspruch 1 oder 2, wobei die Formulierung weiter eine therapeutisch wirksame Menge an einem Antitumor-Nekrose-Faktor-Antikörper, insbesondere von dem Antikörper CB 0006, enthält.

16. Verfahren nach Anspruch 15, wobei es sich bei der Hypotonie um eine mit einem chemotherapeutischen Mittel verbundene Hypotonie handelt, insbesondere, wobei es sich bei dem therapeutischen Mittel um den Tumor-Nekrose-Faktor, IL-1 oder IL-2 handelt, oder die Hypotonie durch einen mit einem Endotoxin verbundenen septischen Schock oder einen durch ein Cytokin induzierten Schock bedingt ist.

17. Verfahren nach Anspruch 15, wobei die therapeutisch wirksame Menge 0,1 mg/kg bis 20 mg/kg beträgt.

18. Verfahren nach einem der Ansprüche 1 bis 5, 9, 12 und 15, wobei die Formulierung weiter Ornithin, vorzugsweise in einer Menge von 1-4 g/l, enthält.

19. Verfahren nach einem der Ansprüche 1, 2, 4, 5, 9, 12 und 15, wobei die Formulierung weiter Citrullin, vorzugsweise in einer Menge von 1-2 g/l, enthält.

20. Verfahren zur Herstellung einer parenteralen Formulierung, die bei Verabreichung an ein Lebewesen in der Lage ist, eine systemische Hypotonie zu verhindern, die durch den Tumor-Nekrose-Faktor oder ein Endotoxin in einem Lebewesen induziert wird, wobei die parenterale Formulierung enthält:
Alanin;
Asparginsäure;
Glycin;
Histidin;
Isoleucin;
Leucin;
Lysin;
Methionin;
Phenylalanin;
Prolin;
Serin;
Threonin;
Tryptophan;
Tyrosin;
Valin;
und eine Menge von nicht mehr als 1 g/l Arginin, wobei das Verfahren das Vermischen der genannten Komponenten der Formulierung in den spezifizierten Mengen umfaßt.

21. Formulierung wie in einem der Ansprüche 1 bis 20 definiert.

22. Formulierung wie in einem der Ansprüche 1 bis 20 definiert, zur Verwendung in der Humanmedizin.

23. Verfahren zur Herstellung eines Arzneimittels zur Prophylaxe und Behandlung von Hypotonie, gekennzeichnet durch die Verwendung, als einem wesentlichen Bestandteil, der Formulierung wie in einem der Ansprüche 1 bis 20 definiert.

## Revendications (Revendications pour l'(les) Etat(s) contractant(s) suivant(s): AT, BE, CH, DE, DK, FR, GB, IE, IT, LI, LU, MC, NL, SE)

1. Formulation anti-hypotensive de support nutritionnel ou parentérale comportant un mélange d'acides aminés essentiellement exempt d'arginine, la formulation lors d'une administration à un animal hypotensif étant capable de maintenir la pression sanguine de l'animal à un niveau physiologiquement acceptable, le mélange d'acides aminés étant constitué :
d'alanine,
d'acide aspartique,
de glycine,
d'histidine,
d'isoleucine,
de leucine,
de lysine,
de méthionine,
de phénylalanine,
de proline,
de sérine,
de thréonine,
de tryptophane,
de tyrosine, et
de valine.

2. Formulation selon la revendication 1, dans laquelle le mélange comporte :
4-5 g/l d'alanine,
2-3 g/l d'acide aspartique,
4-5 g/l de glycine,
1-2 g/l d'histidine,
3-4 g/l d'isoleucine,
4-6 g/l de leucine,
3-4 g/l de lysine, 1-2 g/l de méthionine,
1-2 g/l de phénylalanine,
3-4 g/l de proline,
1-2 g/l de sérine,
2-3 g/l de thréonine,
0,5-1,5 g/l de tryptophane,
0,25-0,75 g/l de tyrosine, et
3-4 g/l de valine
ensemble dans un excipient pharmacologiquement acceptable.

3. Formulation selon la revendication 1 ou 2, comportant de plus une quantité thérapeutiquement efficace d'un analogue d'arginine capable d'inhiber la production d'oxyde azotique.

4. Formulation selon la revendication 3, dans laquelle l'analogue d'arginine est une N^{ω}-méthyl-L-arginine, N^{ω}-amino-L-arginine, N^{ω}-nitro-L-arginine, ou un mélange de celles-ci.

5. Formulation selon la revendication 4, dans laquelle la quantité thérapeutiquement efficace de l'analogue est située entre 0,1 mg/kg et 100 mg/kg, de préférence entre 10 mg/kg et 30 mg/kg, de manière plus préférée d'environ 20 mg/kg.

6. Formulation selon l'une quelconque des revendications précédentes, dans laquelle l'hypotension est une hypotension liée à un agent chimiothérapeutique ou est une hypotension accompagnant un choc bactériémique.

7. Formulation selon la revendication 6, dans laquelle l'agent thérapeutique est un facteur de nécrose tumoral, IL-1, IL-2, ou une combinaison de ceux-ci.

8. Formulation selon la revendication 6, dans laquelle le choc bactériémique est un choc bactériémique lié à une endotoxine bactérienne.

9. Formulation selon la revendication 1 ou 2, comportant de plus une quantité thérapeutiquement efficace d'un anticorps anti-endotoxine, en particulier l'anticorps anti-endotoxine HA-1A.

10. Formulation selon la revendication 9, dans laquelle l'hypotension accompagne un choc bactériémique, en particulier un choc bactériémique lié à une endotoxine, plus particulièrement un choc bactériémique lié à une endotoxine bactérienne.

11. Formulation selon la revendication 9, dans laquelle la quantité thérapeutiquement efficace de l'anticorps anti-endotoxine est située entre 0,1 mg/kg et 20 mg/kg.

12. Formulation selon la revendication 1 ou 2, comportant de plus une quantité thérapeutiquement efficace d'un agoniste de récepteur d'interleukine-1 (IL-1).

13. Formulation selon la revendication 12, dans laquelle l'hypotension est une hypotension liée à un agent chimiothérapeutique, en particulier dans laquelle l'agent chimiothérapeutique est un facteur de nécrose tumoral ou IL-2, ou, est une hypotension liée à un choc bactériémique due à une exposition à une endotoxine.

14. Formulation selon la revendication 12, dans laquelle la quantité thérapeutiquement efficace est située entre 1 mg/kg et 100 mg/kg.

15. Formulation selon la revendication 1 ou 2, comportant de plus une quantité thérapeutiquement efficace d'un anticorps de facteur de nécrose anti-tumoral, en particulier l'anticorps CB 0006.

16. Formulation selon la revendication 15, dans laquelle l'hypotension est une hypotension liée à un agent chimiothérapeutique, en particulier dans laquelle l'agent thérapeutique est un facteur de nécrose tumoral, IL-1 ou IL-2, ou l'hypotension est due à un choc bactériémique lié à une endotoxine ou à un choc induit par une cytokine.

17. Formulation selon la revendication 15, dans laquelle la quantité thérapeutiquement efficace est située entre 0,1 mg/kg et 20 mg/kg.

18. Formulation selon l'une quelconque des revendications 1 à 5, 9, 12, et 15, comportant de plus une ornithine, de préférence selon une quantité allant de 1 à 4 g/l.

19. Formulation selon l'une quelconque des revendications 1, 2, 4, 5, 9, 12 et 15, comportant de plus une citruline, de préférence selon une quantité allant de 1 à 2 g/l.

20. Formulation parentérale qui, lors d'une administration à un animal, est capable d'empêcher une hypotension systémique induite par un facteur de nécrose tumoral ou une endotoxine dans un animal, ladite formulation parentérale étant constituée :
d'alanine,
d'acide aspartique,
de glycine,
d'histidine,
d'isoleucine,
de leucine,
de lysine,
de méthionine,
de phénylalanine,
de proline,
de sérine,
de thréonine,
de tryptophane,
de tyrosine,
de valine, et d'une quantité inférieure à 1 g/l d'arginine.

21. Formulation selon l'une quelconque des revendications 1 à 20, destinée à être utilisée en médecine humaine.

22. Utilisation de la formulation selon l'une quelconque des revendications 1 à 20, pour fabriquer un médicament pour la prophylaxie et le traitement d'hypotension.

## Revendications (Revendications pour l'(les) Etat(s) contractant(s) suivant(s): ES, GR)

1. Procédé pour préparer une formulation anti-hypotensive de support nutritionnel ou parentérale comportant un mélange d'acides aminés essentiellement exempt d'arginine, la formulation lors d'une administration à un animal hypotensif étant capable de maintenir la pression sanguine de l'animal à un niveau physiologiquement acceptable, le mélange d'acides aminés étant constitué :
d'alanine,
d'acide aspartique,
de glycine,
d'histidine,
d'isoleucine,
de leucine,
de lysine,
de méthionine,
de phénylalanine,
de proline,
de sérine,
de thréonine,
de tryptophane,
de tyrosine, et
de valine, le procédé consistant à mélanger les composants identifiés de ladite formulation.

2. Procédé selon la revendication 1, dans lequel le mélange comporte :
4-5 g/l d'alanine,
2-3 g/l d'acide aspartique,
4-5 g/l de glycine,
1-2 g/l d'histidine,
3-4 g/l d'isoleucine,
4-6 g/l de leucine,
3-4 g/l de lysine,
1-2 g/l de méthionine,
1-2 g/l de phénylalanine,
3-4 g/l de proline,
1-2 g/l de sérine,
2-3 g/l de thréonine,
0,5-1,5 g/l de tryptophane,
0,25-0,75 g/l de tyrosine, et
3-4 g/l de valine
ensemble dans un excipient pharmacologiquement acceptable, le procédé consistant à mélanger les composants identifiés de ladite formulation selon les quantités spécifiées.

3. Procédé selon la revendication 1 ou 2, dans lequel la formulation comporte de plus une quantité thérapeutiquement efficace d'un analogue d'arginine capable d'inhiber la production d'oxyde azotique.

4. Procédé selon la revendication 3, dans lequel l'analogue d'arginine est une N^{ω}-méthyl-L-arginine, N^{ω}-amino-L-arginine, N^{ω}-nitro-L-arginine, ou un mélange de celles-ci.

5. Procédé selon la revendication 4, dans lequel la quantité thérapeutiquement efficace de l'analogue est située entre 0,1 mg/kg et 100 mg/kg, de préférence entre 10 mg/kg et 30 mg/kg, de manière plus préférée d'environ 20 mg/kg.

6. Procédé selon l'une quelconque des revendications précédentes, dans lequel l'hypotension est une hypotension liée à un agent chimiothérapeutique ou est une hypotension accompagnant un choc bactériémique.

7. Procédé selon la revendication 6, dans lequel l'agent thérapeutique est un facteur de nécrose tumoral, IL-1, IL-2, ou une combinaison de ceux-ci.

8. Procédé selon la revendication 6, dans lequel le choc bactériémique est un choc bactériémique lié à une endotoxine bactérienne.

9. Procédé selon la revendication 1 ou 2, dans lequel la formulation comporte de plus une quantité thérapeutiquement efficace d'un anticorps anti-endotoxine, en particulier l'anticorps anti-endotoxine HA-1A.

10. Procédé selon la revendication 9, dans lequel l'hypotension accompagne un choc bactériémique, en particulier un choc bactériémique lié à une endotoxine, plus particulièrement un choc bactériémique lié à une endotoxine bactérienne.

11. Procédé selon la revendication 9, dans lequel la quantité thérapeutiquement efficace de l'anti-corps anti-endotoxine est située entre 0,1 mg/kg et 20 mg/kg.

12. Procédé selon la revendication 1 ou 2, dans lequel la formulation comporte de plus une quantité thérapeutiquement efficace d'un agoniste de récepteur d'interleukine-1 (IL-1).

13. Procédé selon la revendication 12, dans lequel l'hypotension est une hypotension liée à un agent chimiothérapeutique, en particulier dans lequel l'agent chimiothérapeutique est un facteur de nécrose tumoral ou IL-2, ou, est une hypotension liée à un choc bactériémique due à une exposition à une endotoxine.

14. Procédé selon la revendication 12, dans lequel la quantité thérapeutiquement efficace est située entre 1 mg/kg et 100 mg/kg.

15. Procédé selon la revendication 1 ou 2, dans lequel la formulation comporte de plus une quantité thérapeutiquement efficace d'un anticorps de facteur de nécrose anti-tumoral, en particulier l'anticorps CB 0006.

16. Procédé selon la revendication 15, dans lequel l'hypotension est une hypotension liée à un agent chimiothérapeutique, en particulier dans lequel l'agent thérapeutique est un facteur de nécrose tumoral, IL-1 ou IL-2, ou l'hypotension est due à un choc bactériémique lié à une endotoxine ou à un choc induit par une cytokine.

17. Procédé selon la revendication 15, dans lequel la quantité thérapeutiquement efficace est située entre 0,1 mg/kg et 20 mg/kg.

18. Procédé selon l'une quelconque des revendications 1 à 5, 9, 12, et 15, dans lequel la formulation comporte de plus une ornithine, de préférence selon une quantité allant de 1 à 4 g/l.

19. Procédé selon l'une quelconque des revendications 1, 2, 4, 5, 9, 12 et 15, dans lequel la formulation comporte de plus une citruline, de préférence selon une quantité allant de 1 à 2 g/l.

20. Procédé pour préparer une formulation parentérale qui, lors d'une administration à un animal, est capable d'empêcher une hypotension systémique induite par un facteur de nécrose tumoral ou une endotoxine dans un animal, ladite formulation parentérale étant constituée :
d'alanine,
d'acide aspartique,
de glycine,
d'histidine,
d'isoleucine,
de leucine,
de lysine,
de méthionine,
de phénylalanine,
de proline,
de sérine,
de thréonine,
de tryptophane,
de tyrosine,
de valine, et d'une quantité inférieure à 1 g/l d'arginine, le procédé consistant à mélanger les composants identifiés de ladite formulation selon les quantités spécifiées.

21. Formulation selon l'une quelconque des revendications 1 à 20.

22. Formulation selon l'une quelconque des revendications 1 à 20, destinée à être utilisée en médecine humaine.

23. Procédé pour fabriquer un médicament pour la prophylaxie et le traitement d'hypotension, caractérisé par l'utilisation, en tant que constituant essentiel, de la formulation selon l'une quelconque des revendications 1 à 20.
